(19) Europäisches Patentamt
European Patent Office
Office européen des brevets

(11) EP 4 335 461 A1

(12) **EUROPEAN PATENT APPLICATION**

(43) Date of publication:
**13.03.2024 Bulletin 2024/11**

(21) Application number: **22194919.1**

(22) Date of filing: **09.09.2022**

(51) International Patent Classification (IPC):
***A61K 49/00*** *(2006.01)* ***A61K 49/04*** *(2006.01)*
***A61K 49/10*** *(2006.01)* ***C07D 257/02*** *(2006.01)*

(52) Cooperative Patent Classification (CPC):
**A61K 49/105; A61K 49/0002; A61K 49/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer AG**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

(54) **COMBINATIONS OF CONTRAST AGENTS**

(57) The present invention relates to pharmaceutical compositions comprising contrast agents, to methods for preparing said pharmaceutical compositions and to their use for radiological imaging.

EP 4 335 461 A1

## Description

### FIELD OF THE INVENTION

The present invention relates to the items characterized in the patent claims, i.e. to pharmaceutical compositions comprising contrast agents, to methods for preparing said pharmaceutical compositions and to their use for radiological imaging.

### BACKGROUND

### Introduction

Magnetic resonance imaging (MRI) is powerful radiological technique for the visualization of structure and function of body tissues. MRI is used for medical diagnosis and treatment evaluation. The use of contrast agents can further enhance the diagnostic power of MRI.

Contrast agents alter the intrinsic tissue proton-relaxation T1 and T2 times resulting in an increase of the MRI contrast. Gadolinium-based Contrast Agents (GBCAs) contain at least one paramagnetic ion of the rare earth metal Gadolinium ($Gd^{3+}$), which possesses the highest number of unpaired electrons of any stable ion (seven), creating a high magnetic moment that is effective at enhancing proton relaxation. In GBCAs, the $Gd^{3+}$ ions are typically chelated with organic ligands. Based on the ligand structure of the molecule GBCAs are divided into linear and macrocyclic agents. This structure determines the complex stability and the stability in-vivo. The efficacy of GBCAs to increase the image contrast depends on their pharmacokinetics and the magnetic properties described by their r1 and r2 relaxivities. For marketed GBCAs the r1 relaxivity dominates resulting in a bright image contrast in T1-weighted MRI.

Following intravenous injection all marketed GBCAs distribute passively in the blood and the extracellular-extravascular space and are rapidly eliminated by the kidneys. Besides the unspecific - so-called extracellular agents - liver-specific contrast agents exist. These GBCAs are additionally taken up by hepatocytes and eliminated by the hepatobiliary system and the kidneys. Currently, two liver-specific GBCAs are available for MRI: gadobenic acid (Gd-BOPTA; maketed as Multihance) and gadoxetic acid (Gd-EOB DTPA; marketed as Primovist in Europe and as Eovist in the USA). Both agents have a linear molecular structure and are approved for the detection and differentiation of focal liver lesions. They can provide both dynamic and hepatobiliary phase imaging. The dynamic phase is used for the characterization of lesion vascularity during the arterial, portal-venous, and delayed imaging phase. The liver-specific contrast agents are taken up by healthy liver cells (hepatocytes) while there is no uptake into malignant tumor tissue due to the lack of intact organic anion-transporting polypeptide (OATP) transporters. Therewith, they improve the detection of focal liver lesions by increasing the lesion-to-liver contrast in the hepatobiliary imaging phase. The approved dose for the liver specific GBCAs is lower (0.025 mmol/kg and 0.05 mmol/kg for Primovist/Eovist and Multihance, respectively) than that of the extracellular agents (0.1 mmol/kg body weight). Compared to commercially available extracellular GBCAs, Gd-EOB-DTPA and Gd-BOPTA show higher r1 and r2 relaxivities (Rohrer M et al. Invest Radiol. 2005 Nov;40(11):715-24). The high relaxivity values depend on the affinity of the molecules with plasma proteins through the lipophilic group, which is also responsible for specific hepatocyte uptake.

Some recent reports have shown an increased signal intensity (SI) in the dentate nucleus and globus pallidus brain areas on unenhanced T1-weighted (T1w) MR images in patients with normal renal function who received multiple linear gadolinium-based contrast agent (GBCA) administrations. A visible SI increase is highly associated with linear GBCAs only. So far, this does not correlate with any clinical symptoms. However, in Europe, the European Medicines Agency (EMA) proposed to withdraw GBCAs with a linear structure from the market, except for both liver-specific market products Gd-BOPTA - if exclusively used in the liver - and Gd-EOB-DTPA due to safety concerns. In March 2016, the Pharmacovigilance Risk Assessment Committee (PRAC) of EMA started a procedure to review linear GBCAs and recommended, in March 2017, a suspension of the marketing authorization for all multi-purpose linear GBCAs in the European Union (EU) while supporting the continued use of macrocyclic GBCAs (i.e., contrast agents where the Gadolinium ion is bound to a macrocyclic ligand structure). Following this decision, the GBCA market has broadly moved away from linear Gadolinium-based agents. As of early 2022, liver specific GBCAs (Gd-EOB-DTPA and Gd-BOPTA) are still accepted, because no alternative liver specific macrocyclic GBCA is commercially available. The observed increased signal intensity in the dentate nucleus was also seen after repeated administration of the linear Gd-EOB-DTPA (Kahn J et al. Radiology. 2017;282(3):708 - 716). It is known that macrocyclic GBCAs are more stable against Gd release (Frenzel et al. Invest Radiol. 2008 Dec;43(12):817-28.) Thus, there is a pressing, increased and long standing medical need for the provision of macrocyclic liver-specific contrast agents.

Computed tomography is a non-invasive imaging technique to visualize anatomy and function of the human body using ionizing radiation. The signal intensity is based on the X-ray attenuation characteristics of the tissue and the contrast originates from differences in the ability to attenuate X-rays among different tissues. Unenhanced CT offers a

strong signal contrast between bones and soft tissues as the calcium in the bones attenuates X-rays effectively. A second strong CT contrast exist between soft tissue and air-containing structures as the attenuation of such structure as the respiratory system is rather low. On the other hand, the CT contrast between different soft tissue is low. Therefore, contrast media that locally increase the X-ray attenuation were used (Clauss W, Speck U. Historical development of x-ray contrast media for urography and angiography. In: Vogl T, Clauß W, Li GZ, et al., eds. Computed tomography Berlin Heidelberg, Germany: Springer; 1996:1-11).

All x-ray contrast media approved for intravascular use are iodine-containing monomeric or dimeric substances containing 1 or 2 triiodobenzene cores. These contrast media are formulated with high iodine concentration (150-400 mgI/mL) to enable sufficient attenuation and signal. They passively distribute only in the extracellular volume and are called nonspecific or extracellular contrast media.

Abdominal contrast enhanced CT with available iodinated contrast media is a major application, in especially the oncological field. For the detection and characterization of liver lesions multi-phasic contrast-enhanced CT scans are usually performed to image the distribution of contrast media in different phases of the blood circulation (e.g. arterial, portal-venous, venous). As hepatobiliary phase imaging is not possible, the diagnostic capabilities are limited to the native and dynamic contrast enhanced liver CT phases.

Targeted CT contrast media, comparable to the above-described linear liver specific GBCAs for MRI, are commercially not available. One reason is the lower contrast media sensitivity of CT compared to MRI. The amount of CM needed for a CT signal enhancement is order of magnitude higher (standard dose CT:300-600 mg Iodine/kg vs standard dose MRT: 0.025-0.1 mmol [3.9-15.7mg] Gd/kg).

Besides the differences in dosage of iodine and gadolinium based contrast agents both offer a high attenuation in the x-ray energy spectrum of CT. At identical mass-concentrations the x-ray attenuation of gadolinium is higher than that of iodine (Nowak et al. Med Phys. 2011, Dec 38(12):6469-82).

The medical need for CE-CT with a liver specific contrast agent is high. Multiphase abdominal CT is one of the most important CT applications in particular for oncological patients. In addition to diagnostics, CT offer the opportunity for image guided interventions as tissue biopsies or minimal invasive oncological treatment as RF-ablation. For these interventions tracking of the lesion during the procedure is essential. However, the available extracellular x-ray contrast media enhances the lesion only during a short time-window of the dynamic phase. A long-lasting contrast between lesion and liver would make that procedure much easier and has a great potential to increase the accuracy of targeted tissue sampling (biopsy) and therapeutic interventional treatment. For that purpose, a liver specific uptake of contrast media, resulting in an enhancement of healthy liver tissue during the hepatobiliary phase would be needed.

The issue is twofold: first the lower sensitivity of CT requires higher local contrast concentrations to generate the signal enhancement and second the limited uptake efficiency of the liver specific transports at higher contrast media doses. The lower sensitivity can be partially compensated by a higher dose. Thus, a macrocyclic structure is mandatory considering the SI increases in certain brain regions after repeated application of linear GBCAs. The second point, the limited uptake rates at higher doses can only be addressed by the molecule structure/configuration.

Thus, there is an increased medical need to provide new contrast agents for computed tomography imaging. In particular, there is a long-standing need to provide new contrast agents for computed tomography imaging which show advantageous properties similar or comparable to those of macrocyclic contrast agents for magnetic resonance imaging. More particularly, there is a long-standing medical need to provide new liver-specific contrast agents for computed tomography imaging.

### Description of the prior art, problem to be solved and its solution

Today, both groups of GBCAs, i.e. extracellular and liver-specific agents, are used for contrast-enhanced liver MRI. The extracellular agents provide a superior vascular contrast during the dynamic phase than the liver-specific agents as they are approved at a higher dose which outweighs the lower relaxivity. The higher vascular contrast in the dynamic imaging phase allows for a robust characterization of the contrast pattern in early enhancement of the liver lesion. Liver-specific agents have a weaker vascular contrast during the dynamic phase but offer a unique hepatocyte-specific contrast during the hepatobiliary phase. This allows for a robust detection of non-hepatocyte containing lesions and improves the detection and characterization of focal liver lesions and diffuse liver diseases.

Both dynamic phase, and hepatobiliary phase imaging are essential tools for liver MRI. Liver specific GBCAs can provide both dynamic and hepatobiliary phase imaging. To enhance the weaker vascular contrast during the dynamic phase, initial studies on patients have been carried out in which extracellular agents were additionally administered within a single MRI exam before or after the administration of (the liver-specific agent) Gd-EOB DTPA (Welle et al. Abdominal Radiology. 2021; (46)4588-4600). This sequential administration seems beneficial for certain indications; however, the cumulative gadolinium dose is high, and the two injections may result in a suboptimal contrast characteristic. Another approach describes a combined formulation of the liver-specific Gd-EOB-DTPA with a second contrast agent having renal excretion (WO 2017/093336). Renal excretion is a key characteristic of extracellular contrast agents. In

this combination approach the contrast of the dynamic vascular phase can be enhanced compared to a use of a single Gd-EOB-DTPA dose. In contrast to a sequential administration of an extracellular and liver specific GBCA the ratio of the two agents in the combined formulation and the total Gd dose can be adapted.

The described formulation is based on a combination of the liver-specific Gd-EOB-DTPA and a second contrast agent with renal elimination. Gd-EOB DTPA has a linear molecular structure and a lower in-vivo stability than GBCAs with a macrocyclic structure (Frenzel et al. Invest Radiol. 2008 Dec;43(12):817-28.). After repeated use of linear GBCAs increased signal intensity in the dentate nucleus and globus pallidus brain areas were found on unenhanced T1-weighted MRI. The observed increased signal intensity in the dentate nucleus was also seen after repeated administration of the linear Gd-EOB-DTPA (Kahn J et al. Radiology. 2017;282(3):708 - 716).

Thus, there is an urgent need for the provision of pharmaceutical compositions comprising liver-specific GBCAs and extracellular GBCAs which allow for simultaneous application of both GBCAs and which do not make use of linear liver-specific GBCAs. In particular, there is an urgent need for the provision of pharmaceutical compositions comprising liver-specific GBCAs and extracellular GBCAs which can be used in radiological imaging, such as MRI and/or CT.

Preferably, there is an urgent need for the provision of pharmaceutical compositions comprising liver-specific GBCAs and extracellular GBCAs in which the liver-specific GBCAs exhibit as many as possible of the following criteria:

- exhibit high water solubility,
- are chemically stable,
- are stable against metal release from the chelate,
- exhibit high relaxivity,
- exhibit high in vitro uptake into human transfected OATP1B1 HEK cells,
- exhibit high in vitro uptake into human transfected OATP1B3 HEK cells,
- have low protein binding,
- show a favorable pharmacokinetic profile and dual elimination pathway,
- are fast and completely excreted,
- exhibit no long-term retention of Gd3+ both in tissues and in organs,
- are stable against metabolic degradation,
- are well tolerated,
- are suitable for liver imaging,
- are suitable for biliary imaging,
- are suitable for the imaging of liver diseases
- and may exhibit high in vitro uptake (e.g. into rat hepatocytes).

Particularly preferred criteria for liver-specific GBCAs in pharmaceutical compositions comprising liver-specific GBCAs and extracellular GBCAs are those which

- exhibit high relaxivity and/or a high CT atenuation,
- exhibit high in vitro uptake into freshly isolated rat hepatocytes
- exhibit the potential of the preparation of combined formulations
- are suitable for liver and biliary imaging and

- are suitable for CT imaging.

It has been found that the pharmaceutical compositions of the present invention show surprising and advantageous properties which allow for the combination of non-linear liver-specific GBCAs with extracellular GBCAs thus removing the undesired use of linear GBCAs and the simultaneous application of both liver-specific and extracellular GBCAs in radiological imaging.

**DEFINITIONS**

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.
The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen or oxygen atom. Commonly, it is possible for the number of optional substituents, when present, to be 1, 2, 3, 4 or 5, in particular 1, 2 or 3.
As used herein, the term "one or more", e.g. in the definition of the substituents of the compounds of general formula (I) of the present invention, means "1, 2, 3, 4 or 5, particularly 1, 2, 3 or 4, more particularly 1, 2 or 3, even more particularly 1 or 2".
Should a composite substituent be composed of more than one part, *e.g.* ($C_1$-$C_4$-alkoxy)-($C_1$-$C_4$-alkyl)-, it is possible for a given part to be attached at any suitable position of said composite substituent, e.g. it is possible for the $C_1$-$C_4$-alkoxy part to be attached to any suitable carbon atom of the $C_1$-$C_4$-alkyl part of said ($C_1$-$C_4$-alkoxy)-($C_1$-$C_4$-alkyl)-group. A hyphen at the beginning or at the end of such a composite substituent indicates the point of attachment of said composite substituent to the rest of the molecule. Should a ring, comprising carbon atoms and optionally one or more heteroatoms, such as nitrogen, oxygen or sulfur atoms for example, be substituted with a substituent, it is possible for said substituent to be bound at any suitable position of said ring, be it bound to a suitable carbon atom and/or to a suitable heteroatom.
The term "comprising" when used in the specification includes "consisting of".
The term "halogen atom" means a fluorine, chlorine, bromine or iodine atom, particularly a fluorine, chlorine or bromine atom.
The term "$C_1$-$C_n$-alkyl" means a linear or branched, saturated, monovalent hydrocarbon group having n carbon atoms, n being a non-negative integer, n preferably being 1, 2, 3, 4, 5 or 6, i.e. a linear or branched, saturated, monovalent hydrocarbon group having 1, 2, 3, 4, 5 or 6 carbon atoms, *e.g.* a methyl, ethyl, propyl, isopropyl, butyl, sec-butyl, isobutyl, *tert*-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said "$C_1$-$C_n$-alkyl" group has 1, 2, 3 or 4 carbon atoms ("$C_1$-$C_4$-alkyl"), e.g. a methyl, ethyl, propyl, isopropyl, butyl, sec-butyl isobutyl, or *tert*-butyl group, more particularly 1, 2 or 3 carbon atoms ("$C_1$-$C_3$-alkyl"), e.g. a methyl, ethyl, n-propyl or isopropyl group.
The term "$C_1$-$C_n$-alkoxy" means a linear or branched, saturated, monovalent group of formula ($C_1$-$C_n$-alkyl)-O-, in which the term "$C_1$-$C_n$-alkyl" is as defined *supra, e.g.* a methoxy, ethoxy, n-propoxy, isopropoxy, n-butoxy, sec-butoxy, isobutoxy, *tert*-butoxy, pentyloxy, isopentyloxy or n-hexyloxy group, or an isomer thereof.
The term "$C_1$-$C_n$-haloalkyl" means a linear or branched, saturated, monovalent hydrocarbon group in which the term "$C_1$-$C_n$-alkyl" is as defined *supra,* and in which one or more of the hydrogen atoms are replaced, identically or differently, with a halogen atom. Particularly, said halogen atom is a fluorine atom. Said "$C_1$-$C_n$-haloalkyl" group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl.
The term "$C_3$-$C_5$-cycloalkyl" means a saturated, monovalent, mono- or bicyclic hydrocarbon ring which contains 3, 4 or 5 carbon atoms. Said "$C_3$-$C_5$-cycloalkyl" group is for example, a monocyclic hydrocarbon ring, e.g. a cyclopropyl, cyclobutyl or cyclopentyl.
In the context of the present invention, the term "macrocyclic" or "macrocycle" refers to a cyclic macromolecule, preferably to ring chelators (e.g. the twelve-membered ring cyclen, 1,4,7,10-tetraazacyclododecane). Macrocyclic complexes are known to have higher complex stability compared to those featuring acyclic, i.e. linear chelators/ligands (no cyclic / ring ligands).
In the context of the present invention, the term "chelating moiety" or "chelator" refers to a compound capable of binding to the metal by coordinative binding.
In the context of the present invention, the term "extracellular contrast agent" refers to a contrast agent having

a fast extracellular distribution in the body and, no hepatocellular uptake and almost exclusively and fast renal elimination.

In the context of the present invention, the term "liver-specific contrast agent" refers to a contrast agent having hepatocellular uptake and/or having billiary excretion. To differentiate these from extracellular contrast agents, liver-specific contrast agents display an intracellular uptake mediated by transporters (e.g. uptake via organic anion transporters OATPs and excretion via MRP2 transporters in the liver).

In the context of the present invention, the terms "comprising a paramagnetic metal ion and a macrocyclic chelating moiety" and/or "comprising a lanthanide metal ion and a macrocyclic chelating moiety" and/or "comprising a $Gd^{3+}$ metal ion and a macrocyclic chelating moiety" can be used interchangeably with the terms "comprising a metal ion complexed to a macrocyclic chelating moiety" and/or "comprising a lanthanide ion complexed to a macrocyclic chelating moiety" and/or "comprising a $Gd^{3+}$ ion complexed to a macrocyclic chelating moiety", respectively.

## DESCRIPTION OF THE INVENTION

In accordance with a first aspect, the present invention covers a pharmaceutical composition comprising

(i) an extracellular contrast agent; and

(ii) a liver-specific contrast agent comprising a paramagnetic metal ion and a macrocyclic chelating moiety.

Alternatively, in accordance with a first aspect, the present invention covers a pharmaceutical composition comprising

(i) an extracellular contrast agent; and

(ii) a liver-specific contrast agent comprising a paramagnetic metal ion complexed to a macrocyclic chelating moiety.

In accordance with a further embodiment of the first aspect, the present invention covers a pharmaceutical composition comprising

(i) an extracellular contrast agent comprising a paramagnetic metal ion and a macrocyclic chelating moiety; and

(ii) a liver-specific contrast agent comprising a paramagnetic metal ion and a macrocyclic chelating moiety.

In accordance with a further embodiment of the first aspect, the present invention covers a pharmaceutical composition comprising

(i) an extracellular contrast agent comprising a paramagnetic metal ion complexed to a macrocyclic chelating moiety; and

(ii) a liver-specific contrast agent comprising a paramagnetic metal ion complexed to a macrocyclic chelating moiety.

In accordance with a further embodiment of the first aspect, the present invention covers a pharmaceutical composition comprising

(i) an extracellular contrast agent comprising a lanthanide metal ion and a macrocyclic chelating moiety; and

(ii) a liver-specific contrast agent comprising a lanthanide metal ion and a macrocyclic chelating moiety.

In accordance with a further embodiment of the first aspect, the present invention covers a pharmaceutical composition comprising

(i) an extracellular contrast agent comprising a lanthanide metal ion complexed to a macrocyclic chelating moiety; and

(ii) a liver-specific contrast agent comprising a lanthanide metal ion complexed to a macrocyclic chelating moiety.

In accordance with a further embodiment of the first aspect, the present invention covers a pharmaceutical composition comprising

(i) an extracellular contrast agent comprising a $Gd^{3+}$ metal ion and a macrocyclic chelating moiety; and

(ii) a liver-specific contrast agent comprising a $Gd^{3+}$ metal ion and a macrocyclic chelating moiety.

In accordance with a further embodiment of the first aspect, the present invention covers a pharmaceutical composition comprising

(i) an extracellular contrast agent comprising a $Gd^{3+}$ metal ion complexed to a macrocyclic chelating moiety; and

(ii) a liver-specific contrast agent comprising a $Gd^{3+}$ metal ion complexed to a macrocyclic chelating moiety.

In accordance with a further embodiment of the first aspect, the present invention covers a pharmaceutical composition comprising

(i) an extracellular contrast agent comprising a $Gd^{3+}$ metal ion and a macrocyclic chelating moiety; and

(ii) a liver-specific contrast agent selected from a compound of formula (IV) and a compound of formula (V), the compound of formula (IV) having the formula

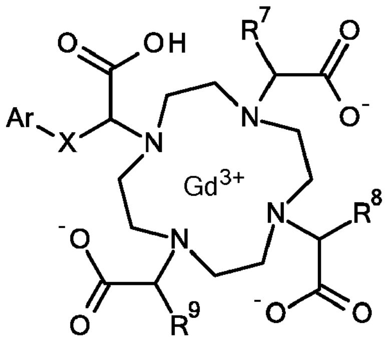

**(IV)** ,

wherein

Ar represents a group selected from

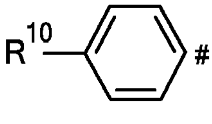

and

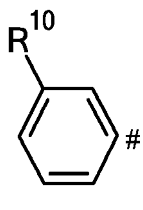

,

wherein # indicates the point of attachment to X,

X represents a group selected from $CH_2$ and $(CH_2)_3$,

$R^7$ and $R^9$ represent, independently for each occurrence, a hydrogen atom or a $-CH_2OH$ group,

$R^8$ represents a hydrogen atom or a group selected from $C_1$-$C_3$-alkyl, $-CH_2OH$, $-(CH_2)_2OH$ and $-CH_2OCH_3$,

$R^{10}$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

or a stereoisomer, a tautomer, an N-oxide, a hydrate, a solvate, or a salt thereof, or a mixture of same;

and the compound of formula (V) having the formula

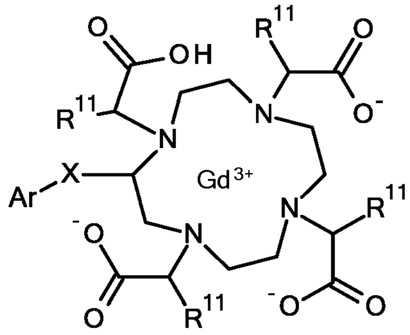

**(V)**

wherein

Ar represents a group selected from

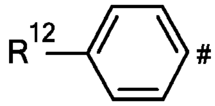

and

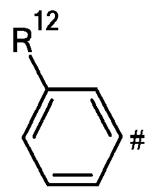

,

wherein # indicates the point of attachment to X,

X represents a group selected from $CH_2$ and $(CH_2)_2$;

$R^{11}$ represents a hydrogen atom or a group selected from $C_1$-$C_3$-alkyl, $-CH_2OH$, $-(CH_2)_2OH$ and $-CH_2OCH_3$,

$R^{12}$ represents a group selected from $C_2$-$C_5$-alkoxy, $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$ and $(H_3C\text{-}CH_2O)\text{-}(CH_2)_2\text{-}O\text{-}(CH_2)_2\text{-}O\text{-}(CH_2)_2\text{-}O\text{-}$,

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent selected from a compound of formula (I), a compound of formula (II) or a compound of formula (III), the compound of formula (I) having the formula

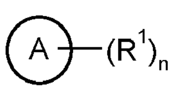

(I) ,

wherein:

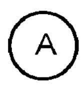

represents a

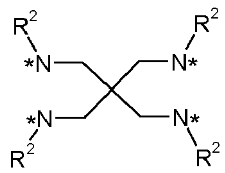

group, wherein * indicates the point of attachment of said group with $R^1$,

$R^1$ represents a group $R^3$,
n represents an integer of 4,
$R^2$ represents a hydrogen atom,
$R^3$ represents a group selected from

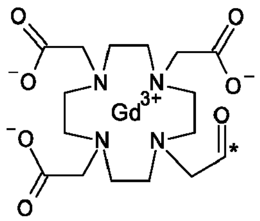

and

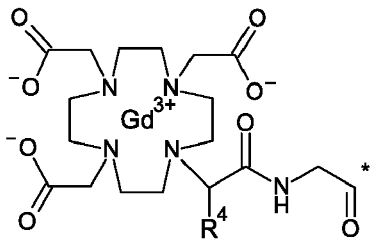

,

wherein * indicates the point of attachment of said group with the rest of the molecule,
$R^4$ represents a hydrogen atom or a methyl group,
or a stereoisomer, a tautomer, an N-oxide, a hydrate, a solvate, or a salt thereof, or a mixture of same;
the compound of formula (II) having the formula

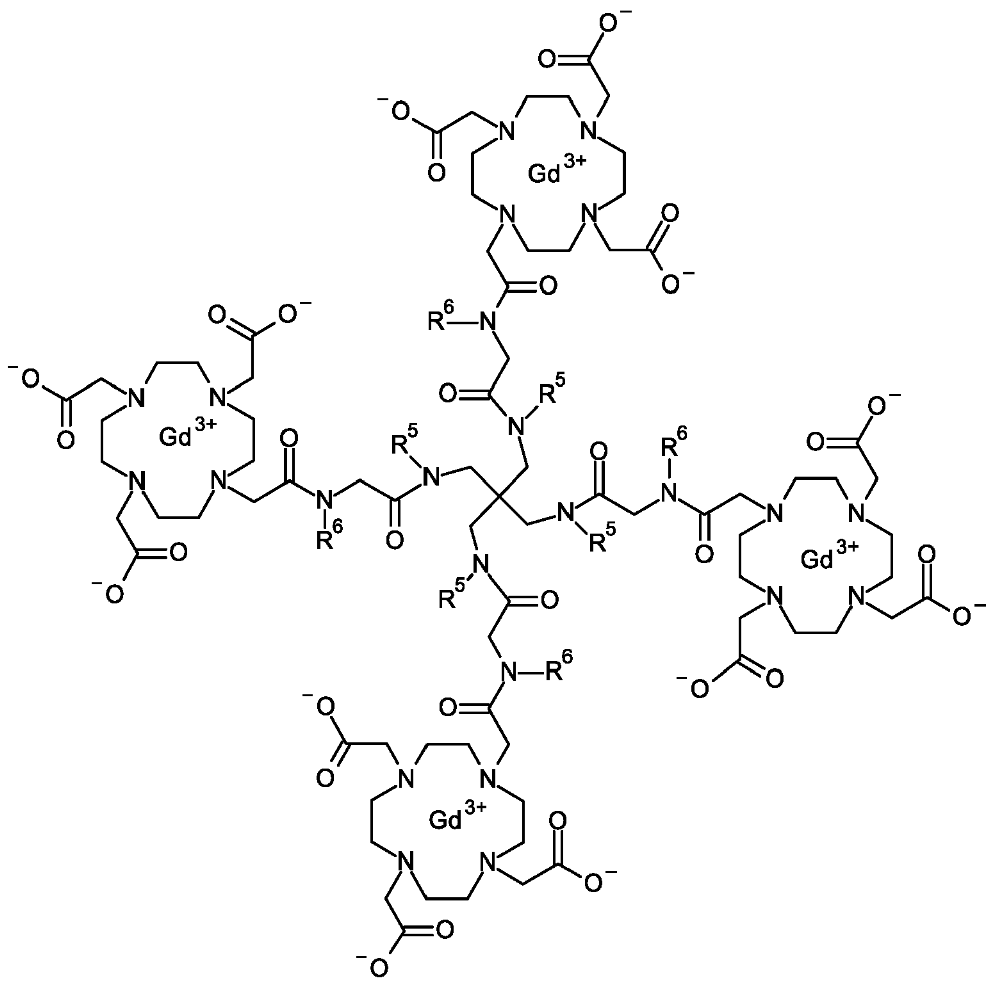

(II) ,

wherein
$R^5$ represents a hydrogen atom,
$R^6$ represents a group selected from
$C_1$-$C_4$-alkyl, $C_3$-$C_5$-cycloalkyl, ($C_1$-$C_2$-alkoxy)-($C_2$-$C_3$-alkyl)- and phenyl, wherein said $C_1$-$C_4$-alkyl group is optionally substituted, identically or differently, with a phenyl group, which phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy,
and

wherein said phenyl group is optionally substituted, one, two or three times, identically or differently, with a halogen atom or a group selected from
$C_1$-$C_3$-alkyl, $C_1$-$C_3$-haloalkyl and $C_1$-$C_3$-alkoxy or a stereoisomer, a tautomer, an N-oxide, a hydrate, a solvate, or a salt thereof, or a mixture of same;

and the compound of formula (III) having the formula,

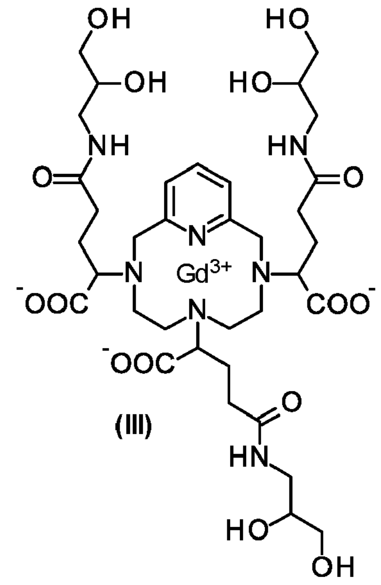

(III)

and

(ii) a liver-specific contrast agent selected from a compound of formula (IV) and a compound of formula (V), the compound of formula (IV) having the formula

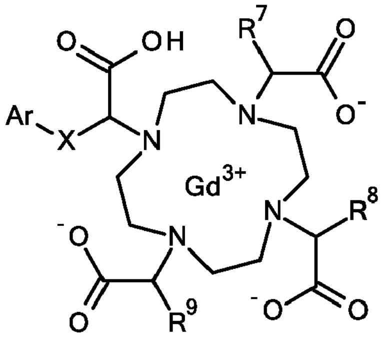

(IV)

,

wherein

Ar represents a group selected from

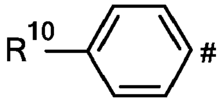

and

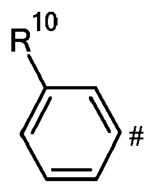

,

wherein # indicates the point of attachment to X,

X represents a group selected from $CH_2$ and $(CH_2)_3$,

$R^7$ and $R^9$ represent, independently for each occurrence, a hydrogen atom or a - $CH_2OH$ group,

$R^8$ represents a hydrogen atom or a group selected from $C_1$-$C_3$-alkyl, $-CH_2OH$, - $(CH_2)_2OH$ and $-CH_2OCH_3$,

$R^{10}$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

or a stereoisomer, a tautomer, an N-oxide, a hydrate, a solvate, or a salt thereof, or a mixture of same;

the compound of formula (V) having the formula

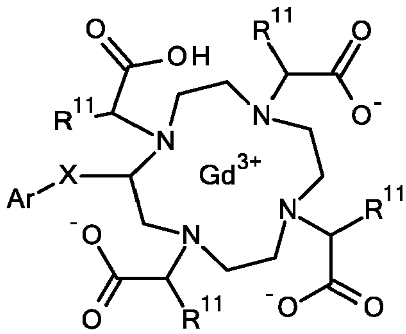

**(V)**

wherein

Ar represents a group selected from

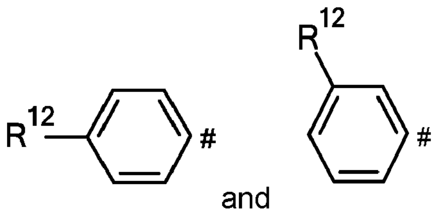

and ,

wherein # indicates the point of attachment to X,

X represents a group selected from $CH_2$ and $(CH_2)_2$;

$R^{11}$ represents a hydrogen atom or a group selected from $C_1$-$C_3$-alkyl, -$CH_2OH$, -$(CH_2)_2OH$ and -$CH_2OCH_3$;

$R^{12}$ represents a group selected from $C_2$-$C_5$-alkoxy, ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-;

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent selected from a compound of formula (I), a compound of formula (II) or a compound of formula (III), the compound of formula (I) having the formula

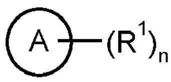

**(I)** ,

wherein:

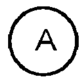

represents a

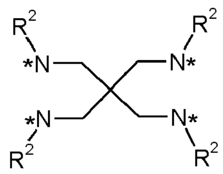

group, wherein * indicates the point of attachment of said group with $R^1$ ;

$R^1$ represents a group $R^3$,

n represents an integer of 4,

$R^2$ represents a hydrogen atom,

$R^3$ represents a group selected from :

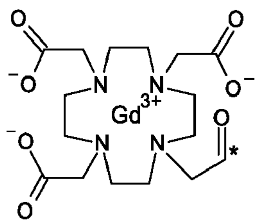

and

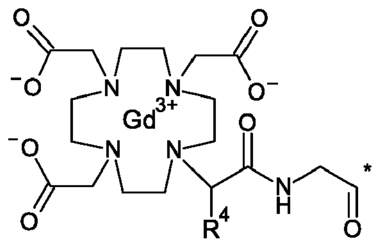

,

wherein * indicates the point of attachment of said group with the rest of the molecule,

$R^4$ represents a hydrogen atom or a methyl group

or a stereoisomer, a tautomer, an N-oxide, a hydrate, a solvate, or a salt thereof, or a mixture of same;

the compound of formula (II) having the formula

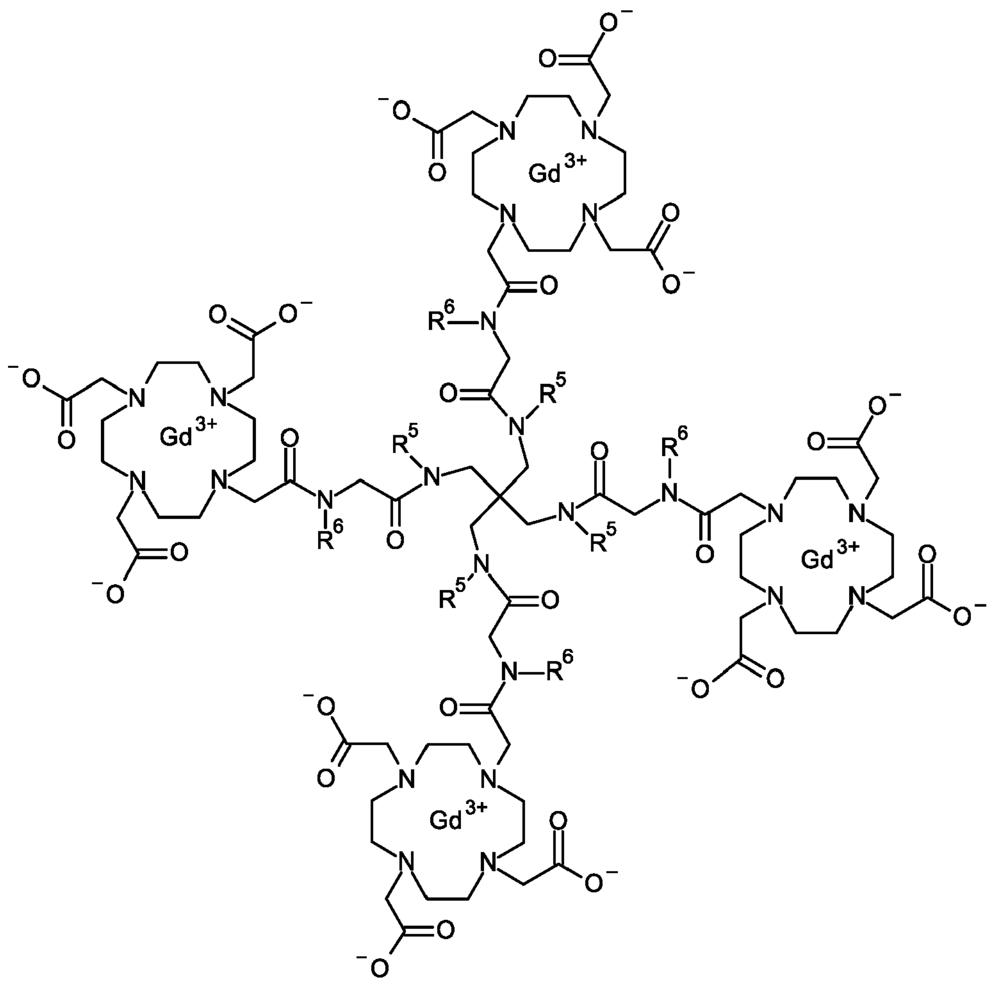

(II) ,

wherein

$R^5$ represents a hydrogen atom;

$R^6$ represents a group selected from:
$C_1$-$C_4$-alkyl, $C_3$-$C_5$-cycloalkyl, ($C_1$-$C_2$-alkoxy)-($C_2$-$C_3$-alkyl)- and phenyl,

wherein said $C_1$-$C_4$-alkyl group is optionally substituted, identically or differently, with a phenyl group, which phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy,
and
wherein said phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy or a stereoisomer, a tautomer, an N-oxide, a hydrate, a solvate, or a salt thereof, or a mixture of same;

the compound of formula (III) having the formula,

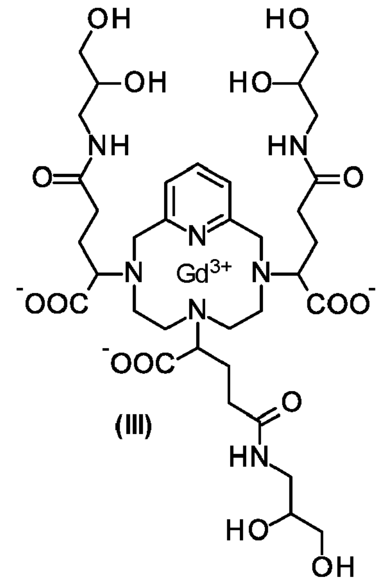

(III)

and

(ii) a liver-specific contrast agent of the formula (IV)

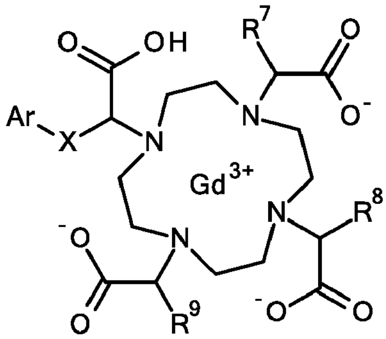

(IV)

,

wherein

Ar represents a group selected from

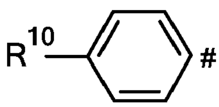

and

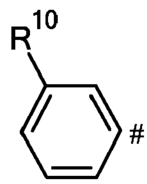

,

wherein # indicates the point of attachment to X,

X represents a group selected from $CH_2$ and $(CH_2)_3$,

$R^7$ and $R^9$ represent, independently for each occurrence, a hydrogen atom or a - $CH_2OH$ group,

$R^8$ represents a hydrogen atom or a group selected from $C_1$-$C_3$-alkyl, $-CH_2OH$, - $(CH_2)_2OH$ and $-CH_2OCH_3$,

$R^{10}$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

wherein said $C_1$-$C_3$-alkoxy groups and $C_2$-$C_5$-alkoxy groups are optionally substituted, one, two, three or four times, with a fluorine atom,

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent selected from a compound of formula (I) and a compound of formula (II), the compound of formula (I) having the formula

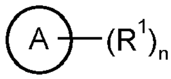

**(I)** ,

wherein:

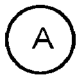

represents a

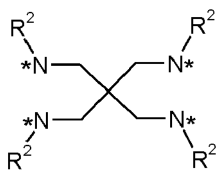

group, wherein * indicates the point of attachment of said group with $R^1$,

$R^1$ represents a group $R^3$,

n represents an integer of 4,

$R^2$ represents a hydrogen atom,

$R^3$ represents a group selected from

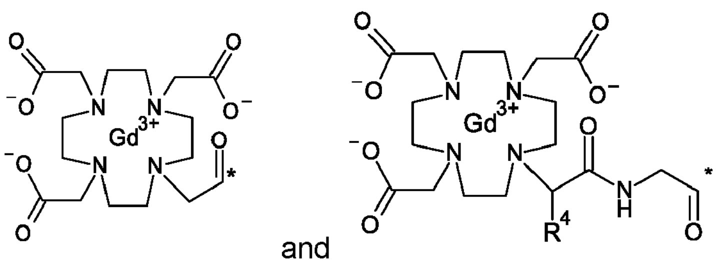

and ,

wherein * indicates the point of attachment of said group with the rest of the molecule,

$R^4$ represents a hydrogen atom or a methyl group, or a stereoisomer, a tautomer, an N-oxide, a hydrate, a

solvate, or a salt thereof, or a mixture of same;

the compound of formula (II) having the formula

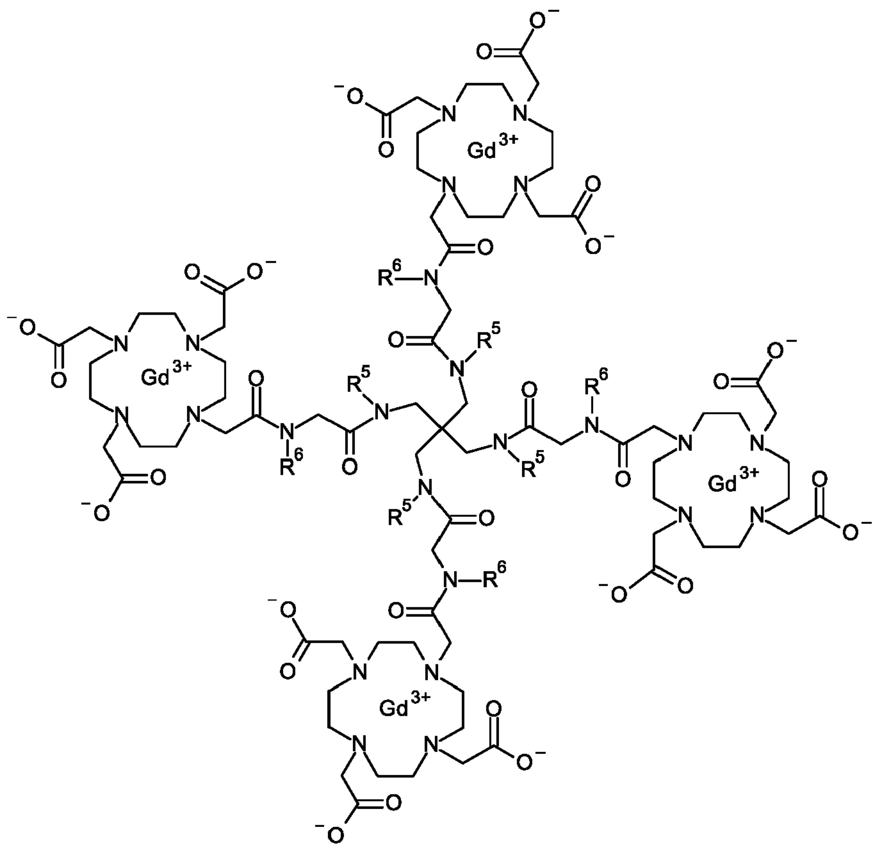

(II) ,

wherein

$R^5$ represents a hydrogen atom,

$R^6$ represents a group selected from
$C_1$-$C_4$-alkyl, $C_3$-$C_5$-cycloalkyl, ($C_1$-$C_2$-alkoxy)-($C_2$-$C_3$-alkyl)- and phenyl,

wherein said $C_1$-$C_4$-alkyl group is optionally substituted, identically or differently, with a phenyl group, which phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy,
and
wherein said phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy or a stereoisomer, a tautomer, an N-oxide, a hydrate, a solvate, or a salt thereof, or a mixture of same;

and
(ii) a liver-specific contrast agent selected from a compound of formula (IV) and a compound of formula (V), the compound of formula (IV) having the formula

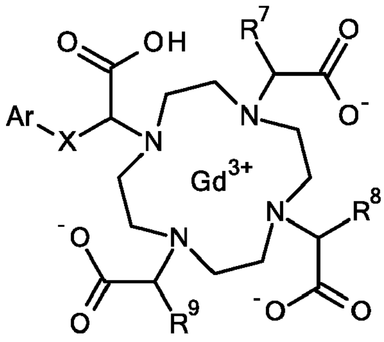

**(IV)**

,

wherein

Ar represents a group selected from

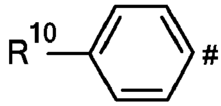

and

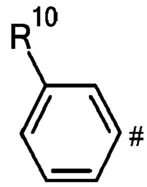

,

wherein # indicates the point of attachment to X,

X represents a group selected from $CH_2$ and $(CH_2)_3$,

$R^7$ and $R^9$ represent, independently for each occurrence, a hydrogen atom or a $-CH_2OH$ group,

$R^8$ represents a hydrogen atom or a group selected from $C_1$-$C_3$-alkyl, $-CH_2OH$, - $(CH_2)_2OH$ and $-CH_2OCH_3$,

$R^{10}$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

or a stereoisomer, a tautomer, an N-oxide, a hydrate, a solvate, or a salt thereof, or a mixture of same;

the compound of formula (V) having the formula

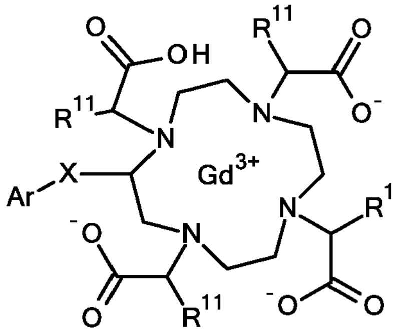

**(V)**

Ar represents a group selected from

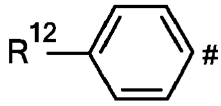

and

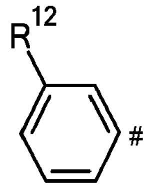

,

wherein # indicates the point of attachment to X,

X represents a group selected from $CH_2$ and $(CH_2)_2$,

$R^{11}$ represents a hydrogen atom or a group selected from $C_1$-$C_3$-alkyl, $-CH_2OH$, $-(CH_2)_2OH$ and $-CH_2OCH_3$;

$R^{12}$ represents a group selected from $C_2$-$C_5$-alkoxy, $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$ and $(H_3C\text{-}CH_2O)\text{-}(CH_2)_2\text{-}O\text{-}(CH_2)_2\text{-}O\text{-}(CH_2)_2\text{-}O\text{-}$;

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent of the formula (I)

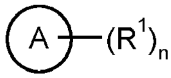

**(I)** ,

wherein:

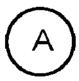

represents a

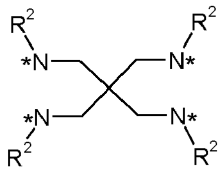

group, wherein * indicates the point of attachment of said group with $R^1$,

$R^1$ represents a group $R^3$,

n represents an integer of 4,

$R^2$ represents a hydrogen atom,

$R^3$ represents a group selected from

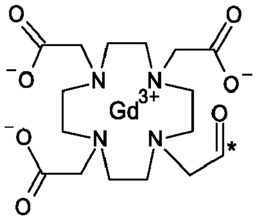

and

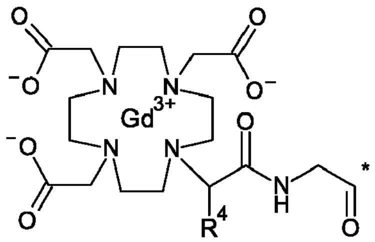

,

wherein * indicates the point of attachment of said group with the rest of the molecule,

$R^4$ represents a hydrogen atom or a methyl group

or a stereoisomer, a tautomer, an N-oxide, a hydrate, a solvate, or a salt thereof, or a mixture of same;

and

(ii) a liver-specific contrast agent of the formula (IV)

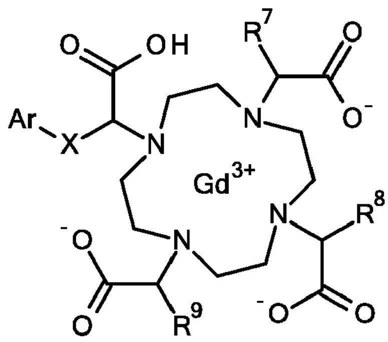

**(IV)**

,

wherein

Ar represents a group selected from

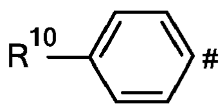

and

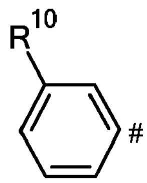

,

wherein # indicates the point of attachment to X,

X represents a group selected from $CH_2$ and $(CH_2)_3$,

$R^7$ and $R^9$ represent, independently for each occurrence, a hydrogen atom or a - $CH_2OH$ group,

$R^8$ represents a hydrogen atom or a group selected from $C_1$-$C_3$-alkyl, -$CH_2OH$, - $(CH_2)_2OH$ and -$CH_2OCH_3$,

$R^{10}$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent of the formula (I)

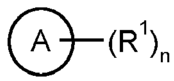

**(I)** ,

wherein:

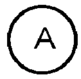

represents a

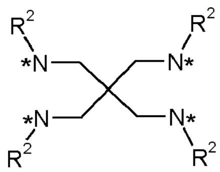

group, wherein * indicates the point of attachment of said group with $R^1$ ;

$R^1$ represents a group $R^3$,

n represents an integer of 4,

$R^2$ represents a hydrogen atom,

$R^3$ represents a group selected from

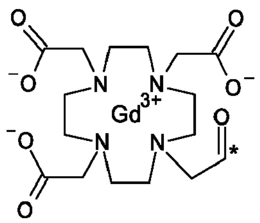

and

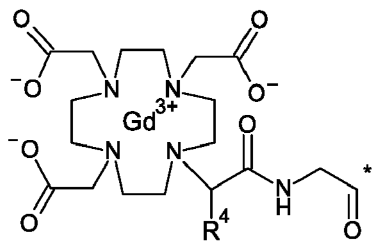

,

wherein * indicates the point of attachment of said group with the rest of the molecule,

$R^4$ represents a hydrogen atom or a methyl group

or a stereoisomer, a tautomer, an N-oxide, a hydrate, a solvate, or a salt thereof, or a mixture of same;

and

(ii) a liver-specific contrast agent of the formula (IV)

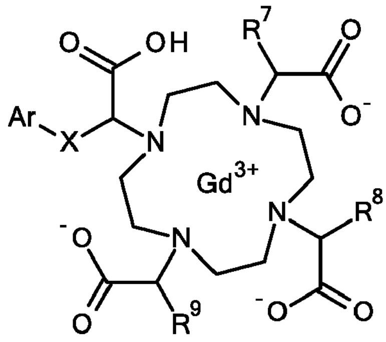

**(IV)** ,

wherein

Ar represents a group selected from

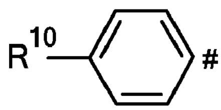

and

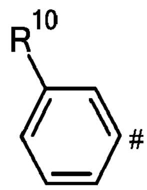

,

wherein # indicates the point of attachment to X,

X represents a group selected from $CH_2$ and $(CH_2)_3$,

$R^7$, $R^8$ and $R^9$ represent, independently for each occurrence, a hydrogen atom or a group selected from $C_1$-$C_3$-alkyl, $-CH_2OH$, $-(CH_2)_2OH$ and $-CH_2OCH_3$,

$R^{10}$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent of the formula (I)

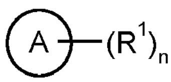

**(I)** ,

wherein:

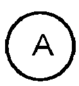

represents a

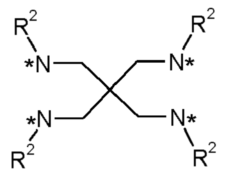

group, wherein * indicates the point of attachment of said group with $R^1$ ;

$R^1$ represents a group $R^3$,
n represents an integer of 4,
$R^2$ represents a hydrogen atom,
$R^3$ represents a group selected from

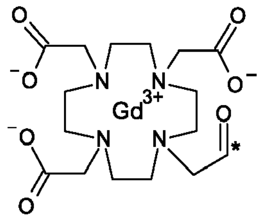

and

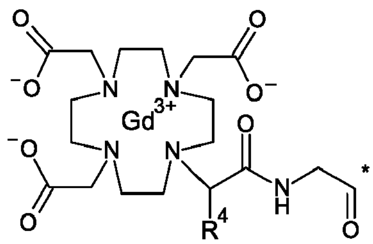

,

wherein * indicates the point of attachment of said group with the rest of the molecule,
$R^4$ represents a hydrogen atom or a methyl group or a stereoisomer, a tautomer, an N-oxide, a hydrate, a solvate, or a salt thereof, or a mixture of same;

and

(ii) a liver-specific contrast agent of the formula (IV)

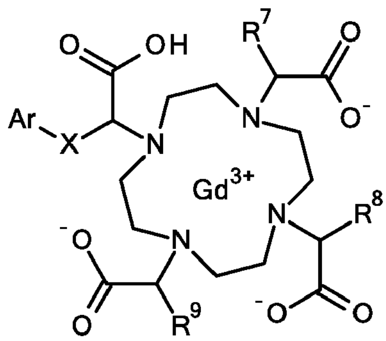

**(IV)**

,

wherein

Ar represents a group selected from

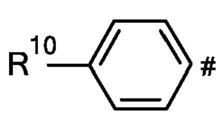

and

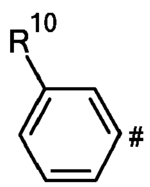

,

wherein # indicates the point of attachment to X,

X represents a group selected from $CH_2$ and $(CH_2)_3$,

$R^7$ and $R^9$ represent, independently for each occurrence, a hydrogen atom or a $-CH_2OH$ group,

$R^8$ represents a hydrogen atom or a group selected from $C_1$-$C_3$-alkyl, $-CH_2OH$, $-(CH_2)_2OH$ and $-CH_2OCH_3$,

$R^{10}$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

or a stereoisomer, a tautomer, an N-oxide, a hydrate, a solvate, or a salt thereof, or a mixture of same.

In accordance with a further embodiment of the first aspect, the present invention covers a pharmaceutical composition comprising

(i) an extracellular contrast agent of the formula

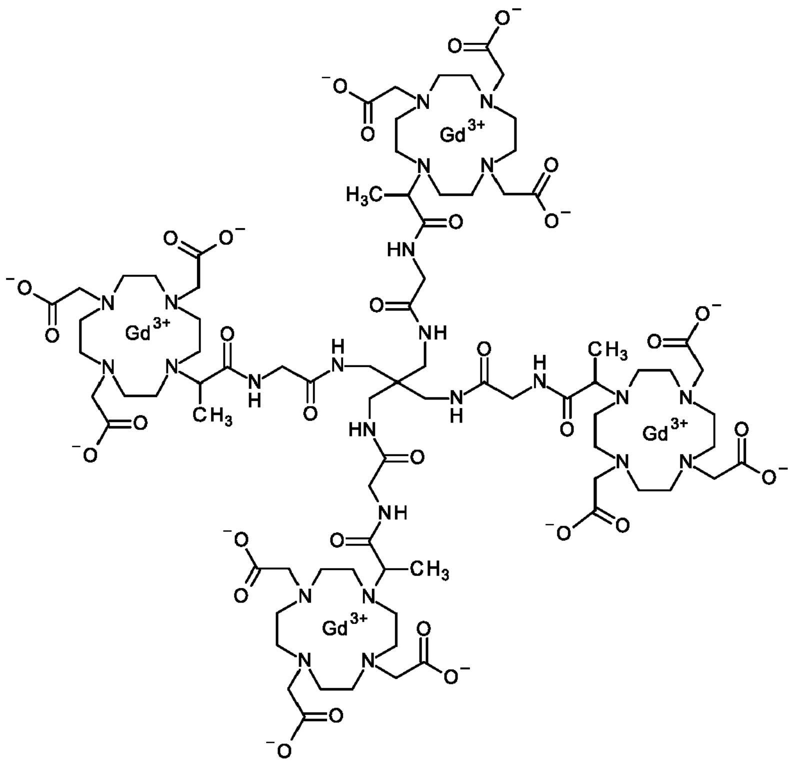

or a stereoisomer, a tautomer, an N-oxide, a hydrate, a solvate, or a salt thereof, or a mixture of same; and

(ii) a liver-specific contrast agent of the formula

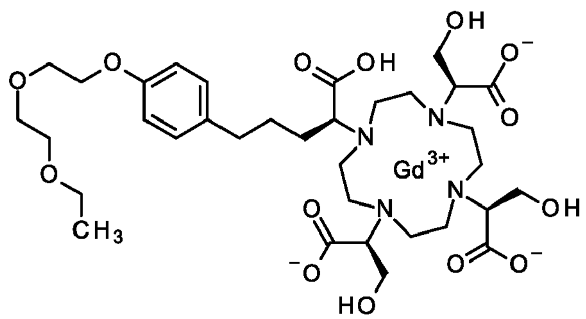

or a stereoisomer, a tautomer, an N-oxide, a hydrate, a solvate, or a salt thereof, or a mixture of same.

The pharmaceutical compositions of the present invention may comprise the contrast agents comprised therein in different ratios or proportions, as long as the resulting pharmaceutical compositions are suitable for the intended use. In particular, the pharmaceutical compositions of the present invention may comprise the contrast agents comprised therein in different ratios or proportions depending on the relaxivities shown by the individual components of said pharmaceutical compositions.

In accordance with one embodiment, the mole ratio of extracellular contrast agent to liver-specific contrast agent lies in the range of from 10:1 to 1:10.

In a preferred embodiment, the amounts of extracellular contrast agent and of liver-specific contrast agent lies are the same or an excess of liver-specific contrast agent is used. Thus, in a preferred embodiment, the mole ratio of extracellular contrast agent to liver-specific contrast agent lies in the range of from 1:1 to 1:10, preferably in the range of from 1:1 to 1:5, more preferably in the range of from 1:1 to 1:3.

Thus, the present invention covers a pharmaceutical composition comprising

(i) an extracellular contrast agent; and

(ii) a liver-specific contrast agent comprising a paramagnetic metal ion and a macrocyclic chelating moiety,

wherein the mole ratio of extracellular contrast agent to liver-specific contrast agent lies in the range of from 1:1 to 1:10, preferably in the range of from 1:1 to 1:5, more preferably in the range of from 1:1 to 1:3.

Further, the present invention covers a pharmaceutical composition comprising

(i) an extracellular contrast agent of the formula (I)

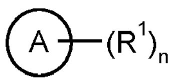

**(I)** ,

wherein:

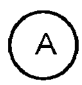

represents a

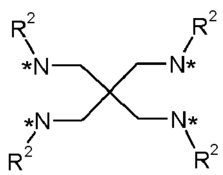

group, wherein * indicates the point of attachment of said group with $R^1$ ;

$R^1$ represents a group $R^3$,

n represents an integer of 4,

$R^2$ represents a hydrogen atom,

$R^3$ represents a group selected from

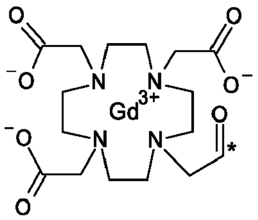

and

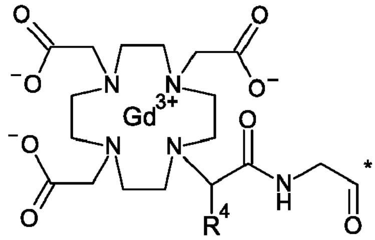

,

wherein * indicates the point of attachment of said group with the rest of the molecule,

$R^4$ represents a hydrogen atom or a methyl group

or a stereoisomer, a tautomer, an N-oxide, a hydrate, a solvate, or a salt thereof, or a mixture of same;

and

(ii) a liver-specific contrast agent of the formula (IV)

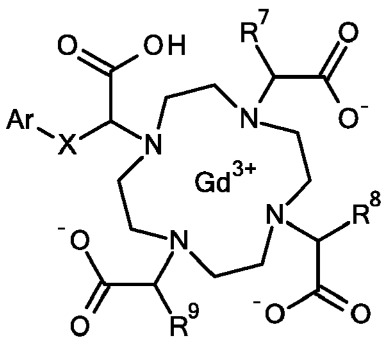

**(IV)**

,

wherein

Ar represents a group selected from

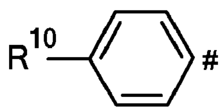

and

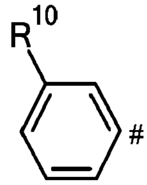

,

wherein # indicates the point of attachment to X,

X represents a group selected from $CH_2$ and $(CH_2)_3$,

$R^7$, $R^8$ and $R^9$ represent, independently for each occurrence, a hydrogen atom or a group selected from $C_1$-$C_3$-alkyl, $-CH_2OH$, $-(CH_2)_2OH$ and $-CH_2OCH_3$,

$R^{10}$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$, or a stereoisomer, a tautomer, an N-oxide, a hydrate, a solvate, or a salt thereof, or a mixture of same,

wherein the mole ratio of extracellular contrast agent to liver-specific contrast agent lies in the range of from 1:1 to 1:10, preferably in the range of from 1:1 to 1:5, more preferably in the range of from 1:1 to 1:3.

Further, the present invention covers a pharmaceutical composition comprising

(i) an extracellular contrast agent of the formula

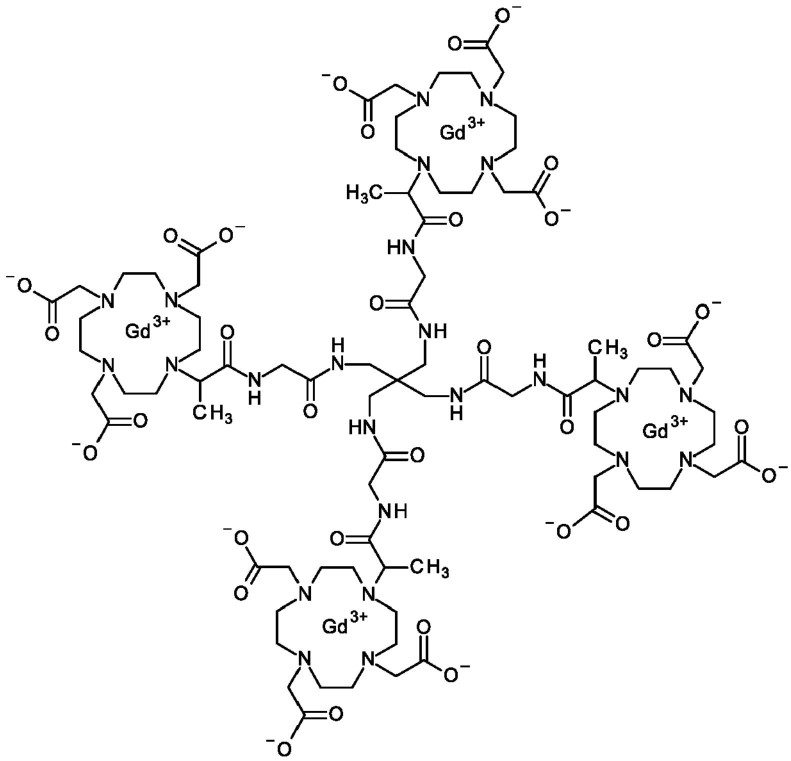

or a stereoisomer, a tautomer, an N-oxide, a hydrate, a solvate, or a salt thereof, or a mixture of same; and

(ii) a liver-specific contrast agent of the formula

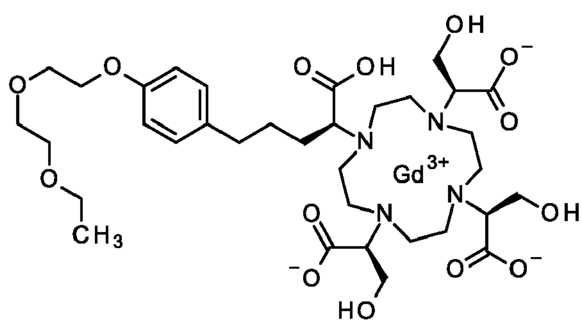

or a stereoisomer, a tautomer, an N-oxide, a hydrate, a solvate, or a salt thereof, or a mixture of same,

wherein the mole ratio of extracellular contrast agent to liver-specific contrast agent lies in the range of from 1:1 to 1:10, preferably in the range of from 1:1 to 1:5, more preferably in the range of from 1:1 to 1:3.

In a further preferred embodiment, the amounts of extracellular contrast agent and of liver-specific contrast agent lies are the same or an excess of extracellular contrast agent is used. Thus, in a preferred embodiment, the mole ratio of extracellular contrast agent to liver-specific contrast agent lies in the range of from 1:1 to 10:1, preferably in the range of from 1:1 to 5:1, more preferably in the range of from 1:1 to 3:1.

Thus, the present invention covers a pharmaceutical composition comprising

(i) an extracellular contrast agent; and

(ii) a liver-specific contrast agent comprising a paramagnetic metal ion and a macrocyclic chelating moiety,

wherein the mole ratio of extracellular contrast agent to liver-specific contrast agent lies in the range of from 1:1 to 10:1, preferably in the range of from 1:1 to 5:1, more preferably in the range of from 1:1 to 3:1.

Further, the present invention covers a pharmaceutical composition comprising

(i) an extracellular contrast agent of the formula (I)

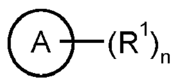

(I) ,

wherein:

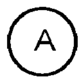

represents a

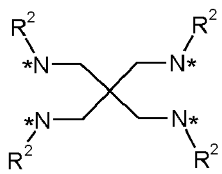

group, wherein * indicates the point of attachment of said group with $R^1$ ;

$R^1$ represents a group $R^3$,

n represents an integer of 4,

$R^2$ represents a hydrogen atom,

$R^3$ represents a group selected from

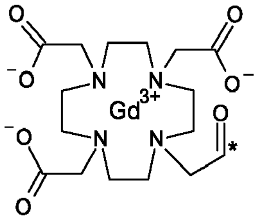 and 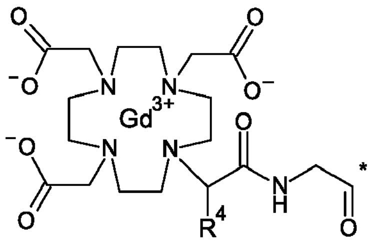 ,

wherein * indicates the point of attachment of said group with the rest of the molecule,

$R^4$ represents a hydrogen atom or a methyl group or a stereoisomer, a tautomer, an N-oxide, a hydrate, a solvate, or a salt thereof, or a mixture of same;

and

(ii) a liver-specific contrast agent of the formula (IV)

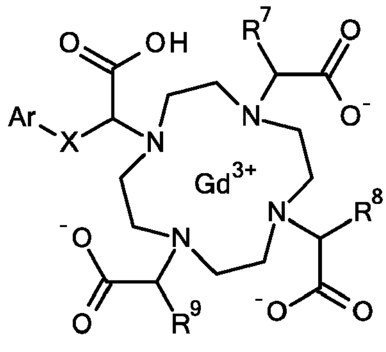

**(IV)** ,

wherein

Ar represents a group selected from

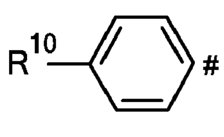 and 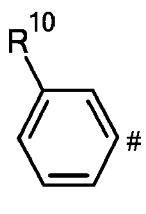 ,

wherein # indicates the point of attachment to X,

X represents a group selected from $CH_2$ and $(CH_2)_3$,

$R^7$, $R^8$ and $R^9$ represent, independently for each occurrence, a hydrogen atom or a group selected from $C_1$-$C_3$-alkyl, $-CH_2OH$, $-(CH_2)_2OH$ and $-CH_2OCH_3$,

$R^{10}$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$, or a stereoisomer, a tautomer, an N-oxide, a hydrate, a solvate, or a salt thereof, or a mixture of same

wherein the mole ratio of extracellular contrast agent to liver-specific contrast agent lies in the range of from 1:1 to 10:1, preferably in the range of from 1:1 to 5:1, more preferably in the range of from 1:1 to 3:1.

Further, the present invention covers a pharmaceutical composition comprising

(i) an extracellular contrast agent of the formula

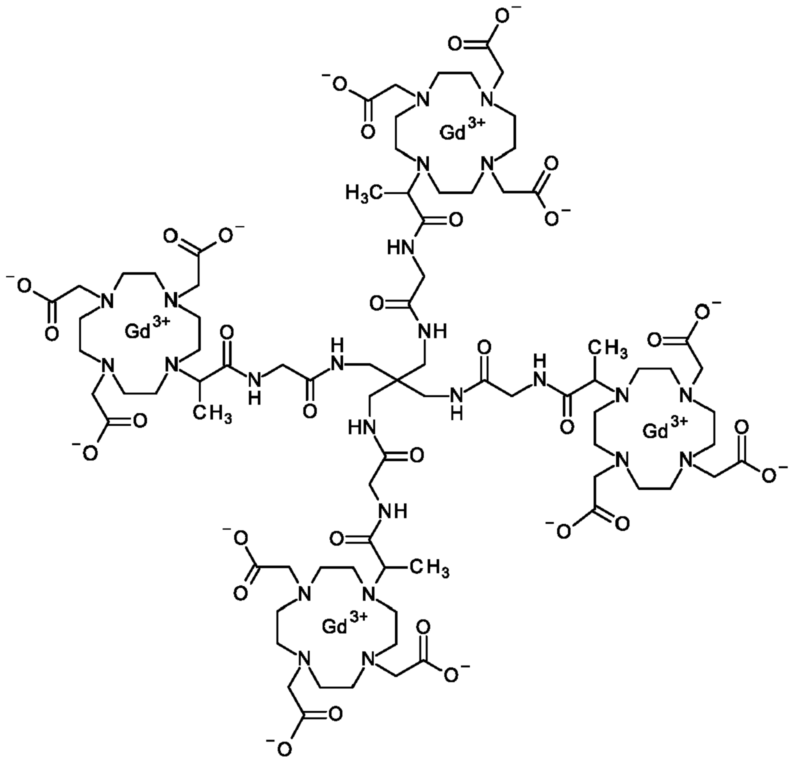

or a stereoisomer, a tautomer, an N-oxide, a hydrate, a solvate, or a salt thereof, or a mixture of same; and

(ii) a liver-specific contrast agent of the formula

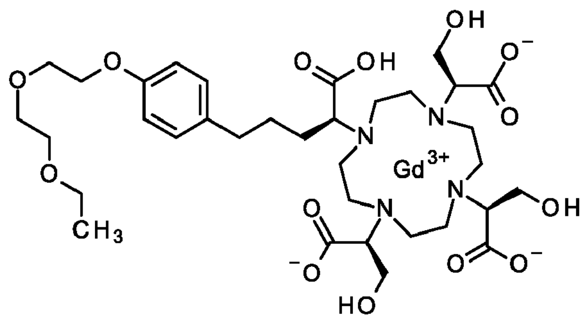

or a stereoisomer, a tautomer, an N-oxide, a hydrate, a solvate, or a salt thereof, or a mixture of same,

wherein the mole ratio of extracellular contrast agent to liver-specific contrast agent lies in the range of from 1:1 to 10:1, preferably in the range of from 1:1 to 5:1, more preferably in the range of from 1:1 to 3:1.

The pharmaceutical compositions of the present invention comprise contrast agents which show high relaxivity.

In particular, it is preferred that the extracellular contrast agent has a relaxivity higher than 3 $L{\cdot}mmol^{-1}{\cdot}s^{-1}$ when measured at 1.5 T in plasma or water at 37°C. Also preferably, the liver-specific contrast agent has a relaxivity higher than 4 $L{\cdot}mmol^{-1}{\cdot}s^{-1}$ when measured at 1.5 T in plasma or water at 37°C.

Thus, the present invention covers a pharmaceutical composition comprising

(i) an extracellular contrast agent having a relaxivity of at least 3 $L{\cdot}mmol^{-1}{\cdot}s^{-1}$ when measured at 1.5 T in water or

plasma; and

(ii) a liver-specific contrast agent comprising a paramagnetic metal ion and a macrocyclic chelating moiety having a relaxivity of at least 4 $L \cdot mmol^{-1} \cdot s^{-1}$ when measured at 1.5 T in water or plasma.

In accordance with another aspect, the present invention covers methods for the preparation of the pharmaceutical compositions of the present invention, said methods comprising the steps described in the Experimental Section herein.

In accordance with another aspect, the present invention covers the use of the pharmaceutical compositions of the present invention for radiological imaging, in particular for diagnostic imaging, more particularly for magnetic resonance imaging (MRI) or computer tomography (CT).

Preferably, the use of a pharmaceutical composition of the invention, *i.e.* of a pharmaceutical composition comprising (i) an extracellular contrast agent; and (ii) a liver-specific contrast agent comprising a paramagnetic metal ion and a macrocyclic chelating moiety, in the diagnosis is performed using magnetic resonance imaging (MRI) or computer tomography (CT).

Preferably, the use of a pharmaceutical composition of the invention, *i.e.* of a pharmaceutical composition comprising (i) an extracellular contrast agent; and (ii) a liver-specific contrast agent comprising a paramagnetic metal ion and a macrocyclic chelating moiety, in the diagnosis is performed using magnetic resonance imaging (MRI).

A further aspect of the invention is the use of a pharmaceutical composition of the invention, *supra,* for magnetic resonance imaging, preferably for oncological, neurological or cardiovascular indications and more preferably for MRI of the central nervous, vascular, renal or hepatobiliary system or of the gastrointestinal tract, more preferably for magnetic resonance imaging of the liver.

Preferably, the use of a pharmaceutical composition of the invention, *i.e.* of a pharmaceutical composition comprising (i) an extracellular contrast agent; and (ii) a liver-specific contrast agent comprising a paramagnetic metal ion and a macrocyclic chelating moiety, *supra,* in the diagnosis is performed using computer tomography (CT).

A further aspect of the invention is the use of a pharmaceutical composition of the invention, *supra,* for computer tomography imaging, preferably for oncological, neurological or cardiovascular indications and more preferably for CT of the central nervous, vascular, renal or hepatobiliary system or of the gastrointestinal tract, more preferably for computer tomography of the liver.

A further aspect of the invention comprises pharmaceutical compositions of the invention for use in diagnostic imaging.

A further aspect of the invention comprises pharmaceutical compositions of the invention for use in magnetic resonance imaging (MRI), preferably for oncological, neurological or cardiovascular indications and more preferably for MRI of the central nervous, vascular, renal or hepatobiliary system or of the gastrointestinal tract, more preferably for magnetic resonance imaging of the liver.

A further aspect of the invention comprises pharmaceutical compositions of the invention for use in magnetic computer tomography imaging, preferably for oncological, neurological or cardiovascular indications and more preferably for CT of the central nervous, vascular, renal or hepatobiliary system or of the gastrointestinal tract, more preferably for magnetic resonance imaging of the liver.

The invention also comprises pharmaceutical compositions of the invention for the manufacture of diagnostic agents.

A further aspect of the invention comprises pharmaceutical compositions of the invention for the manufacture of diagnostic agents.

A further aspect of the invention is the use of the pharmaceutical compositions of the invention for the manufacture of diagnostic agents for magnetic resonance imaging (MRI).

A further aspect of the invention is the use of the pharmaceutical compositions of the invention for the manufacture of diagnostic agents for magnetic resonance imaging (MRI) for oncological, neurological or cardiovascular indications and more preferably for MRI of the central nervous, vascular, renal or hepatobiliary system or of the gastrointestinal tract, preferably for magnetic resonance imaging of the liver.

A further aspect of the invention is the use of the pharmaceutical compositions of the invention for the manufacture of diagnostic agents for computer tomography (CT).

A further aspect of the invention is the use of the pharmaceutical compositions of the invention for the manufacture of diagnostic agents for computer tomography imaging for oncological, neurological or cardiovascular indications and more preferably for CT of the central nervous, vascular, renal or hepatobiliary system or of the gastrointestinal tract, preferably for magnetic resonance imaging of the liver.

A further aspect of the invention is a method of imaging body tissue in a patient, comprising the steps of administering to the patient an effective amount of a pharmaceutical composition of the invention in a pharmaceutically acceptable carrier, and subjecting the patient to magnetic resonance imaging (MRI).

A further aspect of the invention is a method of imaging body tissue in a patient, comprising the steps of administering to the patient an effective amount of a pharmaceutical composition of the invention in a pharmaceutically acceptable carrier, and subjecting the patient to computer tomography (CT).

For the manufacture of diagnostic agents, for example the administration to human or animal subjects, the pharmaceutical compositions of the invention will conveniently be formulated together with pharmaceutical carriers or excipients. The pharmaceutical compositions of the invention may conveniently contain pharmaceutical formulation aids, for example stabilizers, antioxidants, pH-adjusting agents, metal scavengers, electrolytes (e.g. sodium chloride), flavors and the like. The pharmaceutical compositions of the invention may be formulated for parenteral or enteral administration or for direct administration into body cavities. For example, parenteral formulations contain a sterile solution or suspension in a dose of 0.0001-5 mmol gadolinium/kg body weight, preferably 0.001-0.5 mmol gadolinium/kg body weight, more preferably 0.005-0.1 mmol gadolinium/kg body weight of the compound of formula (I) according to this invention. Thus, the pharmaceutical compositions of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

In a further aspect, the invention is directed to a method of diagnosing and health monitoring of patients. This method comprises a) administering to a human in need of such diagnosis a pharmaceutical composition of the invention for detecting the contrast agent in the human as described above and herein, and b) measuring the signal arising from the administration of the pharmaceutical composition to the human, preferably by magnetic resonance imaging (MRI).

In a further aspect, the invention is directed to a method of diagnosing and health monitoring of patients. This method comprises a) administering to a human in need of such diagnosis a pharmaceutical composition of the invention for detecting the contrast agent in the human as described above and herein, and b) measuring the signal arising from the administration of the pharmaceutical composition to the human, preferably by computer tomography imaging.

GENERAL SYNTHESIS

The synthesis of the compounds of formula (I) has been described, for example, in WO2016/193190, which is hereby incorporated by reference. The synthesis of the compounds of formula (II) has been described, for example, in WO2018/096082, which is hereby incorporated by reference. The synthesis of the compound of formula (III) has been described, for example, in WO2022/013454, which is hereby incorporated by reference. The synthesis of the compounds of formula (IV) has been described, for example, in PCT/EP2022/056541, which is hereby incorporated by reference.

The sequence depicted in Scheme 1 may be employed for the preparation of the compounds of general formula (IV) described in this invention, including the compounds of general formula (IV) in the form of a complex with $Gd^{3+}$, particularly when $R^8$ is different from $R^7$ and $R^9$ starting from the known 1,7-bis-Boc-protected cyclen [CAS no. 162148-48,3].

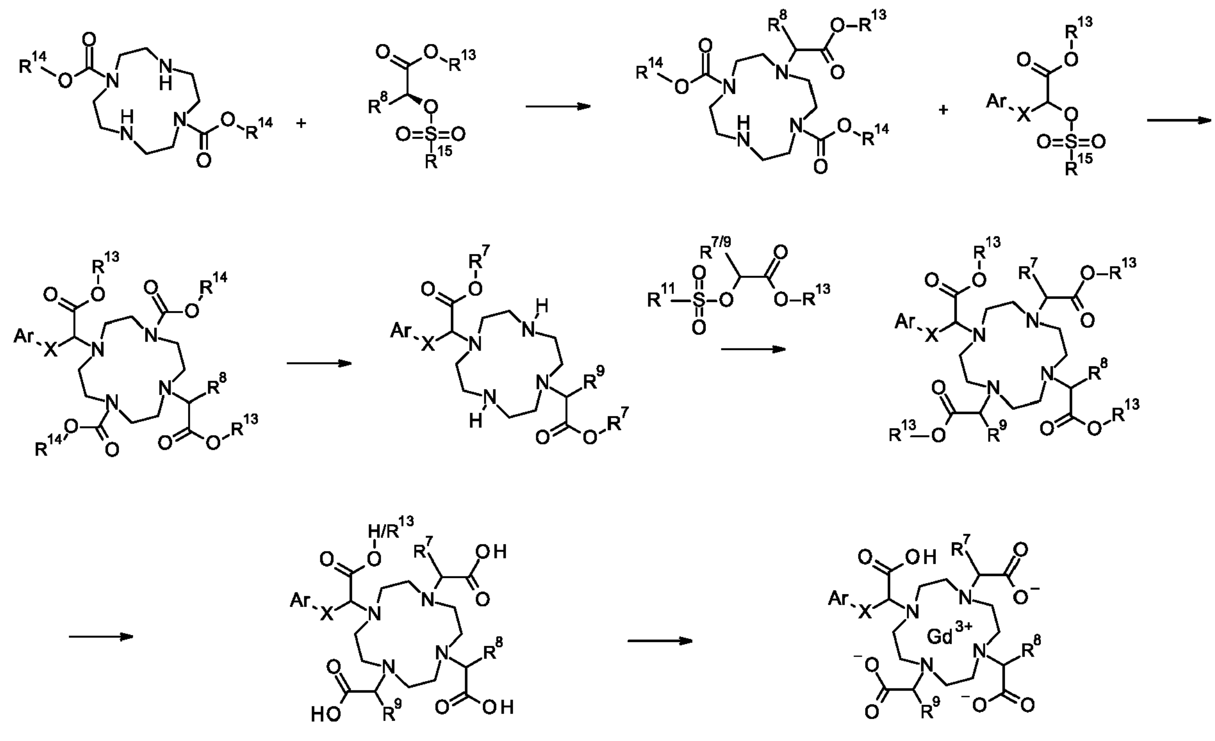

**Scheme 1.**

Alternatively, the sequence depicted in Scheme 2 may be employed for the preparation of the compounds of general formula (IV) described in this invention, including the compounds of general formula (IV) in the form of a complex with $Gd^{3+}$, particularly when $R^7 = R^8 = R^9$ = hydroxymethyl.

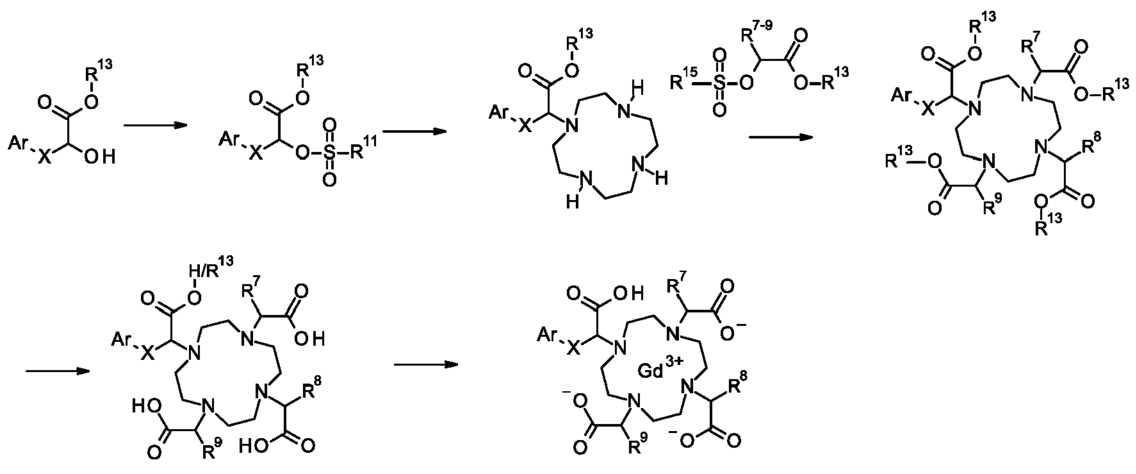

**Scheme 2.**

The compounds of general formula (V) described in this invention, including the compounds of general formula (V) in the form of a complex with $Gd^{3+}$ can be produced using the following general procedures depicted in Schemes 3 and 4 below.

For example, the compounds of general formula (V) described in this invention, including the compounds of general formula (V) in the form of a complex with $Gd^{3+}$ can be prepared by the procedure depicted in Scheme 3, where Ar-X-substituted cyclen is formed by an acid catalyzed tetramerization of benzylaziridines followed by benzyl deprotection.

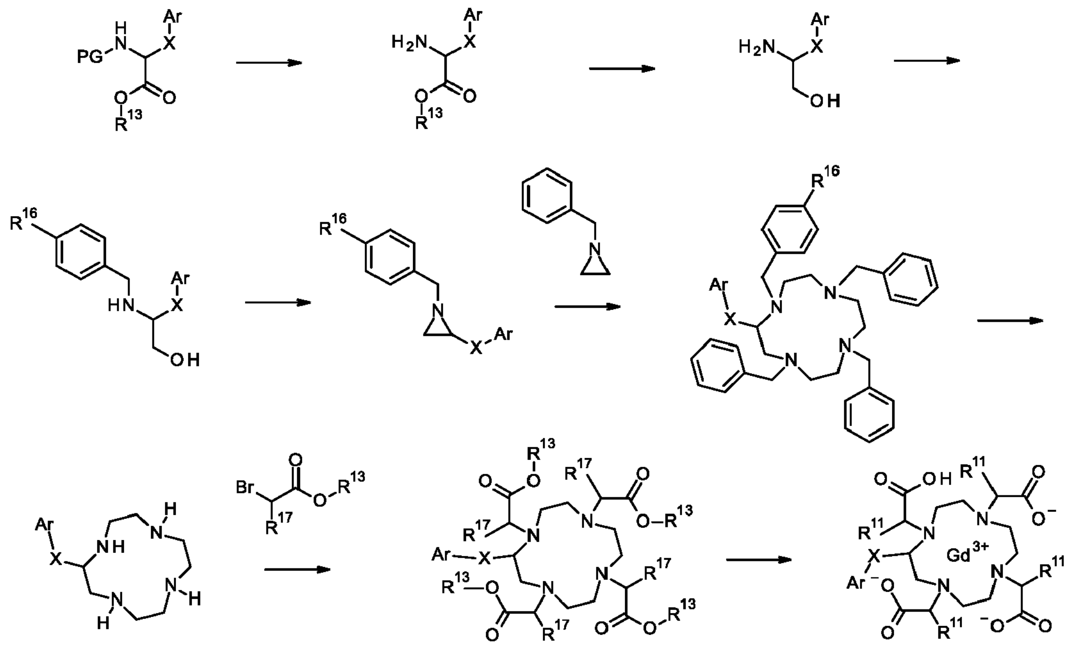

**Scheme 3.**

Alternatively, the compounds of general formula (V) described in this invention, including the compounds of general formula (V) in the form of a complex with $Gd^{3+}$, particularly when $R^{11}$ = hydrogen, can be prepared according to the procedure depicted in Scheme 4, where Ar-X-substituted cyclen is formed by diamide formation followed by amide reduction.

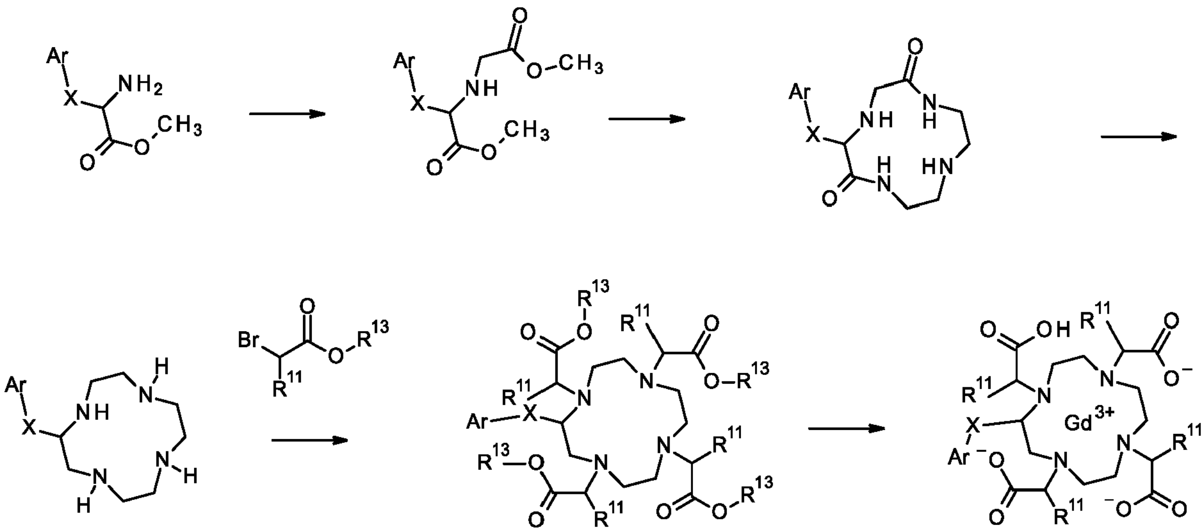

**Scheme 4.**

Literature for the synthetic procedures shown in scheme 3 and 4 can be found in, for example, L. Dai, J. Zhang, Y. Chen,L.E. Mackenzie, R. Pal, and G.-L. Law Inorg. Chem. 2019, 58, 12506-12510, Q. Yuan , P. Xue , M. Fang , E. Fu and C. Wu Synthetic Communications 2003, 33:11, 1911-1916, WO 2019/051197 and WO 97/31905.

Unless otherwise specified, the groups $R^7$ to $R^{11}$ displayed therein have the meaning given in the description above. In case protective group chemistry is required for the introduction of one or more of the groups $R^{11}$, the groups $R^{17}$ can be employed. Thus, should no protective group chemistry be needed, the group $R^{17}$ equals the group $R^{11}$, In general, but not exclusively, the following groups are selected from $R^{13}$ = methyl, ethyl or *tert*-butyl, $R^{14}$ = benzyl or *tert*-butyl, $R^{15}$ = methyl, trifluoromethyl or 4-methylphenyl and $R^{16}$ = methoxy, trifluoromethoxy.

The schemes and procedures described above illustrate synthetic routes to the compounds of general formula

(IV) and (V) of the invention and are not intended to be limiting. It would be apparent to the person skilled in the art that the order of transformations as exemplified in the schemes can be modified in various ways. The order of transformations exemplified in the schemes is therefore not intended to be limiting. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

The contents of the documents which are cited herein are hereby incorporated by reference By way of example, detailed syntheses for representative compounds of the formula (IV) and for compounds of the formula (V) are given in the Experimental Section below.

## DESCRIPTION OF THE FIGURES

**Figure 1:** Computed tomography images of the test sample of formulation A (left) and B (right) placed in the center of a tissue equivalent body phantom.

**Figure 2:** Relative MRI signal enhancement in minipigs after injection of the formulations Combi1 (0.02 mmol Gd/kg) and LS-GBCA 2. For the combination of extracellular and liver-specific GBCAs (Combi1) the signal enhancement in blood (A) is significantly higher, that is particularly relevant during the dynamic imaging phase (0-4 min). In the liver (B) the clinically relevant signal enhancement in the hepatobiliary phase (15-30 min) is similar for both formulations.

**Figure 3:** Relative MRI signal enhancement in minipigs after injection of the formulations Combi1 (0.04 mmol Gd/kg), Combi2 and LS-GBCA1. For the combinations of extracellular and liver-specific GBCAs (Combi1 and Combi2) the signal enhancement in blood (A) is significantly higher, that is particularly relevant during the dynamic imaging phase (0-4 min). In the liver (B) the clinically relevant signal enhancement in the hepatobiliary phase (15-30 min) is comparable for all three formulations.

## EXPERIMENTAL SECTION

| | |
|---|---|
| ACN | acetonitrile |
| aq. | Aqueous |
| AUC | area under the curve |
| bw | body weight |
| CAIPIRINIA | Controlled Aliasinq in Parallel Imaging Results in Higher Acceleration |
| calc. | calculated |
| $CDCl_3$ | chloroform-d |
| CGd | concentration of the compound normalized to the Gadolinium |
| $CHCl_3$ | Chloroform |
| Cltot | total clearance |
| Combi | Combination of liver specific and ectracellular contrast agent |
| Conc. | Concentrated |
| CPMG | Carr-Purcell-Meiboom-Gill (MRI sequence) |
| CV | Column volume(s) |
| d | day(s) |
| $D_2O$ | deuterium oxide |
| DAD | diode array detection/detector |
| DCM | dichloromethane |
| DEA | diethylamine |
| DIPEA | N,N-diisopropylethylamine |

(continued)

| | |
|---|---|
| DMSO | dimethylsulfoxide |
| DMSO-d6 | deuterated dimethylsulfoxide |
| e.e. | enantiomeric excess |
| ECCM | extracellular contrast media |
| EI | electron ionisation |
| ELSD | evaporative liqht scattering detection/detector |
| ESI | electrospray ionisation |
| EtOAc | ethyl acetate |
| EtOH | ethanol |
| FBS | fetal bovine serum |
| FLASH | Fast Low Anqle Shot |
| GBCA | Gadolinium based contrast agent |
| $Gd_2O_3$ | Gadolinium oxide |
| h | hour(s) |
| $H_2$ | Hydrogen |
| $H_2SO_4$ | Sulfuric acid |
| HCC | hepatocellular carcinoma |
| HCl | Hydrochloric acid |
| HCOOH | formic acid |
| HPLC | high performance liquid chromatography |
| HU | Hounsfield units |
| ICP-MS | inductively coupled plasma mass spectrometry |
| IR | inversion recovery |
| IS | Internal standard |
| $K_2CO_3$ | potassium carbonate |
| $K_3PO_4$ | potassium phosphate |
| kDa | kilo Dalton |
| LCMS | liquid chromatography-mass spectroscopy |
| LS-GBCA | liver specific GBCA |
| MeCN | acetonitrile |
| MeOH | methanol |
| min | minute(s) |
| MIPS | Maximum Intensity Projections (MRI Imaging Projection) |
| MRI | magnetic resonance imaqinq |
| MRT | mean residence time |
| MS | mass spectrometry |
| MsCl | Methanesulfonyl chloride |
| MTBE | methyl-tert.-butylether |

(continued)

| | |
|---|---|
| $N_2$ | Nitrogen |
| $Na_2SO_4$ | sodium sulfate |
| NaCl | sodium chloride |
| $NaHCO_3$ | sodium bicarbonate |
| NaOH | sodium hydroxide |
| NMR | nuclear magnetic resonance spectroscopy: chemical shifts (δ) are given in ppm. (br = broad, d=doublet, t=triplet, q=quartet, quin=quintet, sxt= sextet, m=multiplet, mc=multiplet centrosymmetric). |
| PE | petroleum ether |
| RC | reference compound |
| Ri | (where i=1, 2) relaxation rates (1/T1,2) |
| ri | (where i=1, 2) relaxivities in L mmol-1 s-1 |
| Ri(0) | relaxation rate of the respective solvent |
| Rt | retention time |
| RT | room temperature |
| s | second(s) |
| sat. | saturated |
| $SiO_2$ | silicium dioxide |
| T | Tesla |
| t½ γ | plasma half-life, compartment V3 |
| t½ α | plasma half-life, compartment V1 |
| t½ β | plasma half-life, compartment V2 |
| T1,2 | relaxation time |
| TEA | triethylamine |
| TFA | trifluoroacetic acid |
| THF | tetrahydrofuran |
| TI | inversion time |
| TLC | thin layer chromatography |
| UPLC | ultra performance liquid chromatography |
| V1 + V2 | volume, compartments V1+V2 |
| VIBE | Volumetric Interpolated Breath-hold Examination |
| Vc (V1) | volume, central compartment V1 |
| Vd,ss | volume of distribution at steady state |
| [xxx-xx-x] | denotes Chemical Abstracts Registry Numbers |

**Materials and Instrumentation**

The chemicals used for the synthetic work were of reagent grade quality and were used as obtained.

All reagents, for which the synthesis is not described in the experimental section, are either commercially available, or are known compounds or may be formed from known compounds by known methods by a person skilled in the art.

$^1H$-NMR spectra were measured in $CDCl_3$, $D_2O$ or DMSO-$d_6$, respectively (room temperature, Bruker Avance

400 spectrometer, resonance frequency: 400.20 MHz for $^1H$ or Bruker Avance 300 spectrometer, resonance frequency: 300.13 MHz for $^1H$. Chemical shifts are given in ppm relative to sodium (trimethylsilyl)propionate-$d_4$ ($D_2O$) or tetramethylsilane (DMSO-$d_6$) as external standards ($\delta$ = 0 ppm).

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartridges KP-Sil® or KP-NH® in combination with a Biotage autopurifier system (SP4® or Isolera Four®) and eluents such as gradients of hexane/ethyl acetate or DCM/methanol. Especially advantageous in some cases is the use of (preparative) reversed phase C18 columns in combination with acetonitrile/water or methanol/water mixtures, which might be modified with acid or base as necessary. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

Examples were analyzed and characterized by the following HPLC based analytical methods to determine characteristic retention time and mass spectrum:

## Example Compounds

**Method 1:** Instrument: Waters Acquity UPLCMS SingleQuad; Column: Acquity UPLC BEH C18 1.7 $\mu$m, 50×2.1mm; eluent A: water + 0.1 vol % formic acid (99%), eluent B: MeCN; gradient: 0-1.6min 1-99% B, 1.6-2.0min 99% B; flow 0.8 ml/min; temperature: 60°C; DAD scan: 210-400 nm.

**Method 2:** Instrument: Waters Acquity UPLCMS SingleQuad; Column: Acquity UPLC BEH C18 1.7 $\mu$m, 50×2.1mm; eluent A: water + 0.1 vol-% formic acid (99%), eluent B: MeCN; gradient: 0-1.7min 1-45% B, 1.7-1.72min 45-99% B, 1.72-2.0min 99% B; flow 0.8 ml/min; temperature: 60°C; ELSD.

**Method 3:** Instrument: Agilent 1290 UPLCMS 6230 TOF; column: BEH C 18 1.7 $\mu$m, 50x2.1mm; Eluent A: water + 0.05 % formic acid (99%); Eluent B: MeCN + 0.05 % formic acid (99%); gradient: 0-1.7min 2-90% B, 1.7-2.0min 90% B; flow 1.2 ml/min; temperature: 60°C; DAD scan: 190-400 nm.

**Method 4:** Instrument: Waters Acquity UPLCMS SingleQuad; Column: Acquity UPLC BEH C18 1.7 $\mu$m, 50×2.1mm; eluent A: water + 0.2 vol % aqueous ammonia (32%), eluent B: MeCN; gradient: 0-1.6min 1-99% B, 1.6-2.0min 99% B; flow 0.8 ml/min; temperature: 60°C; DAD scan: 210-400 nm.

**Method 5:** Instrument: Waters Acquity UPLCMS SingleQuad; Column: Acquity UPLC BEH C18 1.7 $\mu$m, 50×2.1mm; eluent A: water + 0.1 vol-% formic acid (99%), eluent B: MeCN; gradient: 0-1.7min 1-45% B, 1.7-1.72min 45-99% B, 1.72-2.0min 99% B; flow 0.8 ml/min; temperature: 60°C; ELSD.

**Method 6:** Instrument: Waters Acquity UPLCMS SingleQuad; Column: Acquity UPLC BEH C18 1.7 $\mu$m, 50x2.1mm; eluent A: water + 0.2 vol-% aqueous ammonia (32%), eluent B: MeCN; gradient: 0-1.7min 1-45% B, 1.7-1.72min 45-99% B, 1.72-2.0min 99% B; flow 0.8 ml/min; temperature: 60°C; ELSD.

**Method 7:** HPLC instrument type: SHIMADZU LC-20AD; column: Kinetex C18 LC Column 4.6X50mm,5um; mobile phase A: 0.0375% TFA in water (v/v), B: 0.01875% TFA in MeCN (v/v); gradient: 0.0min 0% $\beta\rightarrow$ 4.2min 60% $\beta\rightarrow$ 5.3min 60% $\beta\rightarrow$ 5.31min 0% $\beta\rightarrow$ 6.0min 0% B; flow rate: 1.5 mL/min; oven temperature: 50°C; UV detection: 220 nm & 254 nm & 215 nm.

**Method 8:** MS instrument type: SHIMADZU LCMS-2020; Kinetex EVO C18 2.1X30mm,5um; mobile phase A: 0.0375% TFA in water (v/v), B: 0.01875% TFA in MeCN (v/v); gradient: 0.0min 5% $\beta\rightarrow$ 0.8min 95% $\beta\rightarrow$ 1.2min 95% $\beta\rightarrow$ 1.21min 5% $\beta\rightarrow$ 1.55min 5% B; flow rate: 1.5 mL/min; oven temperature: 50°C; UV detection: 220 nm & 254 nm.

**Method 9:** MS instrument type: SHIMADZU LCMS-2020; column: Kinetex EVO C18 2.1X30mm,5um; mobile phase A: 0.025% $NH_3{\cdot}H_2O$ in water (v/v) , B: MeCN; gradient: 0.0min 5% $\beta\rightarrow$ 0.8min 95% $\beta\rightarrow$ 1.2min 95% $\beta\rightarrow$ 1.21min 5% $\beta\rightarrow$ 1.55min 5% B; flow rate: 1.5 mL/min; oven temperature: 40°C; UV detection: 220 nm & 254 nm.

**Method 10:** MS instrument type: SHIMADZU LC-20AB; column: Kinetex EVO C18 2.1X30mm,5um; mobile phase A: 0.0375% TFA in water (v/v), B: 0.01875% TFA in MeCN (v/v); gradient: 0.0min 5% $\beta\rightarrow$ 0.8min 95% $\beta\rightarrow$ 1.20min 95% $\beta\rightarrow$ 1.21min 5% $\beta\rightarrow$ 1.55min 5% B; flow rate: 1.5 mL/min; oven temperature: 50°C; UV detection: 220 nm & 254 nm.

**Method 11:** MS instrument type: SHIMADZU LCMS-2020; Kinetex EVO C18 2.1X30mm,5um; mobile phase A: 0.0375% TFA in water (v/v), B: 0.01875% TFA in MeCN (v/v); gradient: 0.0min 5% $\beta\rightarrow$ 0.8min 95% $\beta\rightarrow$ 1.2min 95% $\beta\rightarrow$ 1.21min 5% $\beta\rightarrow$ 1.55min 5% B; flow rate: 1.5 mL/min; oven temperature: 50°C; UV detection: 220 nm & 254 nm.

**Method 12:** MS instrument type: SHIMADZU LCMS-2020; column: Kinetex EVO C18 2.1X30mm,5um; mobile phase A: 0.025% NH3·H2O in water (v/v) , B: MeCN; gradient: 0.0min 5% $\beta\rightarrow$ 0.8min 95% $\beta\rightarrow$ 1.2min 95% $\beta\rightarrow$ 1.21min 5% $\beta\rightarrow$ 1.5min 5% B; flow rate: 1.5 mL/min; oven temperature: 40°C; UV detection: 220 nm & 254 nm.

**Method 13:** Instrument: SHIMADZU LCMS-2020 SingleQuad; Column: Chromolith@Flash RP-18E 25-2 MM; eluent A: water + 0.0375 vol % TFA, eluent B: MeCN + 0.01875 vol % TFA; gradient: 0-0.8min, 5-95% B, 0.8-1.2min 95% B; flow 1.5 ml/min; temperature: 50°C; DAD: 220 nm & 254 nm.

**Examples 1 and 2 - Synthesis of Compounds of formula (IV)**

**Intermediate 1-1**

**Step 1**

4-(2-ethoxyethoxy)benzaldehyde

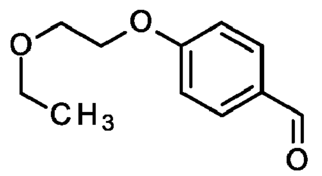

4-Fluorobenzaldehyde (5.00 g, 40.3 mmol; [459-57-4]), 2-ethoxyethan-1-ol (12 ml, 120 mmol; [110-80-5]) and cesium carbonate (15.8 g, 48.3 mmol; [534-17-8]) were added to a reaction flask containing DMF (100 ml), and the mixture was stirred at 70°C until the 4-fluorobenzaldehyde starting material was consumed (by monitoring with LCMS). Water was added, and the aqueous phase extracted with MTBE, the organic phase was washed with NaCl (sat. aq.), dried over $Na_2SO_4$, filtered and the solvent removed under reduced pressure. N-Heptane was added to the crude product, following which it was removed under reduced pressure, followed by chromatography ($SiO_2$, Biotage 100 g Ultra column, A = Hexane, B = EtOAc, 0%B to 50%B) yielding the title compound as a pale yellow crystalline solid (6.83 g, 83 % yield).

LC-MS (Method 4): $R_t$ = 0.95min; MS (ESIpos): m/z = 195 $[M+H]^+$.

$^1$H NMR (DMSO-d6, 400 MHz): δ (ppm) 9.87 (s, 1H), 7.83-7.88 (m, 2H), 7.11-7.17 (m, 2H), 4.19-4.23 (m, 2H), 3.70-3.74 (m, 2H), 3.50 (q, J = 7.1 Hz, 2H), 1.12 (t, J = 7.1 Hz, 3H).

**Step 2**

ethyl-3-[4-(2-ethoxyethoxy)phenyl]oxirane-2-carboxylate

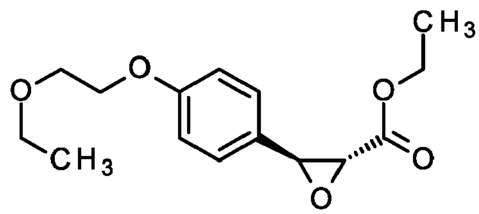

Sodium hydride (2.1 g, 60% in mineral oil, 52 mmol), was added to THF (200 ml), and ethyl chloroacetate (5.6 ml, 53 mmol) added dropwise under $N_2$. 4-(2-ethoxyethoxy)benzaldehyde (6.83 g, 35.2 mmol) was then added in THF (30 ml) and the reaction was allowed to warm to RT and stirred for 3d. The mixture was then added to an ice water mixture, and the aqueous layer extracted three times with EtOAc. The product was then purified by chromatography ($SiO_2$, Biotage, Ultra 100g column, A = Hexane, B = EtOAc, 0%B to 50%B), yielding the racemic title compound (6.60 g, 64 % yield).

LC-MS (Method 4): $R_t$ = 1.14min; MS (ESIpos): m/z = 281.3 $[M+H]^+$.

$^1$H NMR (DMSO-$d_6$, 400 MHz): δ (ppm) 7.27-7.31 (m, 2H), 6.92-6.97 (m, 2H), 4.15-4.24 (m, 2H), 4.06-4.10 (m, 3H), 3.81 (d, *J* = 2.0 Hz, 1H), 3.65-3.71 (m, 2H), 3.49 (q, *J* = 7.0 Hz, 2H), 1.24 (t, *J* = 7.1 Hz, 3H), 1.12 (t, *J* = 7.1 Hz, 3H).

**Step 3**

ethyl 3-[4-(2-ethoxyethoxy)phenyl]-2-hydroxypropanoate

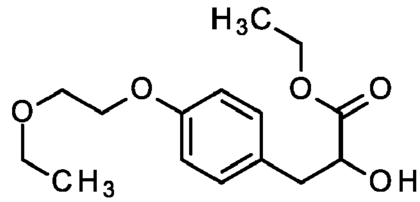

Ethyl-3-[4-(2-ethoxyethoxy)phenyl]oxirane-2-carboxylate (6.60 g, 23.5 mmol) was dissolved in ethanol (110 ml), palladium (607 mg, 10 % on carbon, 571 μmol) added and the mixture hydrogenated with 1 atm of $H_2$ overnight. The mixture was then filtered through a glass fiber filter and concentrated to dryness under reduced pressure. Purification by chromatography ($SiO_2$, Biotage Ultra: 50g column, A = Hexane, B = EtOAc, 0%B to 50%B) yielded the title compound, **Intermediate 1-1,** as a racemate (5.39 g, 77 % yield).

$^1H$ NMR (400 MHz, DMSO-$d_6$) δ ppm 0.94 - 1.23 (m, 7H), 2.72 - 2.88 (m, 2H), 3.46 - 3.52 (m, 2H), 3.64 - 3.70 (m, 2H), 3.95 - 4.18 (m, 5H), 5.48 (d, J=6.08 Hz, 1H), 6.81 - 6.91 (m, 2H), 7.08 - 7.13 (m, 2H).

**Intermediate 2-1**

**Step 1**

(5R)-2,2-dimethyl-5-(prop-2-en-1-yl)-1,3-dioxolan-4-one

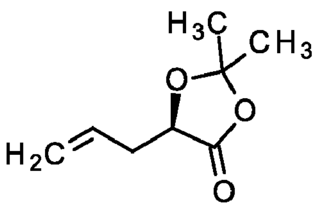

To a solution of (2R)-2-hydroxypent-4-enoic acid (35.0 g, 301 mmol, [413622-10-3]) in acetone (700 ml) were added pyridinium p-toluene sulfonate (37.9 g, 151 mmol) and 2,2-dimethoxypropane (251 g, 2.41 mol) at room temperature. The mixture was stirred at 60°C for 3h. The mixture was concentrated to give a residue. It was combined with the residue of an identical experiment (35 g). The combined residues were diluted with EtOAc and filtered through a pad of Celite. The filtrate was concentrated to give a residue. The residue was purified by column chromatography ($SiO_2$,1000 mesh, petroleum ether: EtOAc = 1: 0, then 20: 1) to give (5R)-5-allyl-2,2-dimethyl-1,3-dioxolan-4-one (51.0 g, 327 mmol, 54%) as yellow oil.

$^1H$ NMR (400 MHz, DMSO-$d_6$): δ [ppm] = 5.84-5.67 (m, 1H), 5.21-5.08 (m, 2H), 4.73-4.67 (m, 1H), 2.60-2.51 (m, 1H), 2.46-2.34 (m, 1H), 1.54 (s, 3H), 1.52 (s, 3H).

**Step 2**

(5R)-5-[(2E)-3-{4-[2-(2-ethoxyethoxy)ethoxy]phenyl}prop-2-en-1-yl]-2,2-dimethyl-1,3-dioxolan-4-one

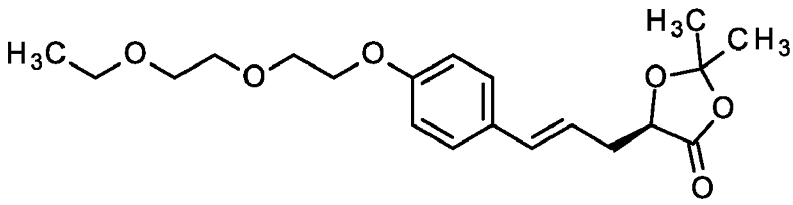

A mixture of (5R)-2,2-dimethyl-5-(prop-2-en-1-yl)-1,3-dioxolan-4-one (25.0 g, 160 mmol), 1-[2-(2-ethoxyethoxy)ethoxy]-4-iodobenzene (53.8 g, 160 mmol, [2305345-75-7]), palladium(II) acetate (3.59 g, 16.0 mmol), tri-2-tolylphosphine (4.87 g, 16.0 mmol) and DIPEA (70 ml, 400 mmol) in MeCN (500 ml) was stirred at 80°C for 12h. The mixture was concentrated to give a residue. It was combined with the residues of two identical experiments (25 g and 3 g). The combined residues were diluted with EtOAc and washed with $NH_4Cl$ (sat. aq.). The organic phase was washed with NaCl (sat. aq.), dried over anhydrous $Na_2SO_4$ filtered and concentrated. The residue was purified by flash column chromatography ($SiO_2$, petroleum ether: EtOAc = 1: 0 to 10: 1, then 10: 1) to give (5R)-5-[(2E)-3-{4-[2-(2-ethoxyethoxy)ethoxy]phenyl}prop-2-en-1-yl]-2,2-dimethyl-1,3-dioxolan-4-one (64.0 g, 176 mmol, 73%) as yellow oil.

LC-MS (Method 13): $R_t$ = 0.951min; MS (ESIpos): m/z = 365.2 $[M+H]^+$.

$^1H$ NMR (400 MHz, DMSO-$d_6$): δ [ppm] = 7.32 (d, *J* = 8.8 Hz, 2H), 6.90 (d, *J* = 8.8 Hz, 2H), 6.46 (d, *J* = 16 Hz, 1H), 6.12-5.99 (m, 1H), 4.81-4.73 (m, 1H), 4.13-4.05 (m, 2H), 3.79-3.69 (m, 2H), 3.63-3.55 (m, 2H), 3.53-3.47 (m, 2H). 3.43 (q, *J* = 6.8 Hz, 2H). 2.78-2.65 (m, 1H), 2.60-2.52(m, 1H), 1.55 (s, 3H), 1.55 (s, 3H), 1.10 (t, *J* = 7.2 Hz, 3H).

**Step 3**

(5*R*)-5-(3-{4-[2-(2-ethoxyethoxy)ethoxy]phenyl}propyl)-2,2-dimethyl-1,3-dioxolan-4-one

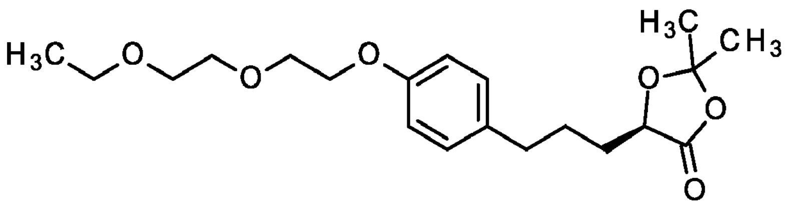

To a solution of (5*R*)-5-[(2*E*)-3-{4-[2-(2-ethoxyethoxy)ethoxy]phenyl}prop-2-en-1-yl]-2,2-dimethyl-1,3-dioxolan-4-one (59.0 g, 162 mmol) in THF (1.2 l) was added palladium (2.9 g, 10% on carbon, 5.54 mmol) at RT. The mixture was stirred at RT under $H_2$ atmosphere (15 psi) for 12h. The mixture was filtered, and the filtrate was concentrated to give the title compound (60.0 g) as a yellow oil.

LC-MS (Method 13): $R_t$ = 0.987min; MS (ESIpos): m/z = 367.1 $[M+H]^+$.

**Step 4**

methyl (2R)-5-{4-[2-(2-ethoxyethoxy)ethoxy]phenyl}-2-hydroxypentanoate

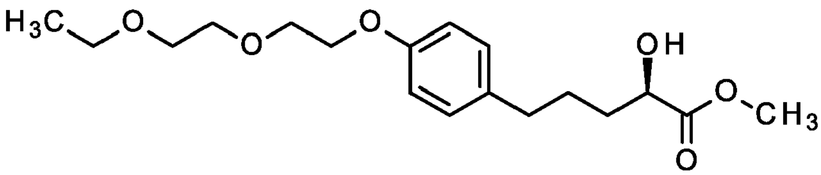

To a solution of (5R)-5-(3-{4-[2-(2-ethoxyethoxy)ethoxy]phenyl}propyl)-2,2-dimethyl-1,3-dioxolan-4-one (60.0 g, 164 mmol) in MeOH (600 ml) was 4-toluenesulfonic acid (2.82 g, 16.4 mmol) at RT. The mixture was stirred at RT for 12h. The mixture was combined with an earlier experiment (5.2 g), concentrated and diluted with EtOAc. The organic phase was washed with $NaHCO_3$ (sat. aq.) and NaCl (sat. aq.). The organic phase was dried over anhydrous $Na_2SO_4$,

filtered and concentrated. The residue was purified by reverse phase column (Agela HP1000; Welch Ultimate XB_C18 100*400mm 20-40 μm; 200 ml/min; A = water (0.1% formic acid), B = MeCN; B%: 10%B-45B% in 35min, 50%B 20min; RT; UV 220/254 nm) to give the title compound **(Intermediate 2-1),** 48.4 g, 142 mmol, 80%) as a yellow oil.

LC-MS (Method 13): $R_t$ = 0.630min; MS (ESIpos): m/z = 341.3 $[M+H]^+$.

LC-MS (Method 3): $R_t$ = 1.00min (91% DAD); MS (ESIpos): m/z = 341 $[M + H]^+$.

Chiral HPLC (Thermo Fisher UltiMate 3000; YMC Cellulose SB 3μ, 100x4.6; A = hexane + 0.1 vol % DEA; B = ethanol; 10%B; 1.4 ml/min; 25°C; 280 nm): $R_t$ = 2.85min (4.94%), $R_t$ = 3.57min (85.21%). e.e. = 89%.

Specific Rotation: $\alpha_D^{20}$ = -7.46° +/- 0.58° (c=1, $CHCl_3$).

$^1$H NMR (CHLOROFORM-d, 400 MHz): δ (ppm) 7.05-7.10 (m, 2H), 6.82-6.86 (m, 2H), 4.20 (ddd, *J* = 7.0, 5.7, 4.1 Hz, 1H), 4.12 (dd, *J* = 5.6, 4.3 Hz, 2H), 3.86 (dd, *J* = 5.1, 4.1 Hz, 2H), 3.77 (s, 3H), 3.71-3.74 (m, 2H), 3.60-3.64 (m, 2H), 3.54 (q, *J* = 7.1 Hz, 2H), 2.74 (d, *J* = 5.6 Hz, 1H), 2.53-2.64 (m, 2H), 1.74-1.86 (m, 2H), 1.61-1.73 (m, 2H), 1.22 (t, *J* = 7.1 Hz, 3H).

$^{13}$C NMR (101MHz, CHLOROFORM-d) δ = 175.67, 156.96, 134.13, 129.21 (2C), 114.49 (2C), 70.87, 70.30, 69.85, 69.80, 67.42, 66.69, 52.53, 34.53, 33.84, 26.68, 15.15.

**Intermediate 2-2**

**Step 1**

methyl (2R)-3-tert-butoxy-2-hydroxypropanoate

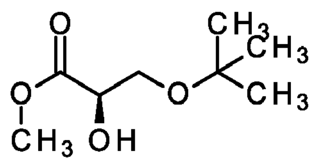

Into a three-necked round bottom flask equipped with mechanical stirrer were placed 2-methyl-propan-2-ol (370 ml, 3.9 mol) and magnesium trifluormethanesulfonate (79.0 g, 245 mmol; [60871-83-2]) and EtOAc (370 ml) were added. The reaction was placed under $N_2$ and brought to 50°C (measured in flask). Then methyl (2R)-oxirane-2-carboxylate (21 ml, 240 mmol, [111058-32-3]) were added dropwise and the mixture was stirred at 60°C for 3.5d. After cooling to RT, EtOAc was added. The reaction mixture was washed twice with NaCl (3.1 M aq.) and the organic phase concentrated under reduced pressure. The residue was co-distilled with toluene 3 times to remove unreacted methyl glycidate. The resulting raw product was purified by chromatography (Isolera LS, Sfär 350 g, A = Hexane, B = DCM, C = EtOAc, A = 3CV, A to B in 5 CV, B 4 CV, B to 50%C in 6CV). The interesting fractions were pooled, concentrated under reduced pressure and co-distilled with toluene once to give the title compound 37.3 g (68 as a clear oil.

$^1$H NMR (CHLOROFORM-d, 400 MHz): δ (ppm) 3.77 (s, 3H), 3.60-3.66 (m, 2H), 1.15 (s, 9H).

**Step 2**

methyl (2R)-3-tert-butoxy-2-[(trifluoromethanesulfonyl)oxy]propanoate

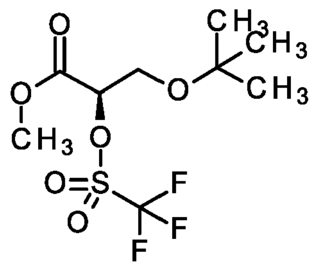

Methyl (2R)-3-tert-butoxy-2-hydroxypropanoate (5.00 g, 28.4 mmol) in DCM (4.0 ml) was cooled to -70°C under $N_2$ and lutidine (4.0 ml, 34 mmol) was added. Trifluoromethanesulfonic anhydride (30 ml, 1.0 M in DCM, 30 mmol) was

added dropwise, after which the mixture was stirred at - 70°C for 2.5 h. Then the mixture was allowed to warm to -20°C and was quenched with ice-water. The phases were separated, and the aqueous phase extracted with DCM. The combined organic phases filtered through a water repellent filter and concentrated under reduced pressure. the title compound Intermediate 2-2 (9,45 g (>100%)), which was used without further purification.

Specific rotation: $\alpha_D^{20}$ = +20.9° +/- 0.2° (c=1, $CHCl_3$).

$^{19}F$ NMR (CHLOROFORM-d, 471 MHz): δ (ppm) -75.86 (s, 1F).

$^1H$ NMR (CHLOROFORM-d, 500 MHz): δ (ppm) 5.22 (dd, *J* = 6.9, 2.8 Hz, 1H), 3.86 (s, 3H), 3.85 (dd, *J* = 11.0, 2.8 Hz, 1H), 3.80 (dd, *J* = 11.0, 6.9 Hz, 1H), 1.20 (s, 9H). Residual lutidine triflate is easily quantified: δ (ppm) 8.04 (t, *J* = 7.9 Hz, 0.11H), 7.41 (d, *J* = 7.9 Hz, 0.21H), 2.82 (s, 0.66H); -79.62 (s, 0.1 F).

## Example 1

### Step 1

ethyl-3-[4-(2-ethoxyethoxy)phenyl]-2-[(methanesulfonyl)oxy]propanoate

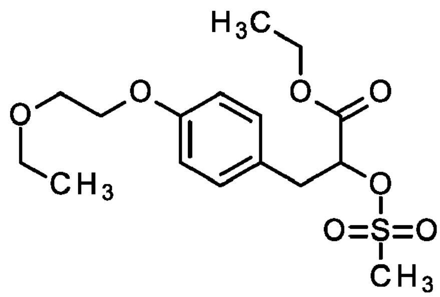

Ethyl-3-[4-(2-ethoxyethoxy)phenyl]-2-hydroxypropanoate (5.39 g, 19.1 mmol, **Intermediate 1-1),** and TEA (5.9 ml, 42 mmol; [121-44-8]) were stirred in THF (56 ml) under $N_2$ at 0-5°C. MsCl (1.6 ml, 21 mmol; [124-63-0]) was added dropwise to the mixture and stirring was continued for 3h. The mixture was then added to a solution of $NaHCO_3$ (50% sat. aq.), and the aqueous layer extracted with MTBE (3x), the combined organic layers washed with a solution of NaCl (sat. aq.), dried over $Na_2SO_4$, filtered and concentrated under reduced pressure yielding the title compound (6.96 g, 90 % purity, 91 % yield).

LC-MS (Method 4): $R_t$ = 1.15min; MS (ESIpos): m/z = 378.3 $[M+NH_4^+]^+$.

$^1H$ NMR (400 MHz, DMSO-d6) δ ppm 1.09 - 1.18 (m, 6H) 3.02 (s, 3H) 3.04 - 3.10 (m, 2H) 3.49 (q, 2H) 3.64 - 3.70 (m, 2H) 4.02 - 4.07 (m, 2H) 4.13 (q, 2H) 5.26 (dd, 1H) 6.85 - 6.90 (m, 2H) 7.14 - 7.19 (m, 2H).

### Step 2

ethyl-2-[4,10-bis(2-tert-butoxy-2-oxoethyl)-1,4,7,10-tetraazacyclododecan-1-yl]-3-[4-(2-ethoxyethoxy)phenyl]propanoate

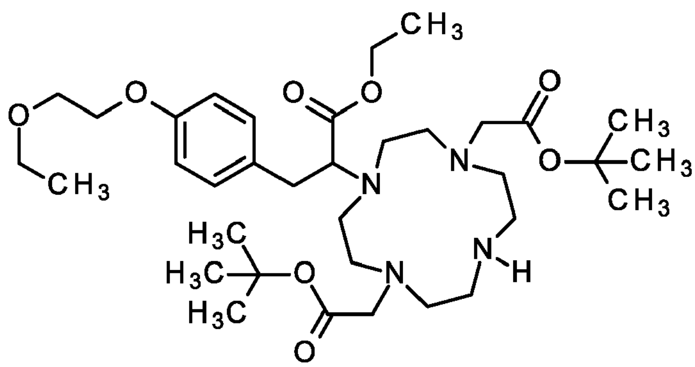

di-tert-butyl 2,2'-(1,4,7,10-tetraazacyclododecane-1,7-diyl)diacetate (.51 g, 11.2 mmol, [162148-48-3]), racemic ethyl-3-[4-(2-ethoxyethoxy)phenyl]-2-[(methanesulfonyl)oxy] propanoate (4.46 g, 12.4 mmol) and $K_2CO_3$ (3.42 g, 24.8 mmol) in MeCN (31 ml) were stirred at 80°C for 24h. The mixture was filtered, the solid washed with EtOH, the organic phases combined and concentrated under reduced pressure to afford the crude title compound (8.40 g, 57% product by ELSD detection).
LC-MS (Method 2): $R_t$ = 1.26min; MS (ESIpos): m/z = 665.8 $[M+H^+]^+$.

**Step** 3

ethyl-2-[(methanesulfonyl)oxy]butanoate

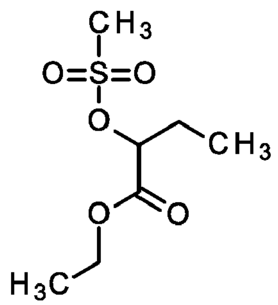

Ethyl-2-hydroxybutanoate (5.00 g, 37.8 mmol, [52089-54-0]) and TEA (12 ml, 83 mmol; [121-44-8]) were stirred in THF (110 ml) under $N_2$ at 0-5°C. MsCl (3.2 ml, 42 mmol; [124-63-0]) was added dropwise to the mixture and stirring was continued for 3h. The mixture was then added to a solution of $NaHCO_3$ (50% sat. aq.), and the aqueous layer extracted with MTBE (3x), the combined organic layers washed with NaCl (sat. aq.), dried over $Na_2SO_4$, filtered and concentrated under reduced pressure yielding the title compound (8.21 g, 103 % yield).
$^1H$ NMR (400 MHz, DMSO-d6) δ ppm 0.93 (t, 3H) 1.22 (t, 3H) 1.71 - 1.95 (m, 2H) 3.25 (s, 3H) 4.19 (dtt, 2H) 5.05 (dd, 1H).

**Step** 4

ethyl-2-[4,10-bis(2-tert-butoxy-2-oxoethyl)-7-{1-ethoxy-3-[4-(2-ethoxyethoxy)phenyl]-1-oxopropan-2-yl}-1,4,7,10-tetraazacyclododecan-1-yl]butanoate

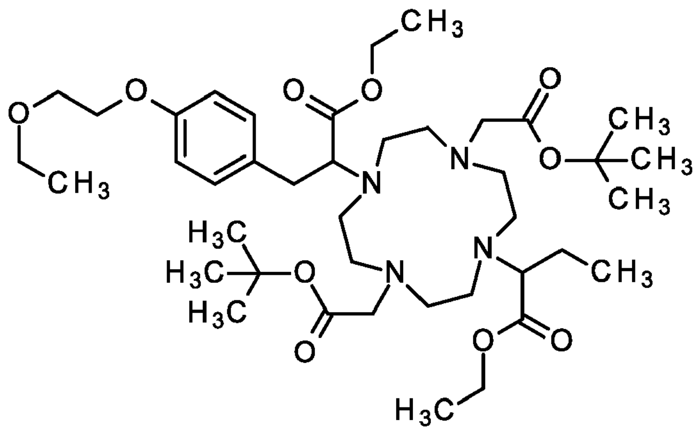

Ethyl-2-[4,10-bis(2-tert-butoxy-2-oxoethyl)-1,4,7,10-tetraazacyclododecan-1-yl]-3-[4-(2-ethoxyethoxy)phenyl]propanoate, Step 2, (4.20 g, 6.32 mmol), ethyl-2-[(methanesulfonyl)oxy] butanoate (2.12 g, 10.1 mmol), $K_2CO_3$ (1.75 g, 12.6 mmol) in MeCN (21 ml) were stirred for 24h at 80°C. The mixture was then filtered, the solid washed with EtOH, the organic phases combined and concentrated under reduced pressure affording the crude title compound (6.50 g).

LC-MS (Method 2): $R_t$ = 1.28min; MS (ESIpos): m/z = 779.9 $[M+H]^+$.

**Step** 5

2,2'-(4-{1-ethoxy-3-[4-(2-ethoxyethoxy)phenyl]-1-oxopropan-2-yl}-10-[1-ethoxy-1-oxobutan-2-yl]-1,4,7,10-tetraazacyclododecane-1,7-diyl)diacetic acid

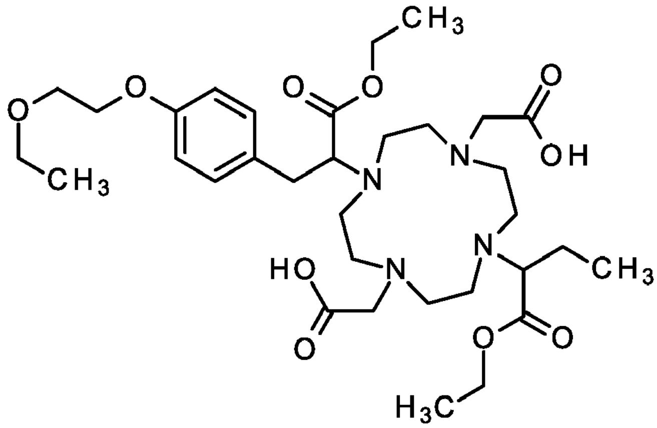

Ethyl-2-[4,10-bis(2-tert-butoxy-2-oxoethyl)-7-{1-ethoxy-3-[4-(2-ethoxyethoxy)phenyl]-1-oxopropan-2-yl}-1,4,7,10-tetraazacyclododecan-1-yl]butanoate (6.50 g, 8.34 mmol) and formic acid (110 ml) were stirred together overnight at 70°C. The mixture was concentrated under reduced pressure, and RP-chromatography (Biotage SNAP Ultra C18 120g, 50ml/min, A = water, B = MeCN, 0%B to 50%B in 30min, 100%B 10min) yielded the title compound as a mixture of diastereomers (1.21 g, 95 % purity, 22 % yield over 5 steps).

LC-MS (Method 2): $R_t$ = 0.76min; MS (ESIpos): m/z = 665.7 $[M+H]^+$.

$^1H$ NMR (DMSO-$d_6$, 600 MHz): $\delta$ (ppm) 7.17-7.24 (m, 2H), 6.82 (d, *J* = 7.8 Hz, 2H), 4.07-4.13 (m, 2H), 3.94-4.04 (m, 4H), 3.74 (dd, *J* = 9.4, 6.4 Hz, 0.6H), 3.66 (t, *J* = 4.7 Hz, 2H), 3.63 (br dd, *J* = 9.0, 6.4 Hz, 0.5H), 3.48 (q, *J* = 6.9 Hz, 2H), 3.31-3.41 (m, 4H), 2.58-3.28 (m, 19H), 1.64-1.78 (m, 1H), 1.53-1.63 (m, 1H), 1.18-1.23 (m, 3H), 1.03-1.13 (m, 6H), 0.81-0.89 (m, 3H).

$^{13}C$ NMR (DMSO-$d_6$, 151 MHz): $\delta$ (ppm) 171.5/171.4, 170.7/170.7, 167.9/168.7 (br. 2C), 156.7, 130.3/130.2 (2C), 129.4/129.5, 113.8/113.8 (2C), 68.1, 66.7, 65.4, 65.3/65.2, 64.1 (br), 59.7/59.7, 59.6/59.7, 55.3/54.4 (2C), 52.4/52.7 (2C), 52.0 (br, 2C), 45.9/45.7 (2C), 45.5 (br, 2C), 33.5/33.3, 21.5/21.3, 14.8, 14.0, 13.9, 13.8, 10.7/10.7.

**Step 6**

gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]butanoate

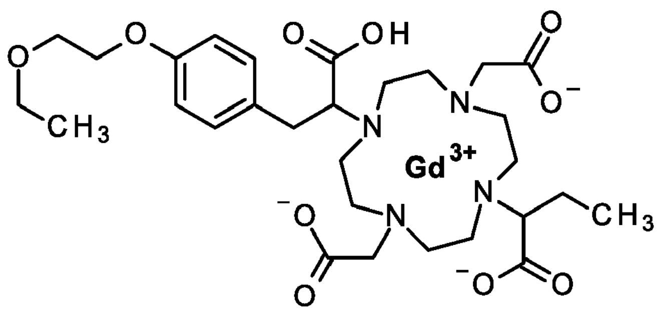

2,2'-(4-{1-Ethoxy-3-[4-(2-ethoxyethoxy)phenyl]-1-oxopropan-2-yl}-10-[1-ethoxy-1-oxobutan-2-yl]-1,4,7,10-tetraazacyclododecane-1,7-diyl)diacetic acid (1.21 g, 1.81 mmol), $Gd_2O_3$ (296 mg, 817 $\mu$mol) and water were stirred

together and heated at 105°C for 24h in a crimp sealed vessel, following which the mixture was heated at 120°C for an additional 6h. The mixture was treated with Chelex100™ (sodium form, washed, approximately 5 g), stirred for 1h, following which it was filtered, concentrated under reduced pressure and purified by RP-chromatography (Biotage SNAP Ultra C18 60g, 50 ml/min, A = water, B = MeCN, 0%B to 50%B in 25min, 100%B 10min) yielding the title compounds as diastereomeric mixtures. Fraction 1 (Example 1, 575 mg, 41 % yield) and Fraction 2 (74 mg, 5 % yield).

Fraction 1

LC-MS (Method 3): $R_t$ = 0.58min (24% DAD) and 0.63min (74% DAD); MS (ESIpos): m/z = 383.7 $[M+2H]^{++}$, 766.4 $[M+H]^+$. Isotope patterns match theoretical values for corresponding gadolinium complex.

Fraction 2

LC-MS (Method 3): $R_t$ = 0.63min (100% DAD); MS (ESIpos): m/z = 383.7 $[M+2H]^{++}$, 766.4 $[M+H]^+$. Isotope patterns match theoretical values for corresponding gadolinium complex.

**Example 2**

**Step 1**

methyl (2R)-5-{4-[2-(2-ethoxyethoxy)ethoxy]phenyl}-2-[(trifluoromethanesulfonyl) oxy]pentanoate

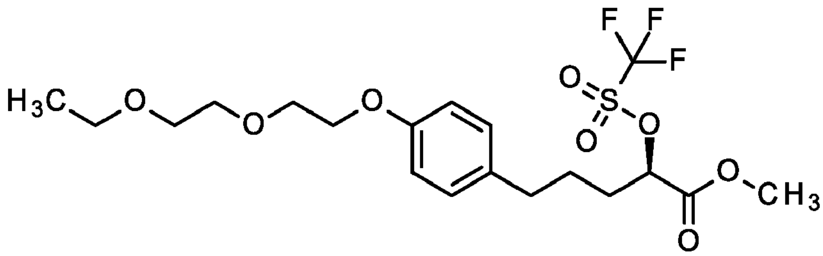

**Intermediate 2-1,** methyl (2R)-5-{4-[2-(2-ethoxyethoxy)ethoxy]phenyl}-2-hydroxypentanoate, (5.00 g, 14.7 mmol) was dissolved in DCM (50 ml), placed under $N_2$ and cooled to -60°C. Lutidine (2.1 ml, 18 mmol; [108-48-5]) was added followed by dropwise addition of trifluoromethanesulfonic anhydride (15 ml, 1.0 M in DCM, 15 mmol; [358-23-6]) at -60°C. The reaction was stirred for 2h and then allowed to warm to ca. 8°C. Diethyl ether was added to precipitate the lutidine triflate, filtered and the filter cake washed with diethyl ether. The combined filtrates were extracted with water and HCl (1 M, aq.), dried ($Na_2SO_4$) and concentrated under reduced pressure. 6,97 g (85%) of the title compound were obtained as reddish oil, which was used directly in the next step.

$^{19}F$ NMR (CHLOROFORM-d, 377 MHz): δ (ppm) -76.04 (s).

$^1H$ NMR (CHLOROFORM-d, 400 MHz): δ (ppm) 7.03-7.07 (m, 2H), 6.82-6.87 (m, 2H), 5.12 (t, *J* = 6.1 Hz, 1H), 4.10-4.14 (m, 2H), 3.86 (dd, *J* = 5.6, 4.3 Hz, 2H), 3.82 (s, 3H), 3.70-3.74 (m, 2H), 3.60-3.63 (m, 2H), 3.54 (q, *J* = 6.9 Hz, 2H), 2.56-2.65 (mc, 2H), 1.95-2.03 (m, 2H), 1.70-1.79 (m, 2H), 1.21 (t, *J* = 7.0 Hz, 3H). The sample contains 1.1 eq (15%) diethyl ether: 3.48 (q, *J* = 7.1 Hz, 0.48H), 1.21 (br t, *J* = 7.0 Hz, 0.69H).

Step 2

methyl (2S)-5-{4-[2-(2-ethoxyethoxy)ethoxy]phenyl}-2-(1,4,7,10-tetraazacyclododecan-1-yl)pentanoate

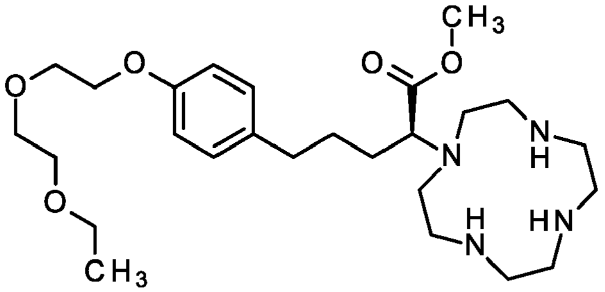

1,4,7,10-tetraazacyclododecane (10.1 g, 58.8 mmol, ca. 5 eq) was dissolved in chloroform (100 ml), DIPEA (2.0 ml, 12 mmol; [7087-68-5]) and a solution of methyl (2R)-5-{4-[2-(2-ethoxyethoxy)ethoxy]phenyl}-2-[(trifluoromethanesulfonyl)oxy]pentanoate (6.94 g) in chloroform (25 ml) added. The reaction was stirred overnight at RT. The mixture was extracted with water (3 x) to remove excess cyclen and sat. NaCl, dried ($Na_2SO_4$) and concentrated under reduced pressure to give the title compound (7,55 g) as an orange-brown oil, part of which was used directly in the next step.

LC-MS (Method 2): $R_t$ = 0.72min; MS (ESIpos): m/z = 496 $[M+H]^+$.

**Step 3**

methyl (2S)-5-{4-[2-(2-ethoxyethoxy)ethoxy]phenyl}-2-{4,7,10-tris[(2S)-3-tert-butoxy-1-methoxy-1-oxopropan-2-yl]-1,4,7,10-tetraazacyclododecan-1-yl}pentanoate

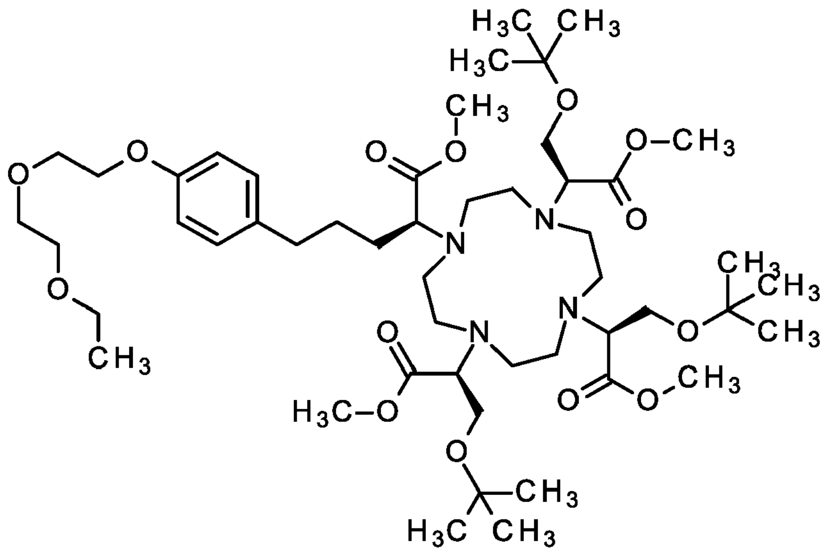

methyl (2S)-5-{4-[2-(2-ethoxyethoxy)ethoxy]phenyl}-2-(1,4,7,10-tetraazacyclododecan-1-yl) pentanoate (5.81 g, 11.7 mmol) was dissolved in MeCN (150 ml) and $K_2CO_3$ (5.68 g, 41.1 mmol) added. A solution of **Intermediate 2-2,** methyl (2R)-3-tert-butoxy-2-[(trifluoromethanesulfonyl)oxy]propanoate (13.3 g, 90 % purity, 38.8 mmol), in MeCN (50 ml) was added dropwise and the mixture stirred overnight at RT. The mixture was filtrated, the filter cake washed (MeCN) and the combined filtrates concentrated under reduced pressure. The residue obtained, 30,90 g, was absorbed to Isolute™ and purified by RP-chromatography (SNAP C18 400 g, 100 ml/min, 254 nm, A = water/0.1% formic acid, B = MeCN, 10% B 6CV, 10% to 41% B in 8.8CV, 41% B 1.7CV, 41% to 80% B in 11CV, 80%B 2.9CV). After lyophilization, two fractions of the title compound were obtained.

Fraction 1: 2,38 g

LC-MS (Method 2): $R_t$ = 1.37min; MS (ESIpos): m/z = 970 $[M+H]^+$.

Fraction 2: 5,76 g

LC-MS (Method 2): $R_t$ = 1.24min; MS (ESIpos): m/z = 970 $[M+H]^+$.

Specific Rotation: $\alpha_D^{20}$ = +14.60° +/- 0.18° (c=1, MeOH).

LC-MS (Method 3): $R_t$ = 1.17min (91% DAD); MS (ESIpos): m/z = 485 $[M + 2H]^{++}$, 970 $[M + H]^+$.

$^1H$ NMR (CHLOROFORM-d, 400 MHz): δ (ppm) 8.39 (s, 0.3H, formiate), 7.01-7.08 (m, 2H), 6.82 (d, *J* = 7.5 Hz, 2H), 4.07-4.13 (m, 2H), 3.48-3.95 (m, 30H), 2.49-3.43 (m, 18H), 1.49-1.88 (m, 4H), 1.09-1.25 (m, 30H).

$^{13}C$ NMR (CHLOROFORM-d, 101 MHz): δ (ppm) 173.4 (br), 171.9 (br, 3C), 157.5, 134.3, 129.6 (2C), 115.0 (2C), 74.4, 74.2 (2C), 71.3, 70.3, 70.2, 67.8, 67.1, 63.9 (br), 63.2 (br, 3C), 60.0 (br, 2C), 59.7 (br), 52.5, 52.2 (2C), 52.1 (br, 8C), 51.2, 35.2, 29.2, 27.8 (6C), 27.7 (3C), 15.6. Some signals are very broad, one C in the propylene-chain is not observed.

The combined yield calculated from step 1 is 74%.

## Step 4

methyl (2S)-5-{4-[2-(2-ethoxyethoxy)ethoxy]phenyl}-2-{4,7,10-tris[(2S)-3-hydroxy-1-methoxy-1-oxopropan-2-yl]-1,4,7,10-tetraazacyclododecan-1-yl}pentanoate

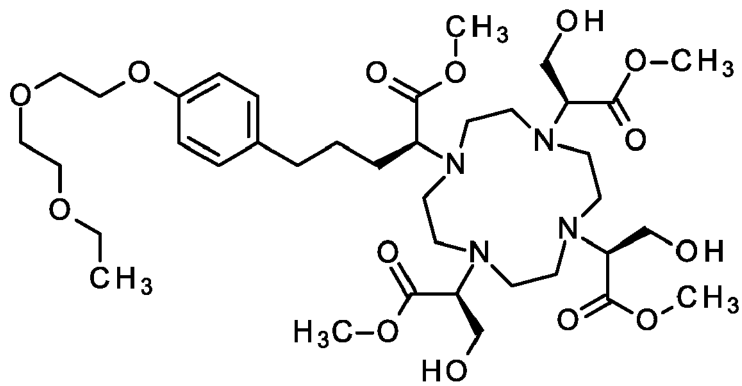

methyl (2S)-5-{4-[2-(2-ethoxyethoxy)ethoxy]phenyl}-2-{4,7,10-tris[(2S)-3-tert-butoxy-1-methoxy -1-oxopropan-2-yl]-1,4,7,10-tetraazacyclododecan-1-yl}pentanoate (2.38 g, 2.46 mmol) was dissolved in 1,4-dioxane (60 ml) and HCl (9.8 ml, 4 M in dioxane, 39.2 mmol) added. The mixture was stirred at 80°C for 4h and then evaporated to dryness under reduced pressure. The raw product, 2,13 g of methyl (2S)-5-{4-[2-(2-ethoxyethoxy)ethoxy]phenyl}-2-{4,7,10-tris[(2S)-3-hydroxy-1-methoxy-1-oxopropan-2-yl]-1,4,7,10-tetraazacyclododecan-1-yl}pentanoate, was used as such in the next step.

## Step 5

(2S)-5-{4-[2-(2-ethoxyethoxy)ethoxy]phenyl}-2-{4,7,10-tris[(1S)-1-carboxy-2-hydroxyethyl]-1,4,7,10-tetraazacyclododecan-1-yl}pentanoic acid

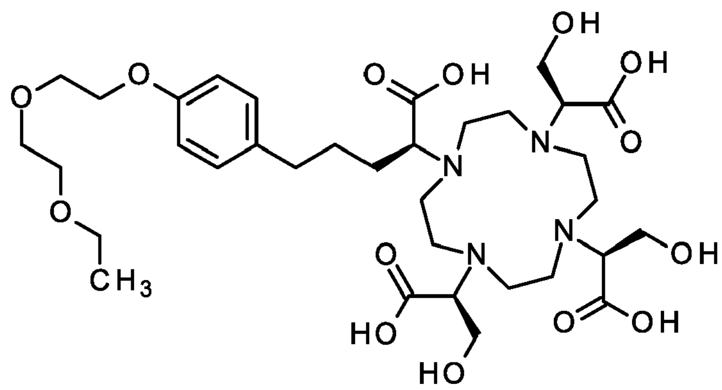

methyl (2S)-5-{4-[2-(2-ethoxyethoxy)ethoxy]phenyl}-2-{4,7,10-tris[(2S)-3-hydroxy-1-methoxy-1-oxopropan-2-yl]-1,4,7,10-tetraazacyclododecan-1-yl}pentanoate (2.13 g, 2.66 mmol) was dissolved in THF (13 ml) and a solution of lithium hydroxide (382 mg, 16.0 mmol) water (13 ml) was added. After stirring at RT overnight, the reaction was nearly complete. *(The presence of one methyl ester does negatively affect the complexation step,* as *the ester is hydrolyzed under the complexation conditions used. Therefore, complete saponification is not mandatory at this point.)* The reaction mixture was evaporated to dryness under reduced pressure to give 2,36 g raw material, which was used in the next reaction.

**Step 6**

gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)

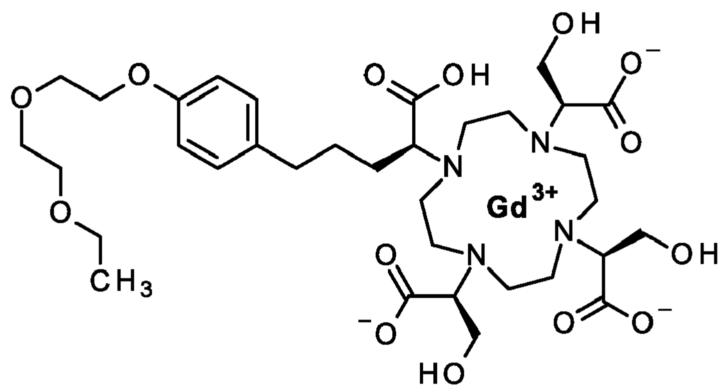

(2S)-5-{4-[2-(2-ethoxyethoxy)ethoxy]phenyl}-2-{4,7,10-tris[(1S)-1-carboxy-2-hydroxyethyl]-1,4,7,10-tetraazacyclododecan-1-yl}pentanoic acid (5.50 g, 7.38 mmol) was dissolved in water (400 ml) and the pH adjusted to 4 with formic acid. $Gd_2O_3$ (1.20 g, 3.32 mmol, 0.9 eq) was added and the mixture stirred at 100°C for 18h and overnight at RT and further 4h at 100°C. Chelex100™ (sodium form, ca. 10 g) were added and the mixture stirred 2d at RT, after which the Xylenol-orange test indicated the absence of free gadolinium. The pH of the solution was adjusted to 7 by addition of ammonium hydroxide (25%) and reduced to about 200 ml under reduced pressure. (*Fraction 2 of step* 3 *was taken through steps 4 to 6 in the* same *way. Although the retention times of the two fractions were different, they behaved in the* same *in the subsequent reactions and were combined at this point*.) The combined reactions (including the resin) were transferred to an empty Biotage cartridge and directly subjected to RP-chromatography (SNAP C18 400g, 100 ml/min, 254 nm. A = water, B = MeCN, 0% B 5CV, 0% to 45% B in 14CV). Interesting fractions were combined and lyophilized to give two fractions of gadolinium (2S,2'S,2"S)-2,2',2"-{10-[(1S)-1-carboxy-4-{4-[2-(2-ethoxyethoxy)ethoxy] phenyl} butyl]-1,4,7,10-tetraazacyclododecane-1,4,7-triyl}tris(3-hydroxypropanoate):

Fraction 1: 2,49 g

Specific rotation: $\alpha_D^{20}$ = +15.95° +/- 0.17° (c=0.84, $H_2O$).

LC-MS (Method 3): $R_t$ = 0.73min (100% DAD); MS (ESIpos): m/z = 450 $[M + 2H]^{++}$, 900 $[M + H]^+$ and some $[2M + 2H]^{++}$; 909 $[2M + H_2O + 2H]^{++}$. The observed isotope pattern conforms to structure.

UPLC-MS (Method 2): $R_t$ = 0.78min (100% ELSD), 0.75min (100% UV 220 nm) MS (ESIpos): m/z = 900 $[M + H]^+$.

Fraction 2: 1,99 g

Specific rotation: $\alpha_D^{20}$ = +16.38° +/- 0.35° (c=0.84, $H_2O$).

LC-MS (Method 3): $R_t$ = 0.73min (100% DAD); MS (ESIpos): m/z = 450 $[M + 2H]^{++}$, 900 $[M + H]^+$ and some $[2M + 2H]^{++}$; 909 $[2M + H_2O + 2H]^{++}$. The observed isotope pattern conforms to structure.

UPLC-MS (Method 2): $R_t$ = 0.78min (100% ELSD), 0.75min (94% UV 220 nm) 0.80min (6%, UV, 220 nm), both peaks MS (ESIpos): m/z = 900 $[M + H]^+$.

Fraction 2 contains some stereoisomer, probably through a racemization event at one of the preceding steps. The combined yield from step 3 is 42%).

**Examples 3 and 4 - Synthesis of Compounds of formula (V)**

**Example 3**

**Gadolinium-2,2',2"-[(2*S*)-10-(carboxymethyl)-2-(4-{2-[2-(2-methoxyethoxy)ethoxy] ethoxy}benzyl)-1,4,7,10-tetraazacyclododecan-1,4,7-triyl]triacetate**

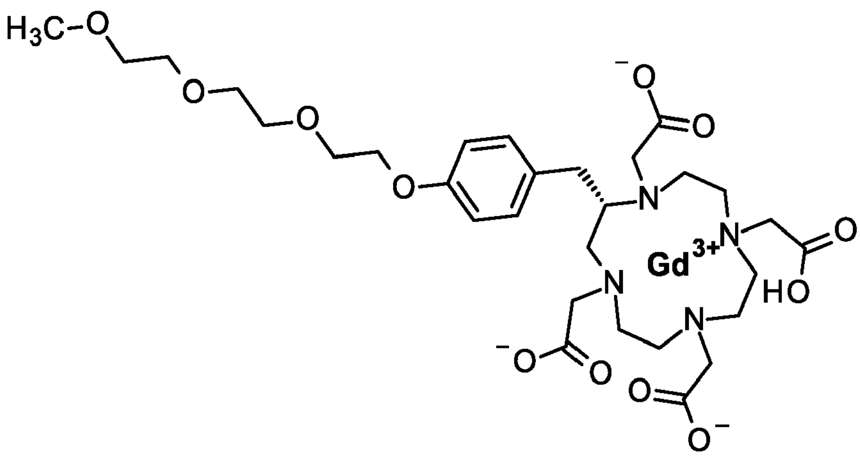

**Intermediate 3-1A**

**ethyl *N*-(*tert*-butoxycarbonyl)-O-{2-[2-(2-methoxyethoxy)ethoxy]ethyl}-L-tyrosinate**

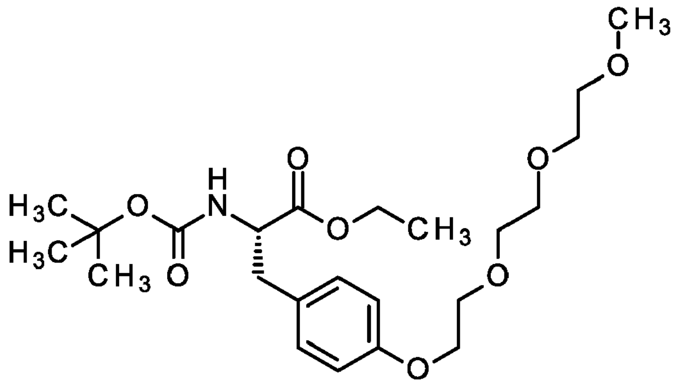

To ethyl *N*-(*tert*-butoxycarbonyl)-L-tyrosinate (5.00 g, 16.2 mmol, CAS:72594-77-5.) in THF (60 ml) was added 2-[2-(2-methoxyethoxy)ethoxy]ethan-1-ol (3.98 g, 24.2 mmol; CAS:112-35-6) followed by triphenylphosphane (8.05 g, 30.7 mmol) and di-*tert*-butyl (E)-diazene-1,2-dicarboxylate (7.07 g, 30.7 mmol) at 0°C. The mixture was stirred for 17 h while it warmed to RT. One half of the solvent was removed under reduced pressure and hexane (60 ml) was added and the mixture was stirred for 1 h while a precipitate formed. The precipitate was removed by filtration. The filtrate was concentrated under reduced pressure and purified by column chromatography (silica, hexane / EtOAc 0-50%) to yield 7.08 g (75% purity, 72% yield) of the title compound.

LC-MS (method 4): $R_t$ = 1.26 min; MS (ESIpos): m/z = 356 $[M+H]^+$.

$^1$H-NMR (400MHz, $CDCl_3$): $\delta$ [ppm]= 1.23 (t, 3H), 1.42 (s, 9H), 3.02 (br t, 2H), 3.38 (s, 3H), 3.53 - 3.59 (m, 2H), 3.64 - 3.70 (m, 6H), 3.71 - 3.75 (m, 2H), 3.79 - 3.89 (m, 2H), 4.07 - 4.12 (t, 2H), 4.12 - 4.20 (q, 2H), 4.44 - 4.55 (m, 1H), 4.95 (br d, 1H), 6.83 (d, 2H), 7.03 (d, 2H).

**Intermediate 3-1B**

**methyl-*N*-(*tert*-butoxycarbonyl)-O-{2-[2-(2-methoxyethoxy)ethoxy]ethyl}-L-tyrosinate**

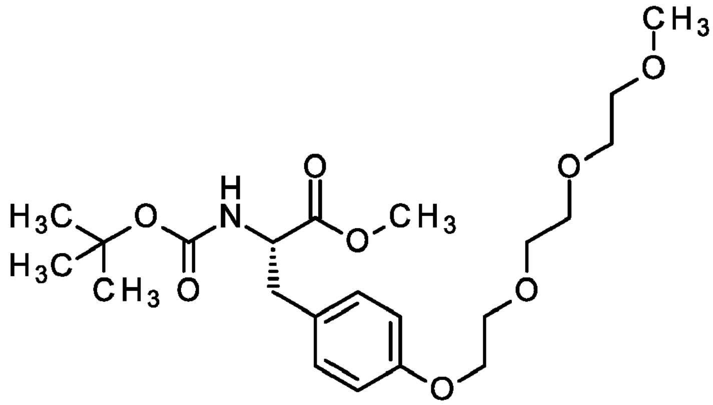

To a solution of methyl-*N*-(*tert*-butoxycarbonyl)-L-tyrosinate (7.13 g, 24.2 mmol) and 2-[2-(2-Methoxyethoxy)ethoxy]ethyl-4-methylbenzensulfonate (16.2 g, 50.7 mmol) in DMF (71 ml) was added potassium carbonate (6,68 g, 48,3 mmol) and 401mg potassium iodide anhydrous (401 mg, 2.42 mmol) and stirred at 80°C for 16h under nitrogen. The mixture was diluted with water and EtOAc and the layers were separated. The water phase was extracted with EtOAc. The combined organic layers were washed with brine, dried over repellent filter and concentrated in vacuum to yield 16.3 g (78 % purity, quantitative) of the title compound.

LC-MS (method 4): $R_t$ = 1.15 min; MS (ESIpos): m/z = 342 $[M+H]-BOC^+$.

$^1$H NMR (400 MHz, $CDCl_3$) $\delta$ [ppm] 1.41 (s, 9H) 2.98 - 3.09 (m, 2H) 3.37 (s, 3 H) 3.53 - 3.56 (m, 2H) 3.64 - 3.69 (m, 5H) 3.70 (s, 3H) 3.72 - 3.76 (m, 2H) 3.84 (dd, 2H) 4.09 (dd, 2H) 4.53 (br d, 1H) 4.94 (br s, 1H) 6.81 - 6.85 (m, 2H) 6.85 - 6.86 (m, 1H) 7.01 (d, 2H).

Optical rotation:$[\alpha]_D$ = 23.2° +/- 0.7° (c = 4.7 mg/ml, 20°C, 589 nm, $CHCl_3$).

**Intermediate 3-2A**

***tert*-butyl-[(2*S*)-1-hydroxy-3-(4-{2-[2-(2-methoxyethoxy)ethoxy]ethoxy}phenyl)propan-2-yl]carbamate**

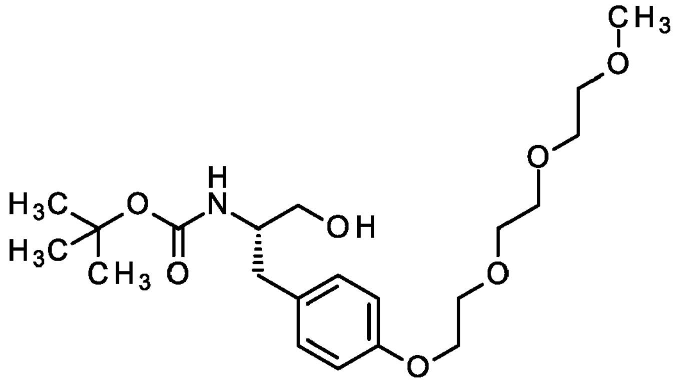

To ethyl-*N*-(*tert*-butoxycarbonyl)-O-{2-[2-(2-methoxyethoxy)ethoxy]ethyl}-L-tyrosinate **(Intermediate 1-1A,** 7.94 g, 17.4 mmol) solved in THF (190 ml) was added lithium aluminium hydride in THF (35 ml, 1.0 M, 35 mmol; CAS:16853-85-3) dropwise at 0°C. The mixture was stirred for 1 h while it warmed to RT. The reaction mixture was diluted with 2N HCl and EtOAc was added, phases were separated and the aqueous phase was extracted with EtOAc. Brine was added to the aqueous phase and was extracted with DCM/EtOH (9:1) two times. The combined organic extracts were dried and concentrated under reduced pressure to yield 3,95g (89% purity, 49% yield) of the title compound.

LC-MS (method 4): $R_t$ = 1.03 min; MS (ESIpos): m/z = 314 [M+H] - $BOC^+$

$^1$H-NMR (400MHz, $CDCl_3$): δ [ppm]= 1.41 (s, 8H), 2.76 (d, 2H), 3.38 (s, 3H), 3.50 - 3.59 (m, 3H), 3.62 - 3.71 (m, 5H), 3.71 - 3.75 (m, 2H), 3.80 (s, 1H), 3.83 - 3.88 (m, 2H), 4.07 - 4.15 (m, 2H), 6.83 - 6.88 (d, 2H), 7.10 (d, 2H).

**Intermediate 3-2B**

**methyl-O-{2-[2-(2-methoxyethoxy)ethoxy]ethyl}-L-tyrosinate**

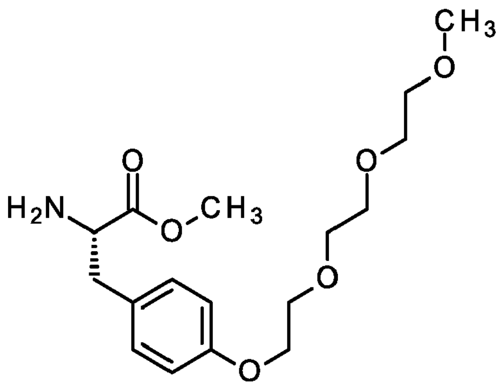

To a solution of methyl-*N*-(*tert*-butoxycarbonyl)-O-{2-[2-(2-methoxyethoxy)ethoxy]ethyl}-L-tyrosinate **(Intermediate 1-1B,** 22.8 g, 51.7 mmol) in DCM (170 ml) was added HCl (4N in dioxane) (65 ml, 4.0 M, 260 mmol) the mixture was stirred for 1,5 h at room temperature. The mixture was concentrated in vacuum. The residue was solved in DCM/EtOH (9+1) and washed with sodium hydrogen carbonate. The water phase was extracted once with DCM/EtOH (20%). The combined organic layer was washed with brine, dried by filtration through a water repellent filter and concentrated in vacuum to yield 14.1 g (79% purity, 63% yield) of the title compound.

LC-MS (method 4): $R_t$ = 0.84 min; MS (ESIpos): m/z = 342 $[M+H]^+$.

$^1$H NMR (400 MHz, $CDCl_3$) δ [ppm]= 2.81 (dd, 1H), 3.01 (dd, Hz, 1H), 3.38 (s, 3H), 3.52 - 3.57 (m, 2H), 3.63 -

3.75 (m, 12H), 3.85 (dd, 2H), 4.07 - 4.14 (m, 2H), 6.83 - 6.87 (m, 2H), 7.04 - 7.12 (m, 2 H).

**Intermediate 3-3A**

**(2*S*)-2-amino-3-(4-{2-[2-(2-methoxyethoxy)ethoxy]ethoxy}phenyl)propan-1-ol**

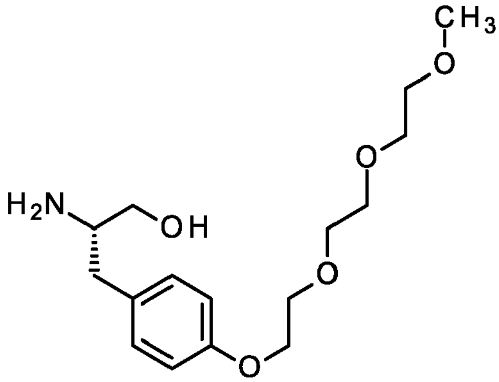

To *tert*-butyl-[(2*S*)-1-hydroxy-3-(4-{2-[2-(2-methoxyethoxy)ethoxy]ethoxy}phenyl)propan-2-yl]carbamate **(Intermediate 1-2A,** 3.95 g, 9.55 mmol) dissolved in DCM (32 ml) was added HCl in dioxane (12 ml, 4.0 M, 48 mmol; CAS:7647-01-0) and the mixture was stirred for 1 h at RT. The solvent was removed under reduced pressure. The residue was solved in DCM/EtOH (10%) and extracted with saturated $NaHCO_3$ solution. The aqueous phase and was extracted once with DCM/EtOH (10%) and twice with DCM/EtOH (20%). The aqueous phase was concentrated under reduced pressure, stirred with EtOH and solids were removed by filtration. The filtrate and the combined organic extracts were dried and concentrated under reduced pressure to yield 2,76 g (93% purity, 86% yield) of the title compound.

LC-MS (method 4): $R_t$ = 0.75 min; MS (ESIpos): m/z = 314 $[M+H]^+$.

$^1$H-NMR (400MHz, $CDCl_3$): δ [ppm]= 2.62 (br dd, 1H), 2.69 - 2.87 (m, 1H), 3.12 - 3.26 (m, 1H), 3.36 - 3.38 (m, 3H), 3.40 - 3.48 (dd, 1H), 3.53 - 3.59 (m, 2H), 3.59 - 3.71 (m, 6H), 3.71 - 3.75 (m, 2H), 3.82 - 3.89 (m, 2H), 4.04 - 4.15 (m, 2H), 6.82 - 6.89 (m, 2H), 7.05 - 7.15 (m, 2H).

**Intermediate 3-3B**

**methyl-O-{2-[2-(2-methoxyethoxy)ethoxy]ethyl}-N-(2-methoxy-2-oxoethyl)-L-tyrosinate**

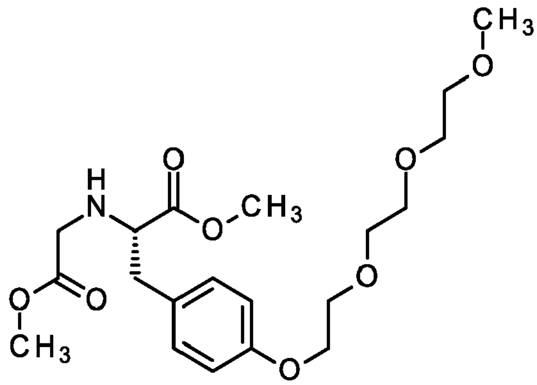

To a solution of methyl-O-{2-[2-(2-methoxyethoxy)ethoxy]ethyl}-L-tyrosinate **(Intermediate 1-2B,** 14.1 g, 41.4 mmol) acetonitrile (65 ml) was added potassium carbonate (8.58 g, 62.1 mmol) and methyl chloroacetate (3.6 ml, 41 mmol) and the mixture was stirred for 16 h at 50°C.The precipitate was filtered off and the filtrate was concentrated under reduced pressure. The residue was purified by column chromatography (silica, hexane / EtOAc 80-100% EtOAc)

to yield 3,71 g (84% purity, 18% yield) of the title compound.

LC-MS (method 4): $R_t$ = 0.97 min; MS (ESIpos): m/z = 414 $[M+H]^+$.

$^1H$ NMR (400 MHz, $CDCl_3$) δ [ppm]= 2.87 - 2.99 (m, 2 H) 3.31 - 3.45 (m, 5H), 3.52 - 3.59 (m, 3H), 3.64 - 3.75 (m, 12H), 3.81 - 3.87 (m, 2H), 4.07 - 4.12 (m, 2H) 6.82 - 6.86 (m, 2H) 7.06 - 7.12 (m, 2H).

Optical rotation:$[\alpha]_D$ = 7.7° +/- 0.8° (c = 4.7 mg/ml, 20°C, 589 nm, $CHCl_3$).

**Intermediate 3-4A**

**(2S)-2-[(*E*)-(4-methoxybenzyliden)amino]-3-(4-(2-[2-(2-methoxyethoxy)ethoxy]ethoxy}phenyl)propan-1-ol**

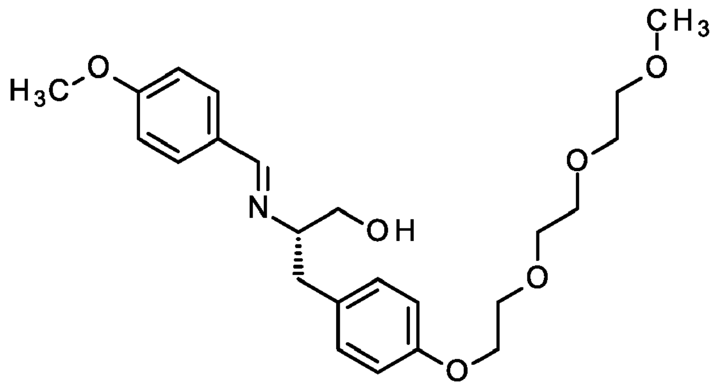

To (2*S*)-2-amino-3-(4-{2-[2-(2-methoxyethoxy)ethoxy]ethoxy}phenyl)propan-1-ol **(Intermediate 1-3A,** 2.28 g, 7.28 mmol) dissolved in toluene (30 ml) was added molecular sieve (5.1 g) and 4-methoxybenzaldehyde (890 µl, 7.3 mmol; CAS:123-11-5) and the mixture was stirred for 2 h at 90°C. Molecular sieve was removed by filtration, the filtrate was concentrated under reduced pressure to yield 3,35g (86% purity, 92% yield) of the title compound.

LC-MS (method 4): $R_t$ = 1.06 min; MS (ESIpos): m/z = 432 $[M+H]^+$

$^1H$-NMR (400MHz, $CDCl_3$): δ [ppm]= 2.35 (s, 1H), 2.65 - 2.66 (m, 1H), 2.75 - 2.94 (m, 2H), 3.36 - 3.39 (m, 3H), 3.52 - 3.57 (m, 4H), 3.63 - 3.70 (m, 4H), 3.70 - 3.74 (m, 2H), 3.79 - 3.83 (m, 2H), 3.84 - 3.85 (m, 3H), 4.05 - 4.09 (m, 2H), 6.77 - 6.80 (m, 2H), 6.98 - 7.06 (m, 4H), 7.63 (d, 2H), 7.91 - 7.94 (m, 1H).

**Intermediate 3-5A**

**(2*S*)-2-[(4-methoxybenzyl)amino]-3-(4-{2-[2-(2-methoxyethoxy)ethoxy]ethoxy}phenyl)propan-1-ol**

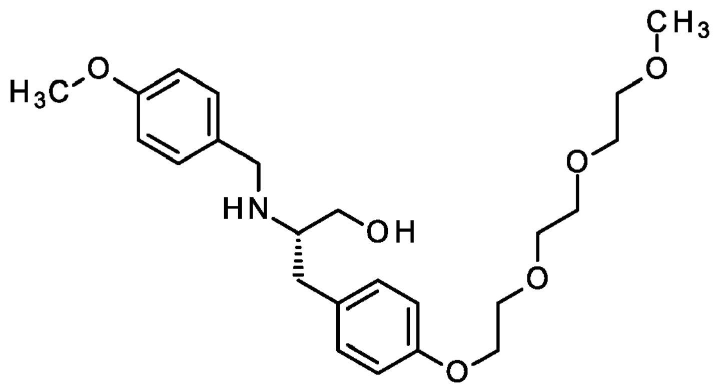

To (2S)-2-[(*E*)-(4-methoxybenzyliden)amino]-3-(4-{2-[2-(2-methoxyethoxy)ethoxy]ethoxy} phenyl)propan-1-ol **(Intermediate 1-4A,** 3.35 g, 7.76 mmol) dissolved in MeOH (23ml) was added sodium borohydride (441 mg, 11.6 mmol; CAS:16940-66-2) at 0°C and the mixture was stirred for 1h. Acetone (2,3 ml) was added and the mixture was warmed

to RT. The mixture was filtered over Celite, washed with MeOH and concentrated under reduced pressure. The residue was solved in DCM/EtOH (9+1) and extracted with water. The aqueous phase and was extracted with DCM/EtOH (9+1). The combined organic extract was washed with brine, dried and concentrated under reduced pressure to yield 2,68 g (93% purity, 74% yield) of the title compound.

LC-MS (method 4): $R_t$ = 1.06 min; MS (ESIpos): m/z = 434 $[M+H]^+$

$^1H$ NMR (400MHz, $CDCl_3$): δ [ppm]= 2.72 - 2.90 (m, 2H), 2.93 - 3.03 (m, 1H), 3.35 - 3.38 (s, 3H), 3.39 - 3.45 (m, 1H), 3.54 - 3.58 (m, 2H), 3.64 - 3.70 (m, 6H), 3.72 - 3.75 (m, 3H), 3.74 - 3.76 (m, 1H), 3.77 - 3.79 (m, 4H), 3.84 - 3.88 (m, 2H), 4.08 - 4.13 (m, 2H), 6.82 - 6.86 (m, 4H), 7.03 - 7.08 (m, 2H), 7.18 - 7.23 (m, 2H).

## Intermediate 3-6A

**(2S)-1-(4-methoxybenzyl)-2-(4-{2-[2-(2-methoxyethoxy)ethoxy]ethoxy}benzyl)aziridine**

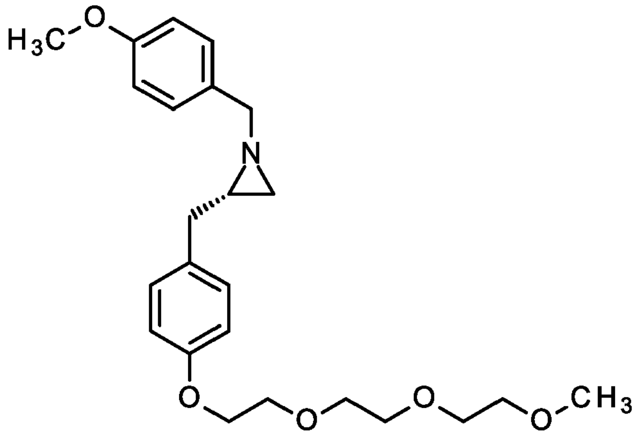

To (2S)-2-[(4-Methoxybenzyl)amino]-3-(4-{2-[2-(2-methoxyethoxy)ethoxy]ethoxy}phenyl) propan-1-ol **(Intermediate 1-5A,** 500 mg, 1.15 mmol) solved in THF (4,5 ml) was added triphenylphosphine (423 mg, 1.61 mmol; CAS:603-35-0) and di-*tert*-butyl-(*E*)-diazen-1,2-dicarboxylate (372 mg, 1.61 mmol; CAS:870-50-8) at 0°C and the mixture was stirred for 16 h while it warmed to RT. One half of the solvent was removed under reduced pressure, hexane (10 ml) was added and the mixture was stirred for 1,5 h while a precipitate formed. The precipitate was removed by filtration. The filtrate was concentrated under reduced pressure and purified by column chromatography (silica gel, hexane / EtOAc, 40-100%) to yield 182 mg (85% purity, 32% yield) of the title compound.

LC-MS (method 4): $R_t$ = 1.20 min; MS (ESIpos): m/z = 416 $[M+H]^+$

$^1H$ NMR (400MHz, $CDCl_3$): δ [ppm]= 1.41 - 1.57 (m, 2H), 1.68 - 1.73 (m, 1H), 2.50 - 2.60 (m, 1H), 2.67 - 2.79 (m, 1H), 3.26 - 3.46 (m, 2H), 3.38 (s, 3H), 3.53 - 3.58 (m, 2H), 3.63 - 3.71 (m, 4H), 3.71 - 3.78 (m, 2H), 3.78 - 3.87 (m, 5H), 4.08 - 4.12 (m, 2H), 6.75 - 6.86 (m, 4H), 7.03 - 7.08 (m, 2H), 7.15 - 7.22 (m, 2H).

## Cyclen intermediate 3-1A

**(2*S*)-1,7,10-tribenzyl-4-(4-methoxybenzyl)-2-(4-{2-[2-(2-methoxyethoxy)ethoxy]ethoxy}benzyl)-1,4,7,10-tetraazacyclododecane**

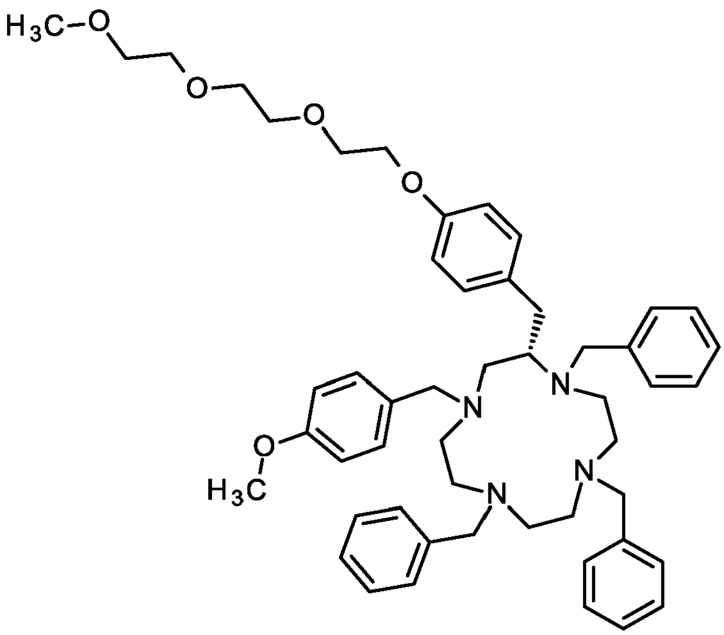

To (2S)-1-(4-methoxybenzyl)-2-(4-{2-[2-(2-methoxyethoxy)ethoxy]ethoxy}benzyl)aziridine **(Intermediate 1-6A,** 1.00 g, 2.41 mmol) and 1-benzylaziridin (20 ml, 0.49 M, 9.6 mmol) (emulsion in water 0,065 g/ml) dissolved in EtOH (13ml) was slowly added p-toluenesulfonic acid monohydrate (572 mg, 3.0 mmol; CAS: 6192-52-5,) in water (0,3 ml) over 8 h at 60°C followed by additional stirring at 60°C for 7 h and reflux for 2 h. After cooling to RT sodium hydroxide (179 mg, 4.47 mmol) solved in water (360 μl) was added and the reaction mixture was extracted twice with DCM. The combined organic extracts were washed with brine, dried, concentrated under reduced pressure and purified by column chromatography (aminophase functionalized silica gel, hexane / EtOAc, 40-100%) to yield 1,02 g (56% purity, 29% yield) of the title compound.

LC-MS (method 1): $R_t$ = 1.26 min; MS (ESIpos): m/z = 815 $[M+H]^+$

**Cyclen intermediate 3-1B**

**(3*S*)-3-(4-{2-[2-(2-methoxyethoxy)ethoxy]ethoxy}benzyl)-1,4,7,10-tetraazacyclododecan-2,6-dione**

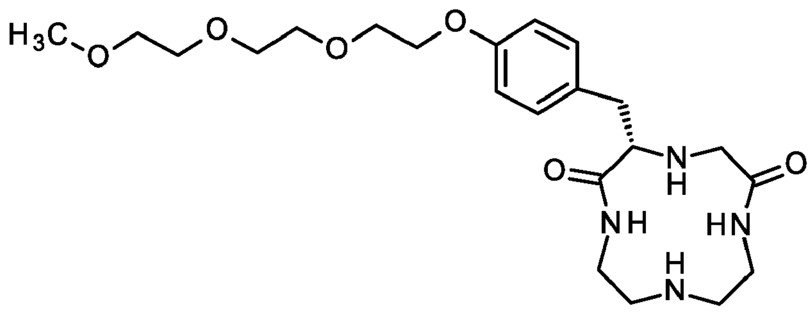

To a solution of Methyl-O-{2-[2-(2-methoxyethoxy)ethoxy]ethyl}-N-(2-methoxy-2-oxoethyl)-L-tyrosinate **(Intermediate 1-3B,** 10.5 g, 25.3 mmol) in Methanol (250ml) was added N-(2-aminoethyl)ethane-1,2-diamine (2.7 ml, 25 mmol) and the mixture was stirred for 30 days at 40°C. The mixture was concentrated under reduced pressure and purified by column chromatography (aminophase functionalized silica gel, 380 g, DCM / EtOH, 0-10%) to yield 4.32 g (41% purity, 15% yield) of the title compound.

LC-MS (method 1): $R_t$ = 0.55 min; MS (ESIpos): m/z = 453 $[M+H]^+$

**Cvclen intermediate 3-2**

**(2*S*)-2-(4-{2-[2-(2-methoxyethoxy)ethoxy]ethoxy}benzyl)-1,4,7,10-tetraazacyclododecane**

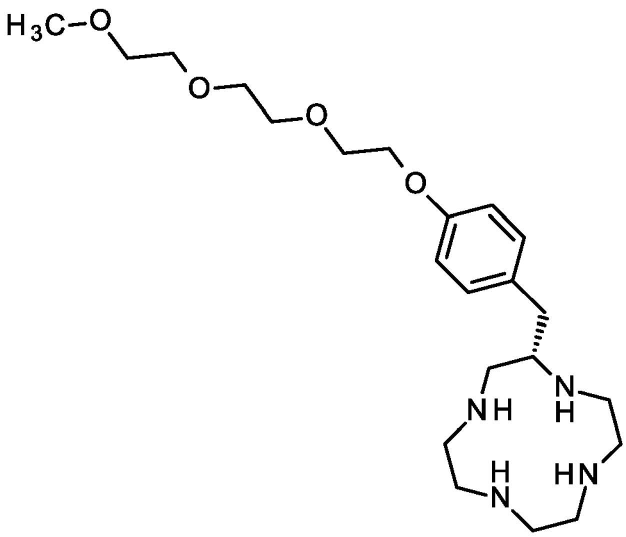

To (2S)-1,7,10-Tribenzyl-4-(4-methoxybenzyl)-2-(4-{2-[2-(2-methoxyethoxy)ethoxy]ethoxy} benzyl)-1,4,7,10-tetraazacyclododecane **(Cyclen intermediate 1-1A**,1.02 g, 1.25 mmol) dissolved in EtOH (13ml) and acetic acid (13ml) was added palladium on activated carbon (200 mg, 10%, 188 μmol; CAS-RN:7440-05-3) and stirred under hydrogen pressure (40 bar) at 80°C for 42 h. The catalyst was removed by filtration and washed with EtOH and THF. The filtrate was concentrated under reduced pressure to yield 1,87 g (30% purity, 77% yield) of the title compound.

LC-MS (method 1): $R_t$ = 0,57 min; MS (ESIpos): m/z = 425 $[M+H]^+$

**Cyclen intermediate 3-2 alternative procedure**

**(2*S*)-2-(4-{2-[2-(2-methoxyethoxy)ethoxy]ethoxy}benzyl)-1,4,7,10-tetraazacyclododecan**

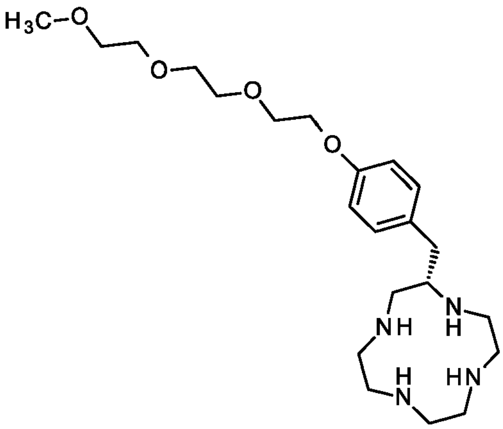

To a suspension of (3*S*)-3-(4-{2-[2-(2-methoxyethoxy)ethoxy]ethoxy}benzyl)-1,4,7,10-tetraaza cyclododecan-2,6-dione **(Cyclen intermediate 1-1B,** 4.32 g, 9.55 mmol) in THF (158 ml) was added Diisobutyl aluminium hydride in toluene (190 ml, 1.0 M, 190 mmol) dropwise and the resulting mixture was stirred 1h at RT. The mixture was cooled to 0°C, toluene / THF (1:1, 320 ml), water (14 ml) and sodium fluoride (32.1 g, 764 mmol) were added and stirring was continued over night at RT. The solid was filtered off and washed with toluene / THF (1:1) und THF. The filtrate was

concentrated under reduced pressure to yield 1,82 g (70% purity, 31% yield) of the title compound.

LC-MS (method 1): $R_t$ = 0.50 min; MS (ESIpos): m/z = 425 $[M+H]^+$

## Cyclen intermediate 3-3

**tetra-*tert*-butyl-2,2',2",2'"-[(2*S*)-2-(4-{2-[2-(2-methoxyethoxy)ethoxy]ethoxy}benzyl)-1,4,7,10-tetraazacyclododecane-1,4,7,10-tetrayl]tetraacetate**

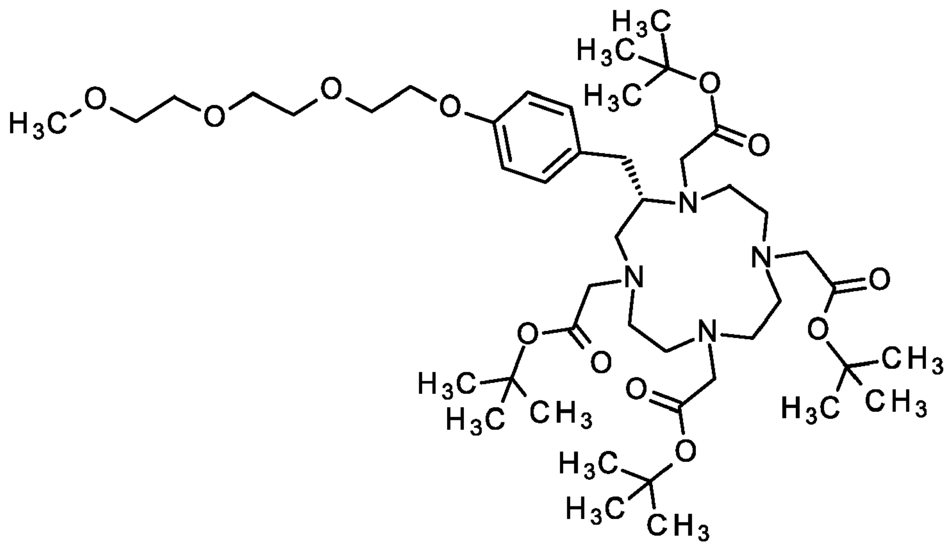

To (2S)-2-(4-{2-[2-(2-methoxyethoxy)ethoxy]ethoxy}benzyl)-1,4,7,10-tetraazacyclododecane (1.37 g, 3.23 mmol) solved in acetonitrile (42 ml) was added potassium carbonate (4,46 g, 32,2 mmol; CAS-RN:584-08-7) and the mixture was stirred for 1h at 50°C. *Tert*-butyl-bromoacetate (3.0 ml, 21 mmol) was added and stirring at RT was continued for 3 days. *Tert*-butyl methyl ether and water were added and phases were separated. The aqueous phase was extracted with *tert*-butyl methyl ether. The combined organic extracts were washed with aqueous sodium hydroxide solution (1M) and brine, dried and concentrated under reduced pressure to yield 2,84 g (43% purity, 43% yield) of the title compound.

LC-MS (method 1): $R_t$ = 1.31 min; MS (ESIpos): m/z = 882 $[M+H]^+$

## Free ligand 3

**2,2',2",2'"-[(2*S*)-2-(4-(2-[2-(2-methoxyethoxy)ethoxy]ethoxy)benzyl)-1,4,7,10-tetraazacyclododecane-1,4,7,10-tetrayl]tetraacetic acid**

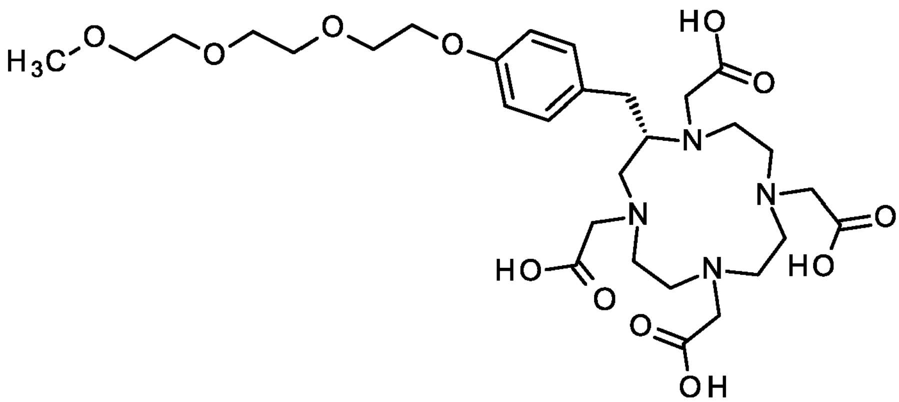

Tetra-tert-butyl-2,2',2",2"'-[(2*S*)-2-(4-{2-[2-(2-methoxyethoxy)ethoxy]ethoxy}benzyl)-1,4,7,10-tetraazacyclododecane-1,4,7,10-tetrayl]tetraacetate (2.84 g, 3.22 mmol) was solved in formic acid (41 ml, 1.1 mol; CAS-RN:64-18-6) and stirred for 18h at 70°C. The reaction mixture was concentrated under reduced pressure and co-distilled twice with toluene / EtOH to yield 2,19 g (60% purity, 62% yield) of the title compound,

LC-MS (method 1): $R_t$ = 0,63 min; MS (ESIpos): m/z = 657 $[M+H]^+$.

## Complex formation 3

**Gadolinium-2,2',2"-[(2*S*)-10-(carboxymethyl)-2-(4-{2-[2-(2-methoxyethoxy)ethoxy] ethoxy}benzyl)-1,4,7,10-tetraazacyclododecane-1,4,7-triyl]triacetate**

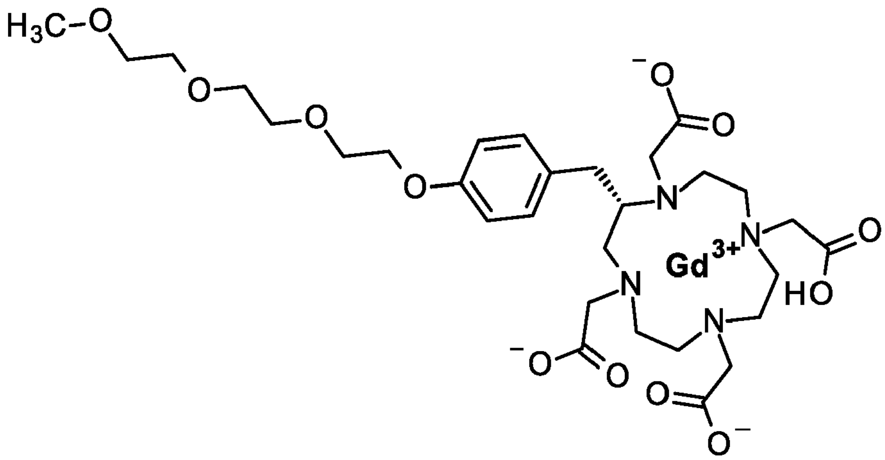

To 2,2',2",2"'-[(2S)-2-(4-{2-[2-(2-methoxyethoxy)ethoxy]ethoxy}benzyl)-1,4,7,10-tetraaza cyclododecane-1,4,7,10-tetrayl]tetraacetic acid (free ligand 1, 2.19 g, 3.33 mmol) suspended in water (54 ml) was added gadolinium oxide (543mg, 1,50 mmol) and pH5 was adjusted by addition of aqueous sodium hydroxide (2 M) and stirred at 100°C for 2h. Chelex was added and the mixture was stirred at RT for 21h, while the pH value was constantly adjusted to 5 by addition of hydrochloric acid (2 M). The mixture was filtered and purified by column chromatography (Biotage®Sfär C18 D, water / acetonitrile, 0-100%) to yield 50,9 mg (98% purity, 2% yield) of the title compound.

LC-MS (method 1): $R_t$ = 0.64 min; MS (ESIneg): m/z = 810 $[M-H]^-$.

## Example 4

**Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetate**

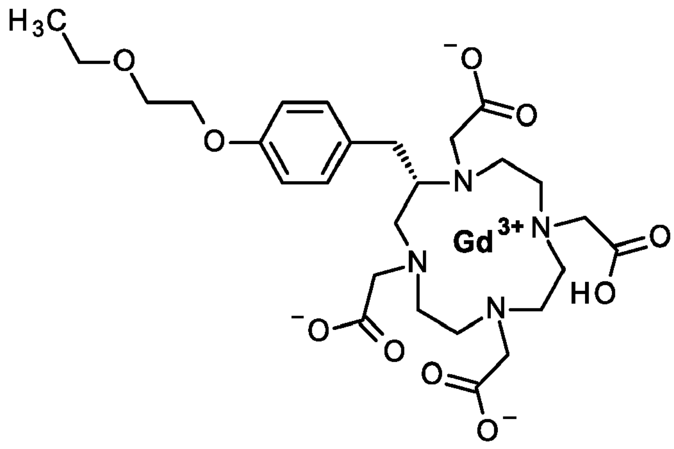

<u>**Intermediate 4-1**</u>

**methyl-*N*-(*tert*-butoxycarbonyl)-O-(2-ethoxyethyl)-L-tyrosinate**

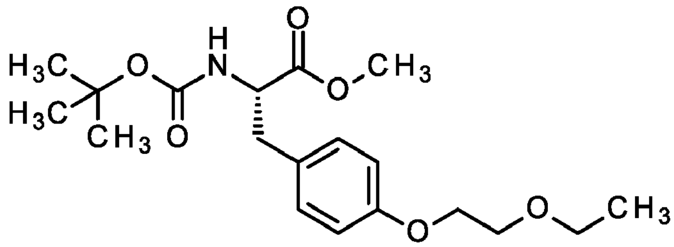

To a solution of methyl *N*-(*tert*-butoxycarbonyl)-L-tyrosinate (60 g, 203 mmol) in *N,N*-dimethyl formamide (150 ml) were added potassium carbonate (28.1 g, 203 mmol) and 1-bromo-2-ethoxyethane (34 ml, 300 mmol) at room temperature and the mixture was stirred 60°C for 16 hours. Ethyl acetate and water were added and layers were separated. The aqueous phase was extracted with ethyl acetate. The combined extract was washed with brine, dried over sodium sulfate, filtered, and concentrated under reduced pressure to give a residue. The residue was purified by column chromatography (silica gel, hexane / ethyl acetate, 0 - 5%) to yield 63,2 g (85% yield) of the title compound.

LC-MS (method 13): $R_t$ = 0.94 min; MS (ESIpos): m/z = 268 $[M-BOC+H]^+$.

<u>**Intermediate 4-2**</u>

***tert*-butyl-{(2*S*)-1-[4-(2-ethoxyethoxy)phenyl]-3-hydroxypropan-2-yl}carbamate**

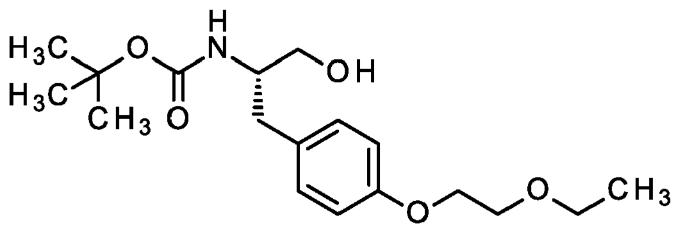

To a solution of methyl-*N*-(*tert*-butoxycarbonyl)-O-(2-ethoxyethyl)-L-tyrosinate (63.2 g, 172 mmol) in tetrahydrofuran (650 ml) was added lithium aluminum hydride (7.63 g, 206 mmol) at 0 °C. The mixture was stirred at room temperature for 2 hours under nitrogen. The mixture was quenched with saturated ammonium chloride solution and saturated potassium sodium tartrate tetrahydrate solution at 0°C. The mixture was stirred for 1 hour at room temperature

and then extracted with ethyl acetate. The combined organic phase was washed with brine, dried over anhydrous sodium sulfate and concentrated under reduced pressure to yield 58 g (99% yield) of the title compound.

LC-MS (method 13): $R_t$ = 0.84 min; MS (ESIpos): m/z = 240 $[M-BOC+H]^+$.

## Intermediate 4-3

**(2S)-2-amino-3-[4-(2-ethoxyethoxy)phenyl]propan-1-olhydrochloride (1:1)**

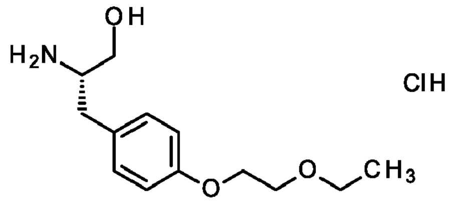

To a solution of *tert*-butyl-{(2*S*)-1-[4-(2-ethoxyethoxy)phenyl]-3-hydroxypropan-2-yl}carbamate (48.0 g, 141 mmol) in methyl acetate (100 ml) was added hydrochloric acid (220 ml, 4.0 M in ethyl acetate, 880 mmol) at room temperature. The mixture was stirred at room temperature for 1 hour. The mixture was concentrated under reduced pressure to yield 43.9 g (113% yield) of the title compound.

LC-MS (method 13): $R_t$ = 0.73 min; MS (ESIpos): m/z = 240 $[M+H]^+$.

## Intermediate 4-4

**(2S)-3-[4-(2-ethoxyethoxy)phenyl]-2-[(4-methoxybenzyl)amino]propan-1-ol**

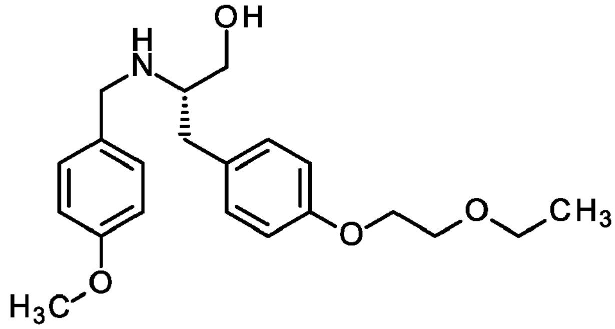

To a solution of (2S)-2-amino-3-[4-(2-ethoxyethoxy)phenyl]propan-1-ol hydrochloride (43.9 g, 159 mmol) in methanol was added sodium acetate (13.1 g, 159 mmol) at room temperature. After 0.5 h acetic acid (57 ml), 4-methoxybenzaldehyde (19 ml, 160 mmol) and sodium cyanoborohydride (15.0 g, 239 mmol) were added at room temperature. The mixture was stirred at 50 °C for 16 hours under nitrogen. The mixture was quenched with saturated sodium bicarbonate solution, and then concentrated to give a residue. The residue was diluted with water, and then extracted with dichloromethane. The combined organic phase was washed with brine, dried over anhydrous sodium sulfate, filtered, concentrated and purified by preparative HPLC (Instrument: Agela-H1000GC500; Column: Phenomenex luna C18 I.D.150mm*H400mm, 15μm; 100 Å; eluent A: water (0.1% formic acid), eluent B: acetonitrile; gradient: 15%-22%,5min;22%,20min; flow 400 ml/min; temperature: room temperature; Detector: UV 220/254 nm) to yield 18,1 g (32% yield) of the title compound.

LC-MS (method 13): $R_t$ = 0.78 min; MS (ESIpos): m/z = 360 $[M+H]^+$.

$^1H$ NMR (400 MHz, DMSO-$d_6$): $\delta$ [ppm] = 7.17 (d, 2H), 7.07 (d, 2H), 6.83 (d, 4H), 4.48 (t, 1H), 4.07-4.00 (m, 2H), 3.72 (s, 3H), 3.69-3.64 (m, 4H), 3.49 (q, 2H), 3.35-3.17 (m, 3H), 2.69-2.53 (m, 3H), 1.12 (t, 3H).

**Intermediate 4-5**

**(2*S*)-2-[4-(2-ethoxyethoxy)benzyl]-1-(4-methoxybenzyl)aziridine**

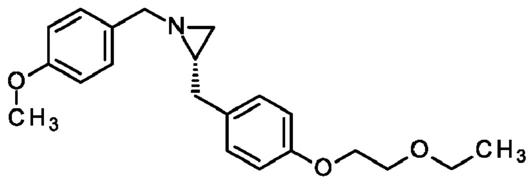

To a solution of (2*S*)-3-[4-(2-ethoxyethoxy)phenyl]-2-[(4-methoxybenzyl)amino]propan-1-ol (11.5 g, 32.0 mmol) in tetrahydrofuran (200 ml) were added tri-n-butylphosphine (12 ml, 48 mmol) and (*E*)-*N,N,N',N'*-tetramethyldiazene-1,2-dicarboxamide (8.26 g, 48.0 mmol) at room temperature. The mixture was stirred at room temperature for 2 hours under nitrogen and filtered. The filtrate was concentrated and purified by flash column chromatography (100-200 mesh, hexane / ethyl acetate, 0 to 50%) to yield 11,5 g (97% purity, quantitative) of the title compound.

LC-MS (method 13): $R_t$ = 0.77 min; MS (ESIpos): m/z = 342 $[M+H]^+$

$^1$H NMR (400 MHz, DMSO-$d_6$): δ [ppm]= 7.19 (d, 2H), 7.08 (d, 2H), 6.85-6.78 (m, 4H), 4.06-4.00 (m, 2H), 3.73 (s, 3H), 3.71-3.64 (m, 2H), 3.49 (q, 2H), 3.33-3.17 (m, 2H), 2.63-2.56 (m, 1H), 2.49-2.43 (m, 1H), 1.72-1.62 (m, 1H), 1.53 (d, 1H), 1.34 (d, 1H), 1.12 (t, 3H).

**Cyclen intermediate 4-1**

**(2S)-1,7,10-tribenzyl-2-[4-(2-ethoxyethoxy)benzyl]-4-(4-methoxybenzyl)-1,4,7,10-tetraazacyclododecane**

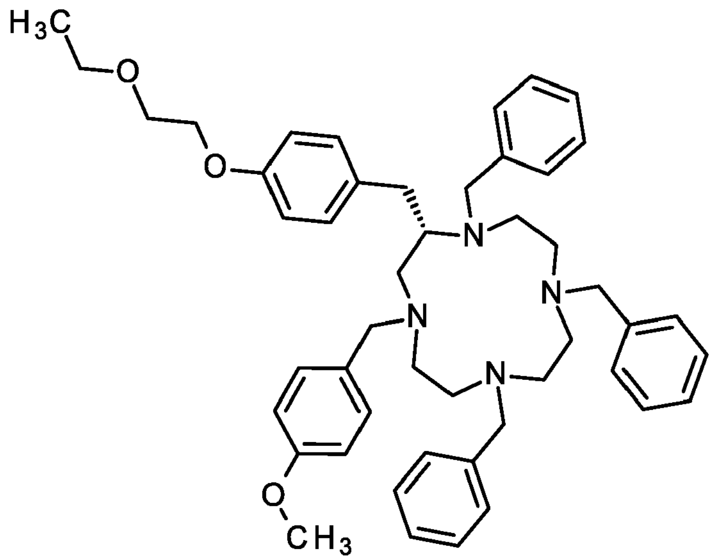

To (2S)-2-[4-(2-ethoxyethoxy)benzyl]-1-(4-methoxybenzyl)aziridine (3.00 g, 8.79 mmol) and 1-benzylaziridine (72 ml, 0.49M, 35 mmol) (emulsion in water 0,065 g/ml) solved in EtOH (47 ml) was added slowly p-toluenesulfonic acid monohydrate (2.09 g, 11.0 mmol) in water (1 ml) over 8 h at 60°C followed by additional stirring at 60°C for 7h and reflux for 2h. After cooling to RT sodium hydroxide (653 mg, 16.3 mmol) in water (1,3 ml) was added and the reaction mixture was extracted twice with DCM. The combined organic extract was washed with brine, dried, concentrated under reduced pressure and purified by column chromatography (aminophase functionalized silica gel, hexane / EtOAc, 40-100%) to yield 930 mg (5,2% yield) of the title compound.

LC-MS (method 1): $R_t$ = 1.37 min; MS (ESIpos): m/z = 741 $[M+H]^+$.

## Cyclene intermediate 4-2

**(2S)-2-[4-(2-ethoxyethoxy)benzyl]-1,4,7,10-tetraazacyclododecane**

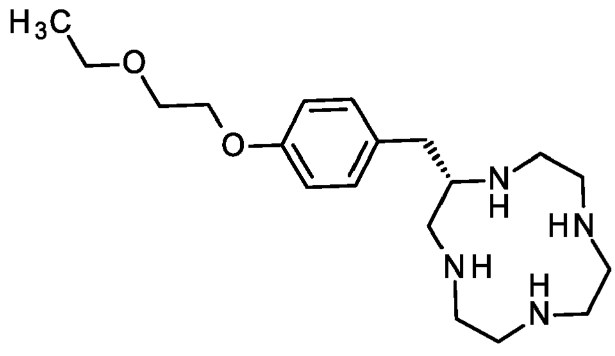

To (2S)-1,7,10-tribenzyl-2-[4-(2-ethoxyethoxy)benzyl]-4-(4-methoxybenzyl)-1,4,7,10-tetraaza cyclododecane (930 mg, 1.25 mmol) solved in EtOH (13 ml) and acetic acid (13 ml) was added palladium on activated carbon (200 mg, 10% purity, 188 μmol) and the mixture was stirred under hydrogen pressure (40 bar) at 80°C for 42 h. The catalyst was removed by filtration and washed with EtOH and THF. The filtrate was concentrated under reduced pressure to yield 897 mg (48% purity, 98% yield) of the title compound.

LC-MS (method 1): $R_t$ = 0.52 min; MS (ESIpos): m/z = 351 $[M+H]^+$

## Cyclene intermediate 4-3

**Tetra-tert-butyl-2,2',2'',2'''-{(2S)-2-[4-(2-ethoxyethoxy)benzyl]-1,4,7,10-tetraaza cyclododecane-1,4,7,10-tetrayl}tetraacetate**

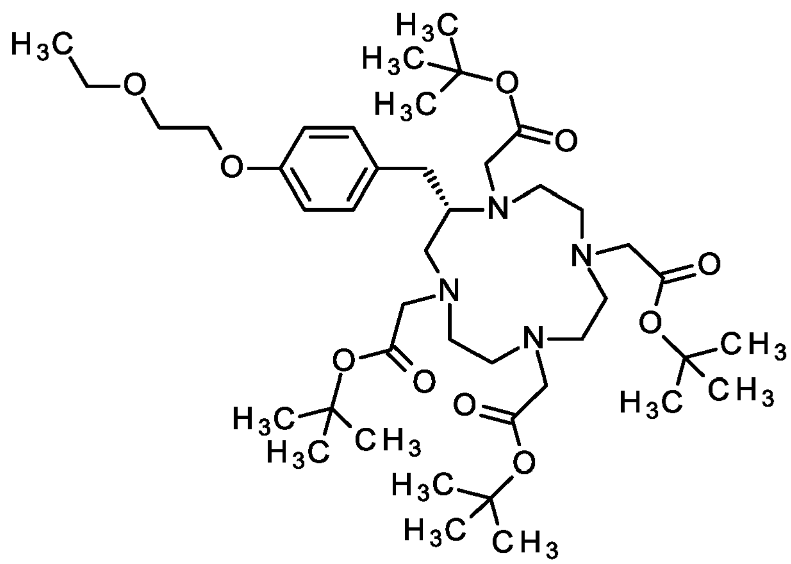

To (2S)-2-[4-(2-ethoxyethoxy)benzyl]-1,4,7,10-tetraazacyclododecan (897 mg, 2.56 mmol) solved in acetonitrile (34 ml) was added potassium carbonate (3.54 g, 25.6 mmol) and stirred for 1h at 50°C. *Tert*-butyl bromoacetate (2.1 ml, 14 mmol) was added and stirred at RT for 90h. *Tert*-butyl methyl ether and water were added and phases were separated. The aqueous phase was extracted with *tert*-butyl methyl ether. The combined organic extracts were washed with aqueous sodium hydroxide solution (1M) and brine, dried and concentrated under reduced pressure to yield 1,37g of (46% purity, 30% yield) of the title compound.

LC-MS (method 1): $R_t$ = 1.32 min; MS (ESIpos): m/z = 808 $[M+H]^+$

**Free ligand 4**

**2,2',2",2"'-((2*S*)-2-[4-(2-ethoxyethoxy)benzyl]-1,4,7,10-tetraazacyclododecane-1,4,7,10-tetrayl}tetraacetic acid**

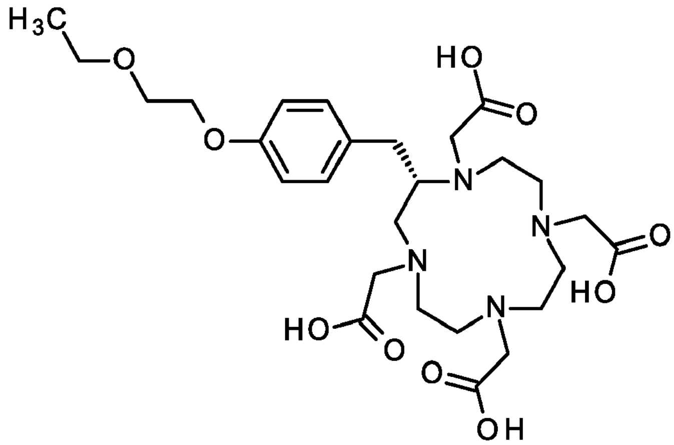

Tetra-*tert*-butyl-2,2',2",2"'-{(2*S*)-2-[4-(2-ethoxyethoxy)benzyl]-1,4,7,10-tetraazacyclododecane-1,4,7,10-tetrayl}tetraacetate (1.37 g, 1.70 mmol) was solved in formic acid (22 ml, 580 mmol) and stirred for 4 h at 70°C. The reaction mixture was concentrated under reduced pressure and co-distilled twice with toluene / EtOH to yield 1,09 g (50% purity, 55% yield) of the title compound.

LC-MS (method 1): $R_t$ = 0.62 min; MS (ESIpos): m/z = 583 $[M+H]^+$.

**Complex formation 4**

**Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetate**

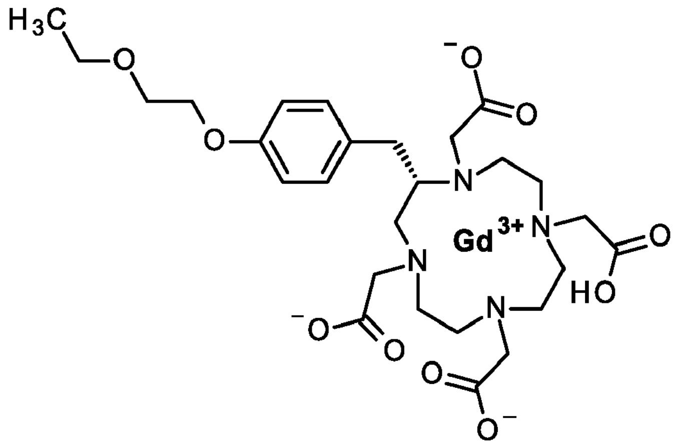

To 2,2',2",2"'-{(2S)-2-[4-(2-ethoxyethoxy)benzyl]-1,4,7,10-tetraazacyclododecane-1,4,7,10-tetrayl}tetra acetic acid (free ligand 4, 1.09 g, 1.87 mmol) in water (30 ml) was added gadolinium oxide (305 mg, 842 µmol) and pH5 was adjusted by addition of aqueous sodium hydroxide (2 M) and stirred at 100°C for 1h. Chelex was added and the mixture was stirred at RT for 16 h. The mixture was filtered and purified by column chromatography (Biotage®Sfär C18 D, water / acetonitrile, 0-100%) to yield 185 mg (95% purity, 13% yield) of the title compound.

LC-MS (method 1): $R_t$ = 0.63 min; MS (ESIpos): m/z = 737 $[M+H]^+$.

**Example A - Relaxivity measurements at 1.4 T**

Relaxivity measurements at 1.41 T were performed using a MiniSpec mq60 spectrometer (Bruker Analytik, Karlsruhe, Germany) operating at a resonance frequency of 60 MHz and a temperature of 37°C. The $T_1$ relaxation times were determined using the standard inversion recovery (IR) method with a fixed relaxation delay of at least 5 × $T_1$. The variable inversion time (TI) was calculated automatically by the standard software of the MiniSpec mq60 (8 steps). The $T_2$ measurements were performed by using a Carr-Purcell-Meiboom-Gill (CPMG) pulse sequence, applying a relaxation delay of at least 5 × $T_1$.

Each relaxivity measurement was performed using three different Gd concentrations (3 concentrations between 0.05 and 2 mM). The $T_1$ and $T_2$ relaxation times of the example compounds were measured in water and human plasma. Human plasma preparation: For each experiment fresh blood was taken from a volunteer using 10 mL citrate-tubes (Sarstedt S-Monovette 02.1067.001, 10 mL, Citrate). The 10 mL citrate- tubes were carefully inverted 10 times to mix blood and anticoagulant and centrifuged for 15min at 1811 g at RT (Eppendorf, Centrifuge 5810R).

The relaxivities $r_i$ (where i=1, 2) were calculated based on the measured relaxation rates $R_i$ in water and plasma:

$$r_i = (R_i - R_{i(0)}) / C_{Gd}$$

where $R_{i(0)}$ represent the relaxation rate of the respective solvent and $C_{Gd}$ the concentration of the compound normalized to the Gadolinium. The Gadolinium concentrations of the investigated solutions were verified by Inductively Coupled Plasma Mass Spectrometry (ICP-MS Agilent 7500a, Waldbronn, Germany). The determined relaxivity values are summarized in Table 1 and 2.

**Table 1:** Relaxivities of investigated extracellular contrast agents in water and human plasma at 1.41 T and relaxivities of Reference compounds 1 and 2 (RC1 and RC2) at 1.5 T in water and bovine plasma. All values are measured at 37°C and normalized to Gd and given in L $mmol^{-1}$ $s^{-1}$.

| No. | Structure | $r_1$ water* | $r_2$ water* | $r_1$ human plasma* | $r_2$ human plasma* |
|---|---|---|---|---|---|
| 1 | 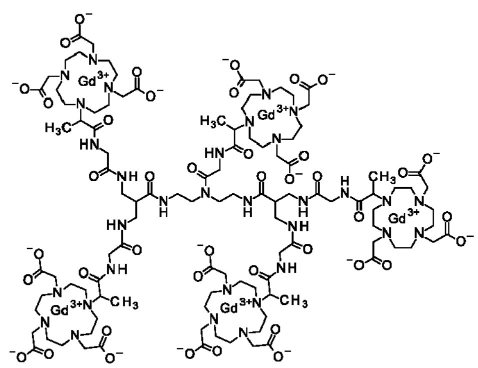 | 11.1 | 12.9 | 13.0 | 19.5 |
| 2 | 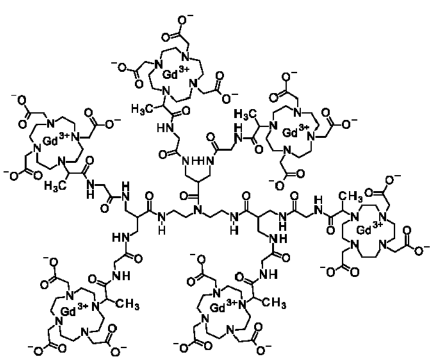 | 12.1 | 14.2 | 13.9 | 17.6 |

(continued)

| No. | Structure | $r_1$ water* | $r_2$ water* | $r_1$ human plasma* | $r_2$ human plasma* |
|---|---|---|---|---|---|
| 3 | 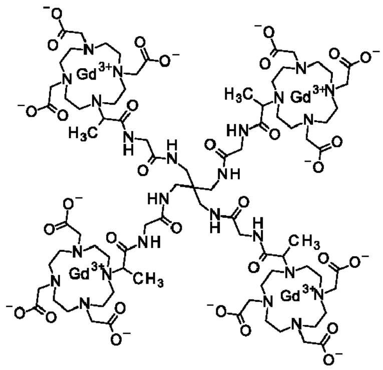 | 10.1 | 11.7 | 11.8 | 14.7 |
| 4 | 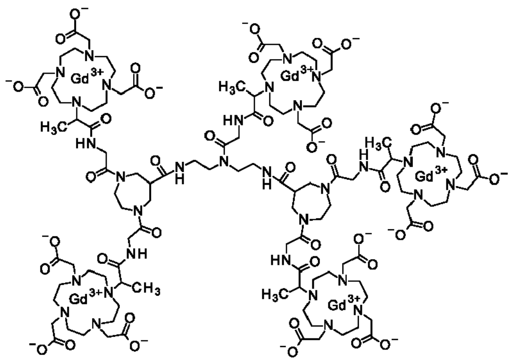 | 11.5 | 13.5 | 13.2 | 16.5 |
| 5 | 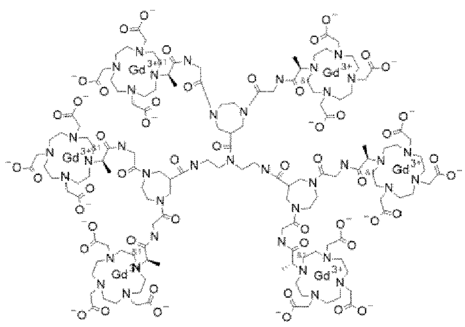 | 13.0 | 15.2 | 14.3 | 17.7 |
| 6 | 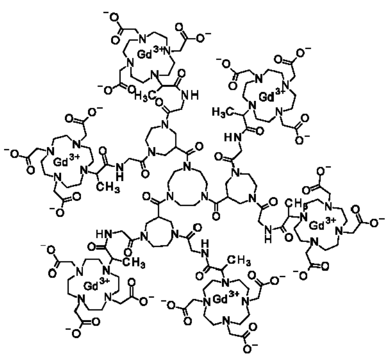 | 13.4 | 15.7 | 14.6 | 18.6 |

(continued)

| No. | Structure | $r_1$ water* | $r_2$ water* | $r_1$ human plasma* | $r_2$ human plasma* |
|---|---|---|---|---|---|
| 7 | 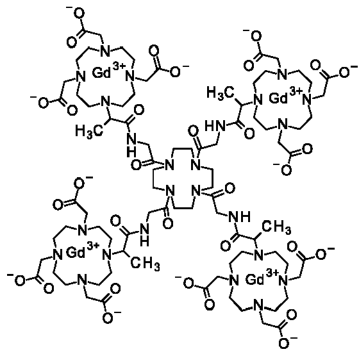 | 10.8 | 12.6 | 12.1 | 14.9 |
| 8 | 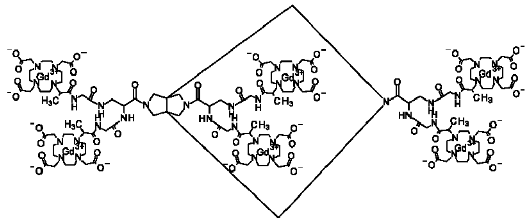 | 12.5 | 14.5 | 14.6 | 18.1 |
| 9 | 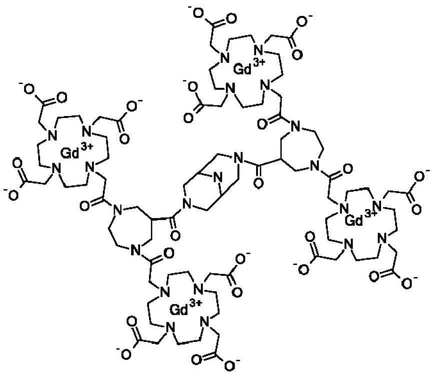 | 7.4 | 8.5 | n.d. | n.d. |
| 10 | 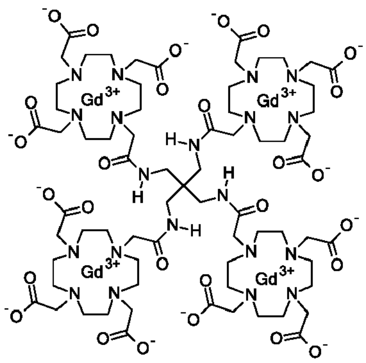 | 7.3 | 8.3 | 9.7 | 11.3 |

(continued)

| No. | Structure | $r_1$ water* | $r_2$ water* | $r_1$ human plasma* | $r_2$ human plasma* |
|---|---|---|---|---|---|
| 11 | 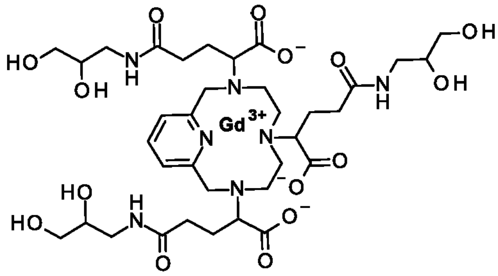 | 12.2** | 15.0** | 12.8** | 15.1** |
| RC 1 | 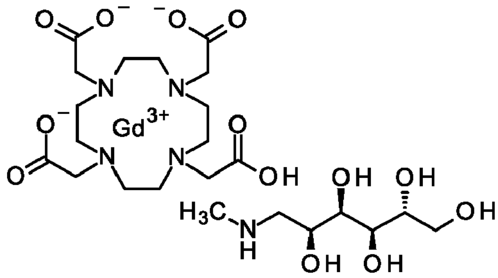 RC1 | 2.9# | 3.2# | 3.6# | 4.3# |
| RC 2 | 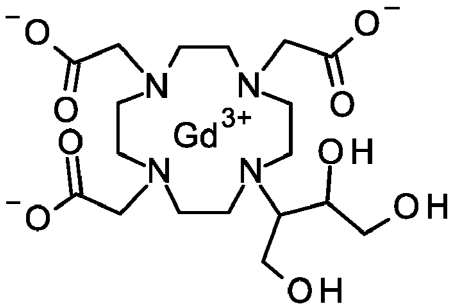 | 3.3# | 3.9# | 5.2# | 6.1# |
| * values are given in L·mmol$^{-1}$·s$^{-1}$<br>** Robic et al. Invest Radiol. 2019 Aug;54(8):475-484. doi: 10.1097/RLI.0000000000000563<br># Invest Radiol. 2005 Nov;40(11):715-24. doi: 10.1097/01.rli.0000184756.66360.d3, in bovine plasma (Kreaber GmbH, Pharmaceutical Raw Material, Ellerbek, Germany) instead of human plasma | | | | | |

**Table 2:** Relaxivities of investigated liver-specific contrast agents in water and human plasma at 1.41 T and relaxivities of Reference compounds 3 and 4 (RC3 and RC4) at 1.5 T in water and bovine plasma. All values are measured at 37°C and normalized to Gd and given in L mmol$^{-1}$ s$^{-1}$.

| No. | Structure | $r_1$ water* | $r_2$ water* | $r_1$ human plasma* | $r_2$ human plasma* |
|---|---|---|---|---|---|
| 12 | 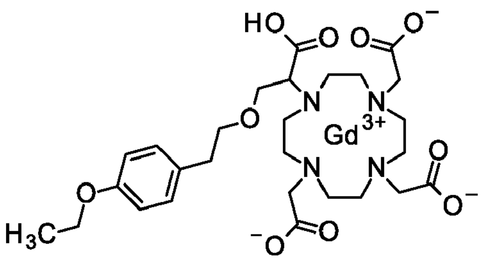 | 4.1 | 4.8 | 6.5 | 10.0 |

(continued)

| No. | Structure | $r_1$ water* | $r_2$ water* | $r_1$ human plasma* | $r_2$ human plasma* |
|---|---|---|---|---|---|
| 13 | 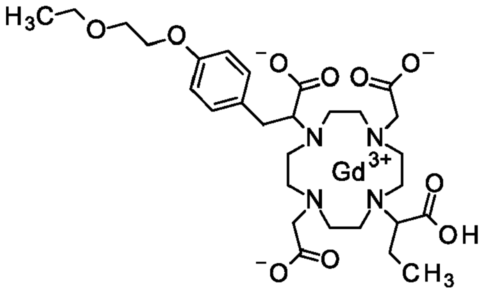 | 4.5 | 5.3 | 7.4 | 11.7 |
| 14 | 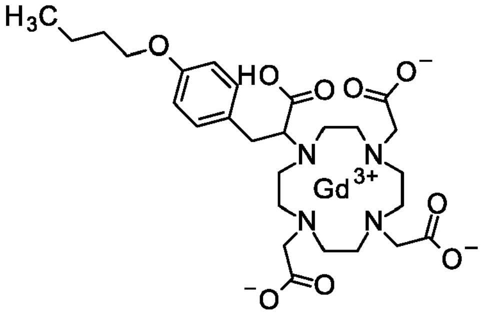 | 4.3 | 5.1 | 13.3 | 26.0 |
| 15 | 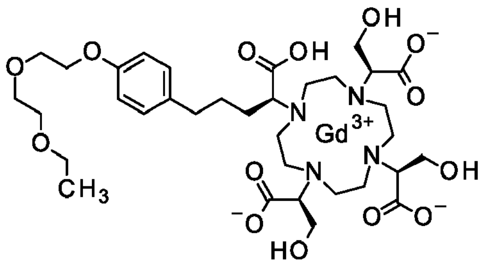 | 5.1 | 6.2 | 8.7 | 13.7 |
| 16 | 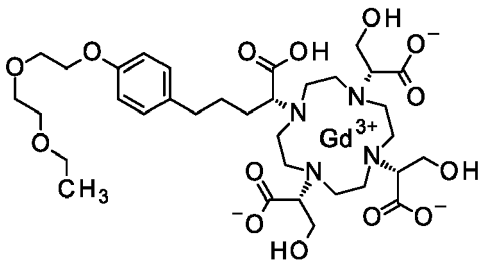 | 5.3 | 6.2 | 9.0 | 16.6 |
| 17 | 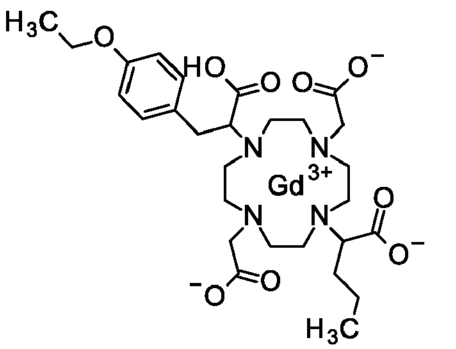 | 4.4 | 5.2 | 7.5 | 12.2 |

(continued)

| No. | Structure | $r_1$ water* | $r_2$ water* | $r_1$ human plasma* | $r_2$ human plasma* |
|---|---|---|---|---|---|
| 18 | 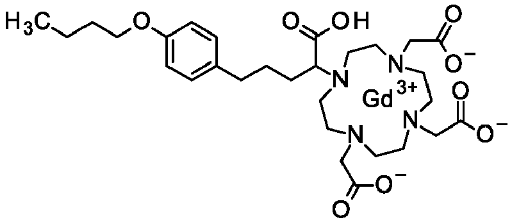 | 4.4 | 5.3 | 15.8 | 34.7 |
| 19 | 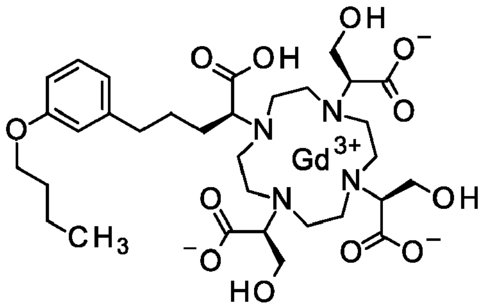 | 4.9 | 5.9 | 18.0 | 39.6 |
| 20 | 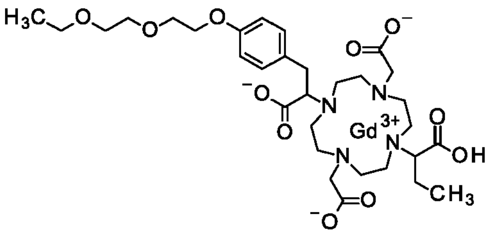 | 4.8 | 5.7 | 6.9 | 10.1 |
| 21 | 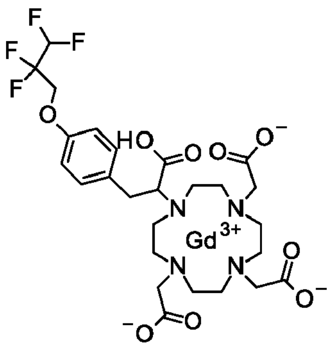 | 3.5 | 4.2 | 10.8 | 20.7 |
| 22 | 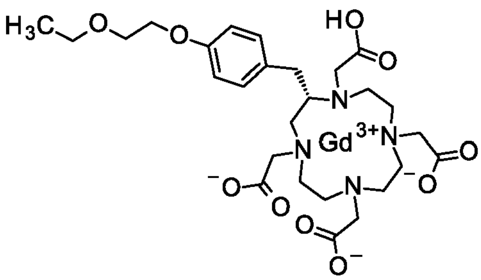 | 4.8 | 5.8 | 6.6 | 8.9 |

(continued)

| No. | Structure | $r_1$ water* | $r_2$ water* | $r_1$ human plasma* | $r_2$ human plasma* |
|---|---|---|---|---|---|
| 23 | 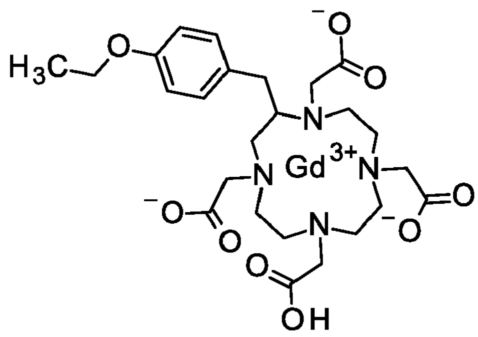 | 4.5 | 5.3 | 6.2 | 8.4 |
| 24 | 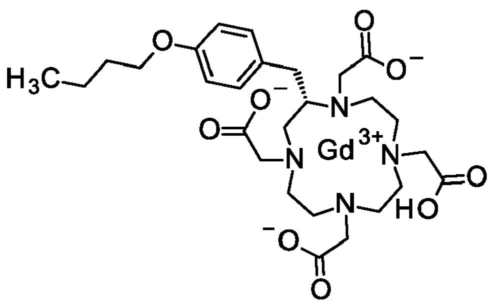 | 5.3 | 9.1 | 9.8 | 16.1 |
| RC 3 | 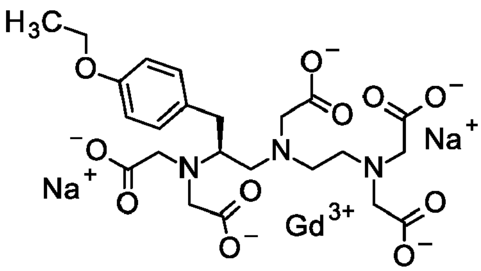 RC3 | 4.7# | 5.1# | 6.9# | 8.7# |
| RC 4 | 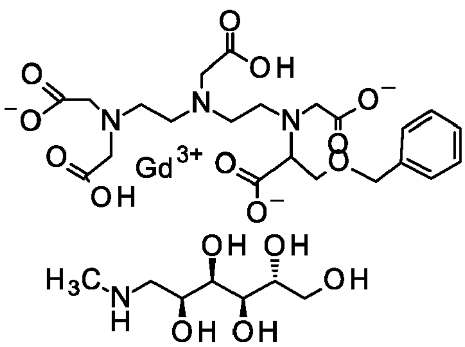 RC4 | 4.0# | 4.3# | 6.3# | 8.7# |

* values are given in $L\cdot mmol^{-1}\cdot s^{-1}$

\# Invest Radiol. 2005 Nov;40(11):715-24. doi: 10.1097/01.rli.0000184756.66360.d3, in bovine plasma (Kreaber GmbH, Pharmaceutical Raw Material, Ellerbek, Germany) instead of human plasma

**Example B** - **Uptake into freshly isolated rat hepatocytes**

Freshly isolated rat hepatocytes were received as described before (Papeleu P. et al. (2006) Isolation of Rat

Hepatocytes. In: Phillips I.R., Shephard E.A. (eds) Cytochrome P450 Protocols. Methods in Molecular Biology, vol 320. Humana Press, Totowa, NJ. https://doi.org/10.1385/1-59259-998-2:229). The rat hepatocytes were incubated (2.5 ·105 cells/well) for 2.5 to 4h in collagen coated 24-well plates (Fa. Beckton Dickinson Biocoat, cell environments, Collagen I Cellware) in Williams E-medium (Fa. Sigma W1878) in an incubator (37°C, 5% CO2 and 95% oxygen). After incubation the adherent hepatocytes were washed once with 37°C Hepes carbonate transport buffer (500 μL/well). The test items and RC3 were prepared from stock solutions (180 to 500 mM concentration) in Hepes carbonate transport buffer at pH 7.4 to reach a final concentration of 100 μmol Gd/L. The specific uptake of the test item or RC3 into rat hepatocytes via OATP1B1 and OATP1B3 was challenged using a known OATP1B1/1B3 inhibitor (Rifampicine, Fa. Sigma R3501, test item and inhibitor 1:1 each 100 μM final concentration). The prepared rat hepatocyte plates were incubated with test solutions (500 μL/well) at 37°C for 10 min using a thermoshaker (Fa. Grant Bio, PHMP). Triplicates were performed for each test item. After incubating the cells were washed once with cold PBS buffer (Fa. Gibco, Dulbeccos PBS (+)). After washing the intact hepatocytes were lysed by addition of 500 μL cold 0.01 % Triton-X 100 in Millipore water for 1h. The strongly hypo-osmolar water led to cell membrane rupture and the release of the intracellular Gd-compound. After 1h 500 μL of 10 nM Terbium ICP-MS standard was added in 2% nitric acid and 0.01% Triton 100 before the Gadolinium concentrations in the lysate were measured using ICP-MS (Agilent 8900, singleQuad, integr. Time 0.3 s, no gas, 5 replicates, Gd m/z 157, Tb m/z 159). As a reference standard RC3 is used in the same well plate. The hepatocyte uptake of the test compound is calculated relative to RC3 (resulting in the unit % of RC3). The results different are summarized in the Table 3.

**Table 3:** Uptake of compounds into freshly isolated rat hepatocytes in % compared to reference compound 1 (RC3, Gd-EOB-DTPA).

| No. | Structure | Uptake rat hepatocytes [% of RC3] | Inhibition Rifampicine [% inhibition] |
|---|---|---|---|
| 12 | 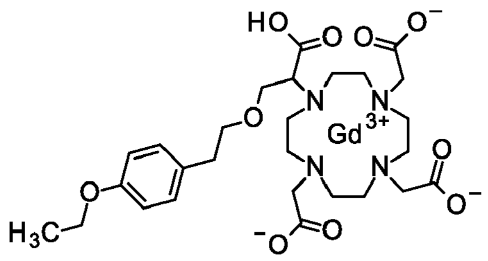 | 29 | 93 |
| 13 | 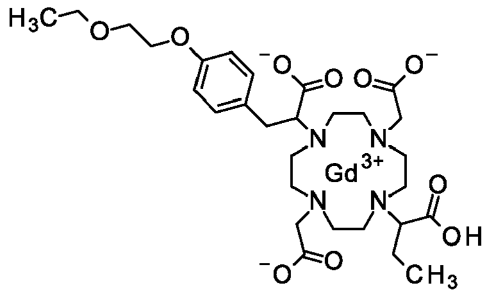 | 89 | 99 |
| 14 | 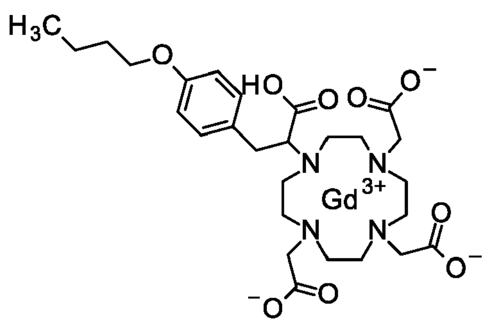 | 96 | 98 |

(continued)

| No. | Structure | Uptake rat hepatocytes [% of RC3] | Inhibition Rifampicine [% inhibition] |
|---|---|---|---|
| 15 | 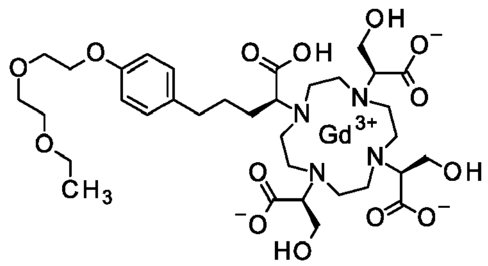 | 109 | 94 |
| 16 | 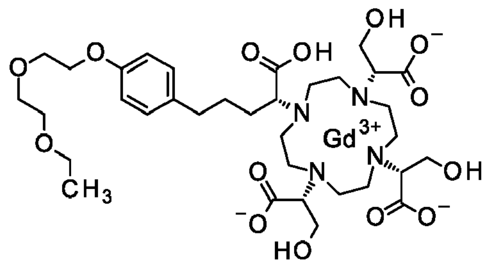 | 92 | 92 |
| 17 | 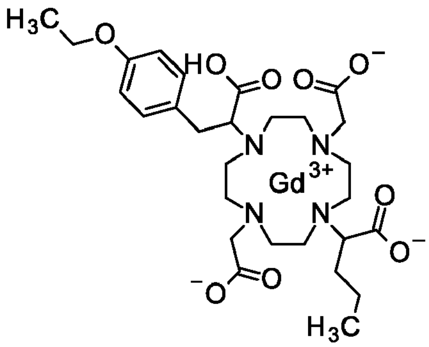 | 87 | 99 |
| 18 | 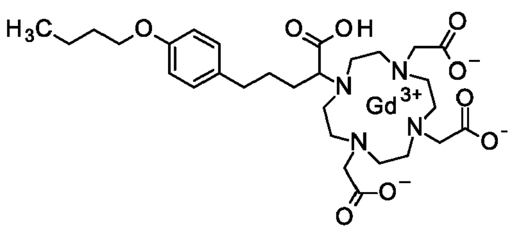 | 87 | 94 |
| 19 | 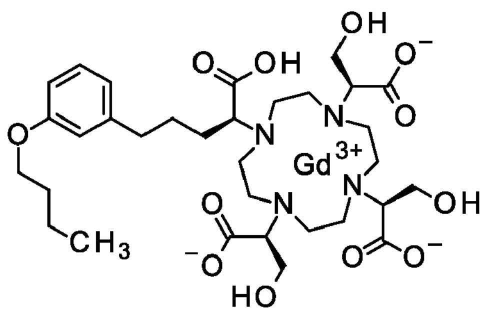 | 98 | 90 |

(continued)

| No. | Structure | Uptake rat hepatocytes [% of RC3] | Inhibition Rifampicine [% inhibition] |
|---|---|---|---|
| 20 | 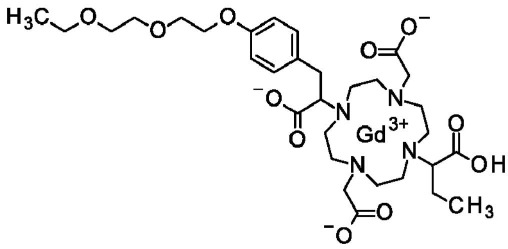 | 90 | 97 |
| 21 | 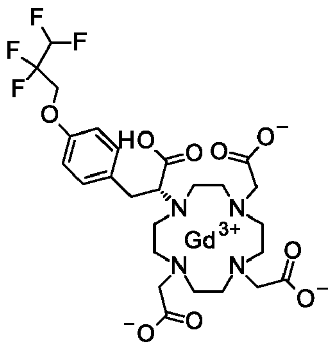 | 106 | 90 |
| 22 | 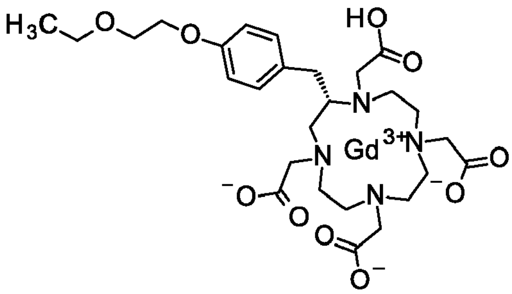 | 67 | 94 |
| 23 | 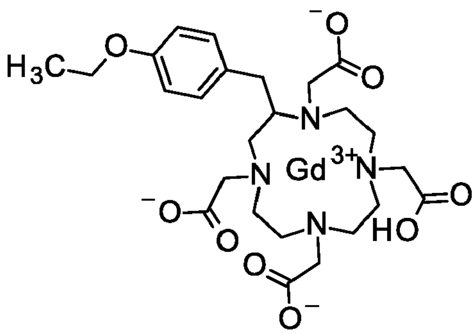 | 73 | 96 |
| 24 | 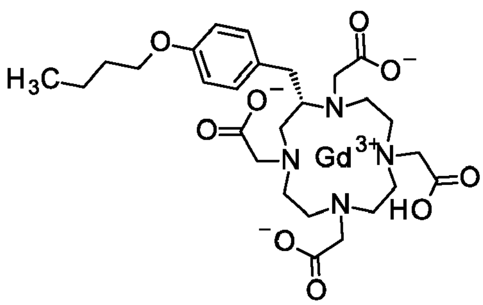 | 137 | 86 |

(continued)

| No. | Structure | Uptake rat hepatocytes [% of RC3] | Inhibition Rifampicine [% inhibition] |
|---|---|---|---|
| RC3 | 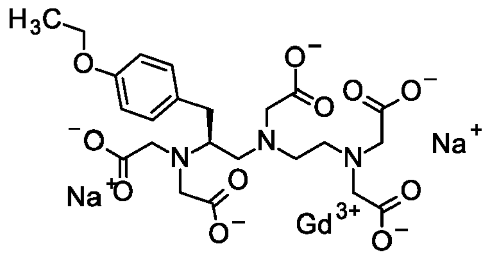 RC3 | 100 | 97 |
| RC4 | 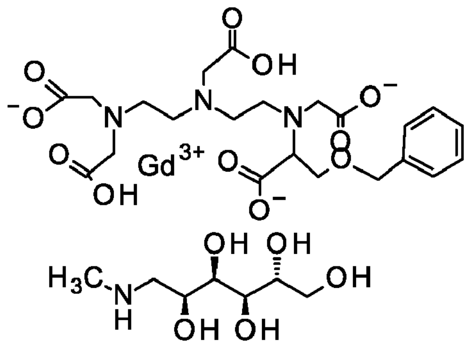 RC4 | 9 | 79 |

**Example C** - **Preparation of formulations**

Preparations of pharmaceutical composition comprising an extracellular contrast agent; and a liver-specific contrast agent were performed in two manufacturing steps. In the first step the formulations for the extracellular contrast agent and the formulation for the liver-specific agent were prepared and autoclaved. In the second step the final formulations were prepared from the extracellular contrast agent formulation and the liver-specific agent formulation.

Example formulation preparation: 608 mg tromethamol-HCl (TRIS, Fa. Merck, #8382Z012709) were dissolved in 500 g Ampuwa (Fa. Fresenius Kabi Deutschland, #19NGA400, 06/2022) and the pH was adjusted with 1070 $\mu$L 4N HCl. Compound 3 (9.531 g) were dissolved in 40 mL 10 mM Trometamol HCl (TRIS buffer) and pH was adjusted with 8 $\mu$L 1 N HCl. The Gd concentration was determined 278 mmol Gd/L using inductively coupled plasma mass spectrometry (ICP-MS, 7500a, Agilent, Waldbronn, Germany). Free ligand H-butrol was added (0.1 mol% = 0.278 mmol/L H-butrol). Final concentration of 200 mmol Gd/L were reached by adding 20.8 mL TRIS buffer. The osmolality was determined (Weskor VAPRO® 5600 Osmometer) and 410 mg NaCl were added to reach an isotonic formulation. The final Gd concentration was determined with 200.7 mmol Gd/L. The liver-specific compound 15 (22 g) were dissolved in 50 mL 10 mM Trometamol HCl (TRIS buffer) and pH was adjusted using HCl (1-4N HCl). The Gd concentration was determined 293.5 mmol Gd/L using inductively coupled plasma mass spectrometry (ICP-MS, 7500a, Agilent, Waldbronn, Germany). Free ligand H-butrol was added (0.1 mol% = 0.294 mmol/L H-butrol). Final concentration of 200 mmol Gd/L were reached by adding 50.1 mL TRIS buffer. The final Gd concentration was determined with 200.0 mmol Gd/L. After sterile filtration (Whatman® Schleich& Schuell, 0.2 $\mu$m) were autoclaved at 121°C, 1 bar for 20 min (including heating time 45 min). Preparations of pharmaceutical composition comprising compound 3 and 15 were manufactured by adding 45 mL of the first formulation to 45 mL of the second formulation (1:1; 100 mmol Gd/L compound 3 and 100 mmol Gd/L compound 15) and by adding 15.5 mL compound + 15.5 mL TRIS-buffer to 31 mL of the second formulation (1:2, 50 mmol compound 3 and 100 mmol Gd/L compound 15).

**Example D** - **MRI in minipigs**

For the animal study the described pharmaceutical composition comprising an extracellular contrast agent (compound 3) and a liver-specific contrast agent (compound 15) were used (Example C). Two additional formulations of the liver-specific test agent (compound 15) were prepared and served as references for the liver specific GBCA signal

enhancement. The ready to use parenteral formulations for the liver MRI minipig study are summarized in table 4.

**Table 4:** Formulations used for the animal study

| **Formulation** *acronym* | **Liver specific GBCA** *Compound 15* (μmol Gd/L) | **Extracellular GBCA** *Compound 3* Formulation (μmol Gd/L) |
|---|---|---|
| Combi1 | 100 | 100 |
| Combi2 | 50 | 100 |
| LS-GBCA1 | 100 | - |
| LS-GBCA2 | 125 | - |

The MRI study was performed on 4 Göttingen minipigs (Ellegaard, Denmark) in a cross-over study design. Each animal received 5 contrast-enhanced liver MRI exams using the formulations specified in table 4. The used contrast protocols of the respective formulations are specified in table 5. The equimolar combination (Combi 1) was tested at two dose levels. The 5 contrast injections per animal were applied in randomized order with at least 2 weeks between the exams. The contrast media were administered using the MRXperion injection system (Medrad, Bayer AG). The injection flow-rate was adapted to normalize the injection-time among the 5 injection protocols. Contrast injection was followed by 20 ml saline chaser, applied at the same flow rate.

**Table 5:** Contrast protocols: Total Gd dose, volume and injection flow rate

| **Formulation** (see table 4) | **Total Gd Dose** (mmol Gd/kg bw) | **Volume** (mL/kg bw) | **Injection Flow Rate** (ml/s) |
|---|---|---|---|
| Combi1 | 0.02 | 0.1 | 1 |
| Combi2 | 0.03 | 0.2 | 2 |
| Combi1 | 0.04 | 0.2 | 2 |
| LS-GBCA1 | 0.02 | 0.2 | 2 |
| LS-GBCA2 | 0.01 | 0.08 | 0.8 |

The animals were investigated under general anesthesia induced with ketamine, 10 mg/kg (Pharmacia, Karlsruhe, Germany) and azaperone, 2 mg/kg i.m. (Stresnil, Janssen-Cilag, Neuss, Germany). The animals were orally intubated, and after intravenous injection of 1.5-5 mg propofol, artificially ventilated. Anesthesia was maintained by intravenous injection with propofol, 9 mg/kg/h (Ratiopharm, Ulm, Germany). The animals heart rates and oxygen saturations were monitored. During image acquisition, end-expiratory breath-holds were used.

MRI scans were performed in a clinical 1.5T MRI system (Avanto Fit, Siemens Healthcare GmbH, Erlangen, Germany). Animals were placed in prone position on the scanner table, equipped with the spine coil. An 18-channel body flex coil was placed over the abdominal region. Liver MRI was performed with a 3D DIXON VIBE sequence (table 6). Liver MRI scans were acquired before (baseline), in dynamic phase (1, 1.83, 4 min p.i.), and in hepatobiliary phase (6, 8, 10, 12, 15, 20, 30 min p.i.).

**Table 6:** MRI sequence parameter

| | |
|---|---|
| Sequence | DIXON VIBE |
| Orientation | Transversal |
| TR (ms) | 6.9 |
| TE 1 (ms) | 2.39 |
| TE 2 (ms) | 4.77 |
| Flip Angle (°) | 10 |
| PAT | CAIPIRINHA |
| PAT factor PE/3D | 2/2 |
| Partial fourier slice | 6/8 |

(continued)

| | |
|---|---|
| Sequence | DIXON VIBE |
| Slices | 88 |
| Slice thickness (mm) | 2 |
| Pixel size (mm×mm) | 1.4×1.4 |
| Matrix | 192x256 |
| Scan time (s) | 18 |
| Image reconstuction | Water / Fat image |

The image data analysis was done on an external workstation (SyngoVia, Siemens Healthcare, Erlangen, Germany) using the DIXON derived water images. Regions of interest (ROIs) were manually placed in the liver parenchyma (4 ROIs) and the portal vein (3 ROIs) at different slice levels. The ROIs were copied to all volumes (i.e. time points). Subsequently, ROI positions were controlled, and corrected if necessary. For each time point the relative signal enhancement (rSE) was calculated based on the signal intensity (SI):

$$\text{Relative signal enhancement (rSE)} = (\text{SI} - \text{SI baseline}) / \text{SI baseline},$$

while SI baseline represents the native SI prior to contrast administration. For each anatomical region the relative signal enhancement per time point was averaged between individual ROIs. The results demonstrate the effect of the combination, comprising of an extracellular contrast agent and a liver-specific contrast agent, on the MRI signal enhancement compared to the liver-specific agent alone. The rSE in blood (determined in the portal vein) is the most relevant parameter and determines the vascular contrast. This is particularly relevant in the dynamic phase (0-4 min). The rSE of formulation Combi1 is about a factor 1.7 higher than the liver-specfic GBCA alone (LS-GBCA 2) (Fig. 2A). On the other hand, the high liver specific signal enhancement of Combi1 during hepatobiliary phase is still given. A typical time point for the clinical assessment of the hepatobiliary phase liver enhancement is 20 min post injection. At this point in time the rsE of Combi1 is only a factor of 1.05 higher than that of the liver specific agent (LS-GBCA2) alone (Fig. 2B). That means that contribution of the extracellular GBCA to the overall rSE in this imaging phase is rather low.

The described signal characteristics of the combination is similar for the higher total Gd doses (Comib1 with 0.04 mmol/kg vs. LS-GBCA1). The blood rSE increases significantly for Combi1 while the differences in rSE during the hepatobiliary phase are small (Fig.3). For the second combination (Combi2) the Gd ratio of the liver-specfic agent is twofold higher than that of the extracellular GBCA. Accordingly, the rSE increase of the blood is lower compared that of Combi1 but still significantly (Factor 1.13) higher than the rSE of the liver specific agent alone (LS-GBCA2).

**Example E - CT phantom measurements**

The x-ray attenuation of the pharmaceutical composition comprising of an extracellular contrast agent and a liver-specific contrast agent comprising a paramagnetic metal ion and a macrocyclic chelating moiety was investigated with computed tomography. Therefore, test samples of the formulations described in example C (Combi1 and Combi2) were placed into the central insert of a body-equivalent CT abdomen phantom. CT imaging was performed using a Dual Source CT Scanner (Somatom Force, Siemens Healthcare, Erlangen, Germany) operating in single source mode. The scan parameters were 120 kV tube voltage, 200 mAs, 0.5s rotation time and image reconstruction as performed using a Br40 kernel, 3 mm slice thickness and a field of view of 400 $mm^2$. CT imaging results in a strong contrast between the test sample and the surrounding tissue equivalent phantom material for both investigated formulations (Figure 1).

For quantitative evaluation of the signal enhancement a region of interest was placed in the around the test tube in 3 adjacent slices. The averaged signal intensity was 1096 Hounsfield units (HU) for test sample A (Combi 1) and 826 HU for sample B (Combi 2).

**Reference Compounds**

**Reference compound 1**
Gadovist®/ Gadavist® (Gd-DO3A-butrol, gadobutrol, Bayer AG, Germany)

**Reference compound 2**
Dotarem® (Gd-DOTA, gadoterate meglumine, Guerbet, France)

**Reference compound 3**
Primovist® (Gd-EOB-DTPA, gadoxetate disodium, Bayer AG, Germany)

**Reference compound 4**
MultiHance® (Gd-BOPTA, gadobenic acid, Bracco, Italy)

## Claims

**1.** A pharmaceutical composition comprising

(i) an extracellular contrast agent; and
(ii) a liver-specific contrast agent comprising a paramagnetic metal ion and a macrocyclic chelating moiety.

**2.** The pharmaceutical composition according to claim 1, comprising

(i) an extracellular contrast agent comprising a lanthanide metal ion complexed to a macrocyclic chelating moiety; and
(ii) a liver-specific contrast agent comprising a lanthanide metal ion complexed to a macrocyclic chelating moiety.

**3.** The pharmaceutical composition according to claim 1 or claim 2, wherein the lanthanide metal ion is $Gd^{3+}$.

**4.** The pharmaceutical composition according to any of claims 1 to 3, wherein

(i) the extracellular contrast agent is selected from a compound of formula (I), a compound of formula (II) or a compound of formula (III), the compound of formula (I) having the formula

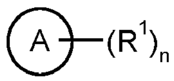

(I) ,

wherein:

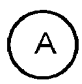

represents a

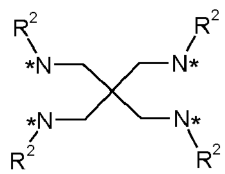

group, wherein * indicates the point of attachment of said group with $R^1$,

$R^1$ represents a group $R^3$,
n represents an integer of 4,
$R^2$ represents a hydrogen atom,
$R^3$ represents a group selected from

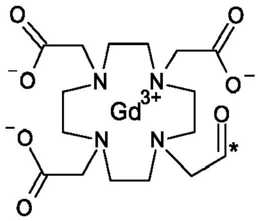 and 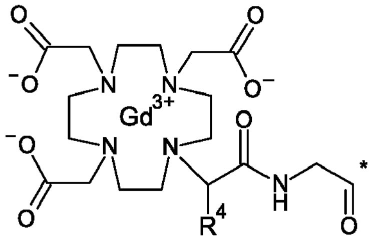 ,

wherein * indicates the point of attachment of said group with the rest of the molecule,
$R^4$ represents a hydrogen atom or a methyl group,
or a stereoisomer, a tautomer, an N-oxide, a hydrate, a solvate, or a salt thereof, or a mixture of same;
the compound of formula (II) having the formula

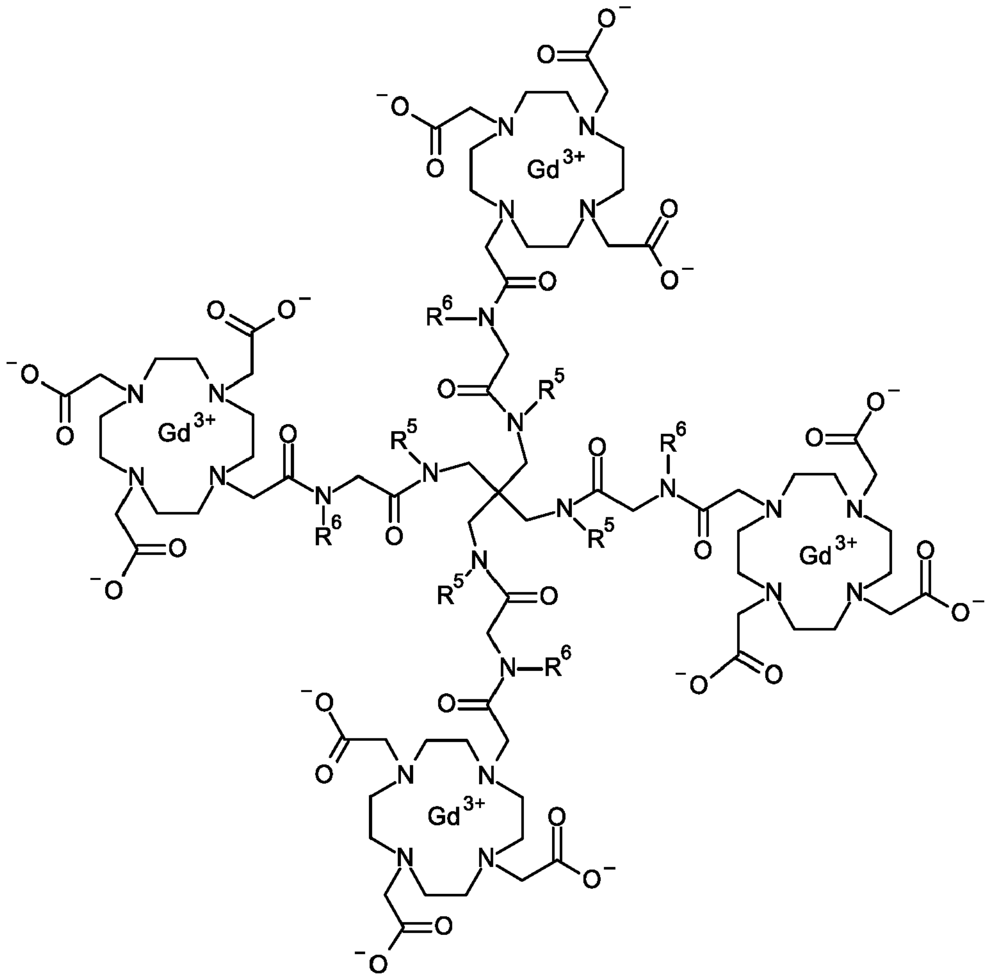

**(II)** ,

wherein
$R^5$ represents a hydrogen atom,
$R^6$ represents a group selected from
$C_1$-$C_4$-alkyl, $C_3$-$C_5$-cycloalkyl, ($C_1$-$C_2$-alkoxy)-($C_2$-$C_3$-alkyl)- and phenyl, wherein said $C_1$-$C_4$-alkyl group is optionally substituted, identically or differently, with a phenyl group, which phenyl group is optionally substituted, one, two or three times, identically or differently, with a halogen atom or a group selected from: $C_1$-$C_3$-alkyl, $C_1$-$C_3$-haloalkyl and $C_1$-$C_3$-alkoxy, and

wherein said phenyl group is optionally substituted, one, two or three times, identically or differently, with a halogen atom or a group selected from $C_1$-$C_3$-alkyl, $C_1$-$C_3$-haloalkyl and $C_1$-$C_3$-alkoxy

or a stereoisomer, a tautomer, an N-oxide, a hydrate, a solvate, or a salt thereof, or a mixture of same; and the compound of formula (III) having the formula,

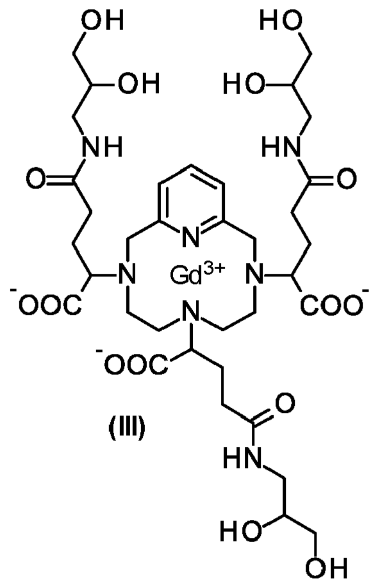

(III)

and

(ii) the liver-specific contrast agent is selected from a compound of formula (IV) and a compound of formula (V), the compound of formula (IV) having the formula

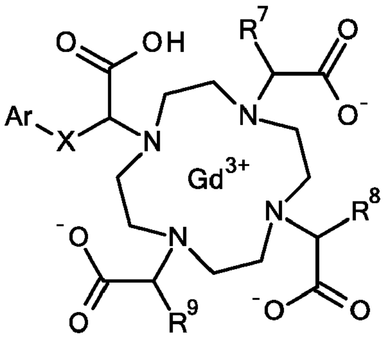

(IV)

,

wherein

Ar represents a group selected from

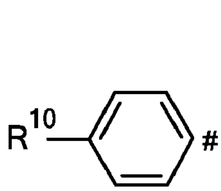

and

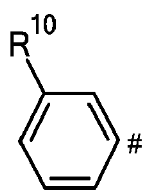

,

wherein # indicates the point of attachment to X,
X represents a group selected from $CH_2$ and $(CH_2)_3$,
$R^7$ and $R^9$ represent, independently for each occurrence, a hydrogen atom or a $-CH_2OH$ group,
$R^8$ represents a hydrogen atom or a group selected from $C_1$-$C_3$-alkyl, $-CH_2OH$, $-(CH_2)_2OH$ and $-CH_2OCH_3$,
$R^{10}$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,
or a stereoisomer, a tautomer, an N-oxide, a hydrate, a solvate, or a salt thereof, or a mixture of same;
the compound of formula (V) having the formula

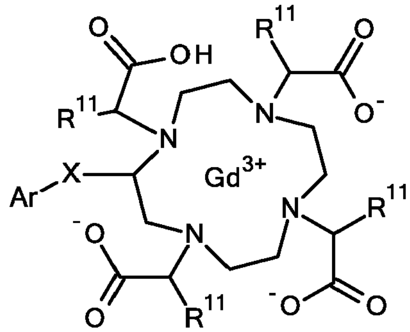

**(V)**

wherein
Ar represents a group selected from

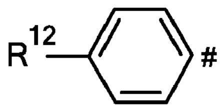

and

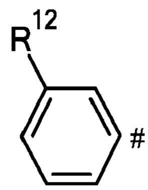

,

wherein # indicates the point of attachment to X,
X represents a group selected from $CH_2$ and $(CH_2)_2$;
$R^{11}$ represents a hydrogen atom or a group selected from $C_1$-$C_3$-alkyl, $-CH_2OH$,$-(CH_2)_2OH$ and $-CH_2OCH_3$;
$R^{12}$ represents a group selected from $C_2$-$C_5$-alkoxy, (H3C-CH2O)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-;
or a stereoisomer, a tautomer, an N-oxide, a hydrate, a solvate, or a salt thereof, or a mixture of same.

**5.** The pharmaceutical composition according to any of claims 1 to 4, wherein

(i) the extracellular contrast agent is selected from a compound of formula (I) and a compound of formula (II), the compound of formula (I) having the formula

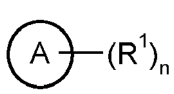

(I) ,

wherein:

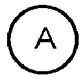

represents a

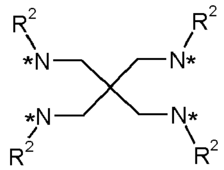

group, wherein * indicates the point of attachment of said group with $R^1$,

$R^1$ represents a group $R^3$,
n represents an integer of 4,
$R^2$ represents a hydrogen atom,
$R^3$ represents a group selected from

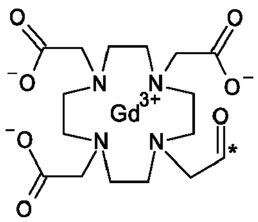

and

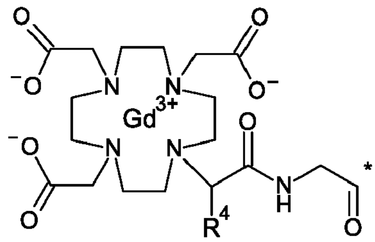

,

wherein * indicates the point of attachment of said group with the rest of the molecule,
$R^4$ represents a hydrogen atom or a methyl group,
or a stereoisomer, a tautomer, an N-oxide, a hydrate, a solvate, or a salt thereof, or a mixture of same;
the compound of formula (II) having the formula

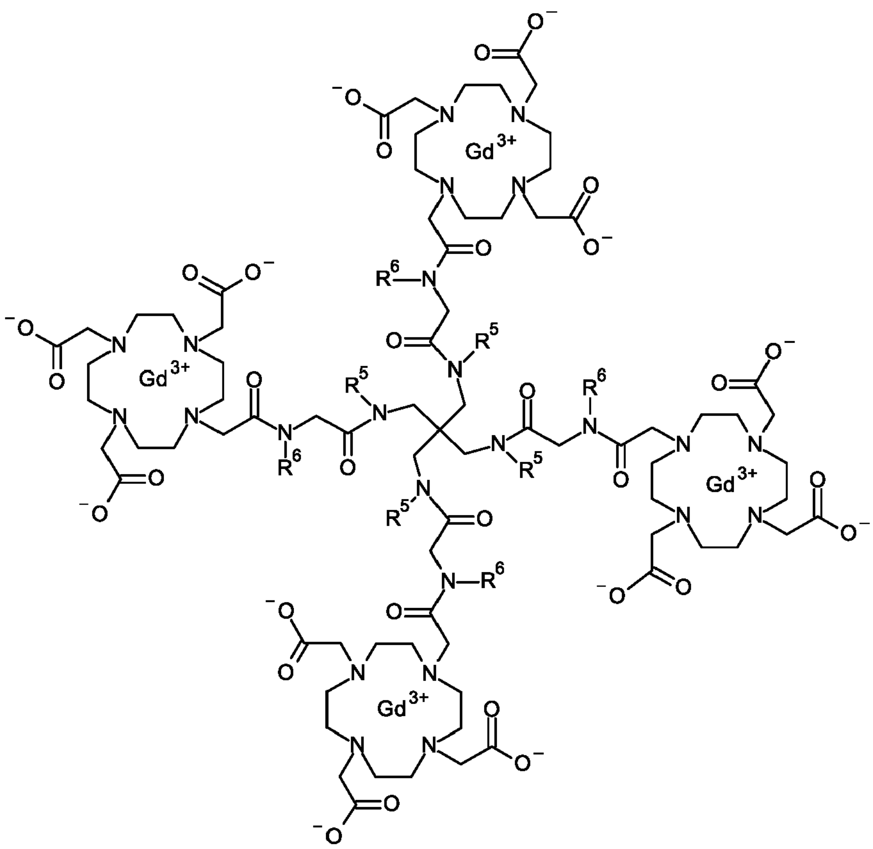

(II) ,

wherein
$R^5$ represents a hydrogen atom,
$R^6$ represents a group selected from
$C_1$-$C_4$-alkyl, $C_3$-$C_5$-cycloalkyl, ($C_1$-$C_2$-alkoxy)-($C_2$-$C_3$-alkyl)- and phenyl,
wherein said $C_1$-$C_4$-alkyl group is optionally substituted, identically or differently, with a phenyl group, which phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy, and

wherein said phenyl group is optionally substituted, one, two or three times, identically or differently, with a halogen atom or a group selected from:
$C_1$-$C_3$-alkyl, $C_1$-$C_3$-haloalkyl and $C_1$-$C_3$-alkoxy or a stereoisomer, a tautomer, an N-oxide, a hydrate, a solvate, or a salt thereof, or a mixture of same;

and
(ii) the liver-specific contrast agent is selected from a compound of formula (IV) and a compound of formula (V), the compound of formula (IV) having the formula

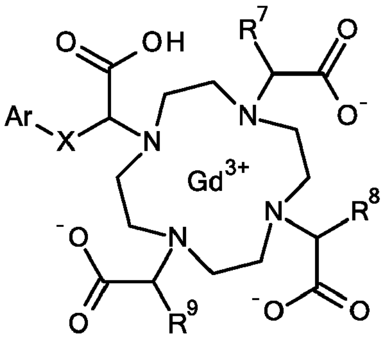

**(IV)**

,

wherein

Ar represents a group selected from

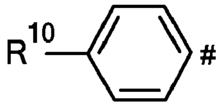

and

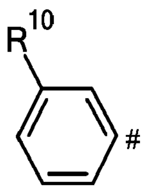

,

wherein # indicates the point of attachment to X,
X represents a group selected from $CH_2$ and $(CH_2)_3$,
$R^7$ and $R^9$ represent, independently for each occurrence, a hydrogen atom or a $-CH_2OH$ group,
$R^8$ represents a hydrogen atom or a group selected from $C_1$-$C_3$-alkyl, $-CH_2OH$, $-(CH_2)_2OH$ and $-CH_2OCH_3$,
$R^{10}$ represents a group selected from $C_2$-$C_5$-alkoxy, $(C_1$-$C_3$-alkoxy$)-(CH_2)_2-O-$, $(C_1$-$C_3$-alkoxy$)-(CH_2)_2-O-(CH_2)_2-O-$ and $(C_1$-$C_3$-alkoxy$)-(CH_2)_2-O-(CH_2)_2-O-(CH_2)_2-O-$, wherein said $C_1$-$C_3$-alkoxy groups and $C_2$-$C_5$-alkoxy groups are optionally substituted, one, two, three or four times, with a fluorine atom,
or a stereoisomer, a tautomer, an N-oxide, a hydrate, a solvate, or a salt thereof, or a mixture of same;
the compound of formula (V) having the formula

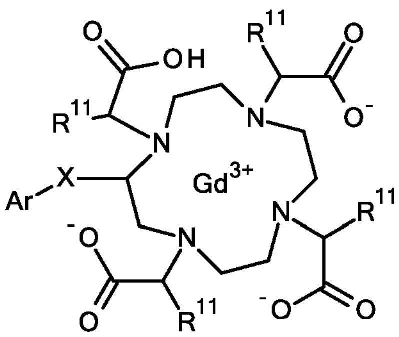

**(V)**

,

wherein
Ar represents a group selected from

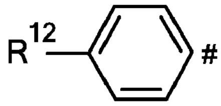

and

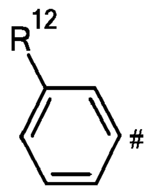

,

wherein # indicates the point of attachment to X,
X represents a group selected from $CH_2$ and $(CH_2)_2$,
$R^{11}$ represents a hydrogen atom or a group selected from $C_1$-$C_3$-alkyl, $-CH_2OH$,$-(CH_2)_2OH$ and $-CH_2OCH_3$;
$R^{12}$ represents a group selected from $C_2$-$C_5$-alkoxy, $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
or a stereoisomer, a tautomer, an N-oxide, a hydrate, a solvate, or a salt thereof, or a mixture of same.

**6.** The pharmaceutical composition according to any of claims 1 to 5, wherein

(i) the extracellular contrast agent is a compound of the formula (I)

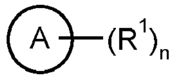

(I) ,

wherein:

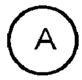

represents a group,

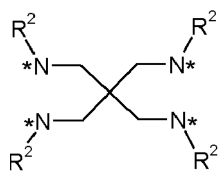

group, wherein * indicates the point of attachment of said group with $R^1$ ;

$R^1$ represents a group $R^3$,
n represents an integer of 4,
$R^2$ represents a hydrogen atom,
$R^3$ represents a group selected from

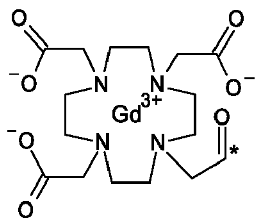

and

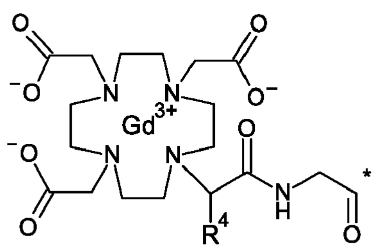

,

wherein * indicates the point of attachment of said group with the rest of the molecule,

$R^4$ represents a hydrogen atom or a methyl group or a stereoisomer, a tautomer, an N-oxide, a hydrate, a solvate, or a salt thereof, or a mixture of same;

and

(ii) the liver-specific contrast agent is a compound of the formula (IV)

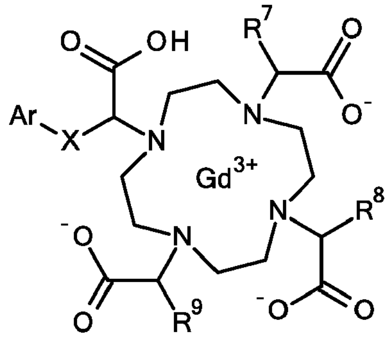

**(IV)** ,

wherein

Ar represents a group selected from

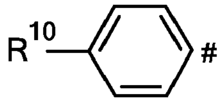

and

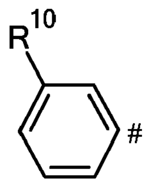

,

wherein # indicates the point of attachment to X,
X represents a group selected from $CH_2$ and $(CH_2)_3$,
$R^7$, $R^8$ and $R^9$ represent, independently for each occurrence, a hydrogen atom or a group selected from $C_1$-$C_3$-alkyl, $-CH_2OH$, $-(CH_2)_2OH$ and $-CH_2OCH_3$,
$R^{10}$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
or a stereoisomer, a tautomer, an N-oxide, a hydrate, a solvate, or a salt thereof, or a mixture of same.

**7.** The pharmaceutical composition according to any of claims 1 to 6, wherein

(i) the extracellular contrast agent is a compound of the formula

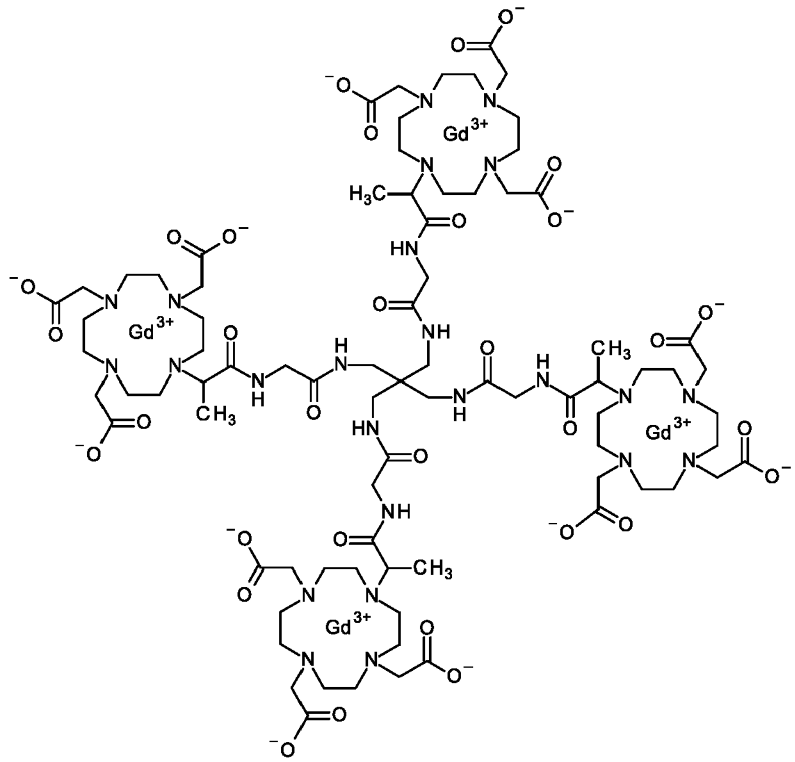

or a stereoisomer, a tautomer, an N-oxide, a hydrate, a solvate, or a salt thereof, or a mixture of same; and

(ii) the liver-specific contrast agent is a compound of the formula

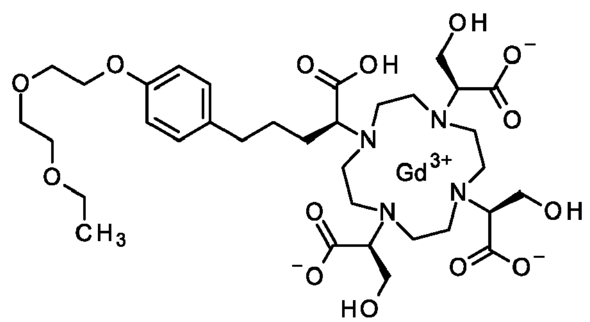

or a stereoisomer, a tautomer, an N-oxide, a hydrate, a solvate, or a salt thereof, or a mixture of same.

**8.** The pharmaceutical composition according to any of claims 1 to 7, wherein the mole ratio of extracellular contrast agent to liver-specific contrast agent lies in the range of from 10:1 to 1:10.

**9.** The pharmaceutical composition according to any of claims 1 to 8, wherein the mole ratio of extracellular contrast agent to liver-specific contrast agent lies in the range of from 1:1 to 1:10, preferably in the range of from 1:1 to 1:5, more preferably in the range of from 1:1 to 1:3.

**10.** The pharmaceutical composition according to any of claims 1 to 8, wherein the mole ratio of extracellular contrast agent to liver-specific contrast agent lies in the range of from 1:1 to 10:1, preferably in the range of from 1:1 to 5:1, more preferably in the range of from 1:1 to 3:1.

**11.** Use of a pharmaceutical composition according to any of claims 1 to 10 for radiological imaging, preferably for diagnostic imaging, more preferably for magnetic resonance imaging (MRI) or computer tomography (CT).

**12.** Use according to claim 11 for magnetic resonance imaging, preferably for oncological, neurological or cardiovascular indications and more preferably for magnetic resonance imaging or computer tomography of the central nervous, vascular, renal or hepatobiliary system or of the gastrointestinal tract.

**13.** Use according to claim 11 or claim 12 for magnetic resonance imaging or computed tomography of the liver.

**14.** A pharmaceutical composition according to any of claims 1 to 10 for use in magnetic resonance imaging (MRI) or computed tomography, preferably for oncological, neurological or cardiovascular indications and more preferably for MRI of the central nervous, vascular, renal or hepatobiliary system or of the gastrointestinal tract,

**15.** A pharmaceutical composition according to any of claims 1 to 10 for use in magnetic resonance imaging or computed tomography of the liver.

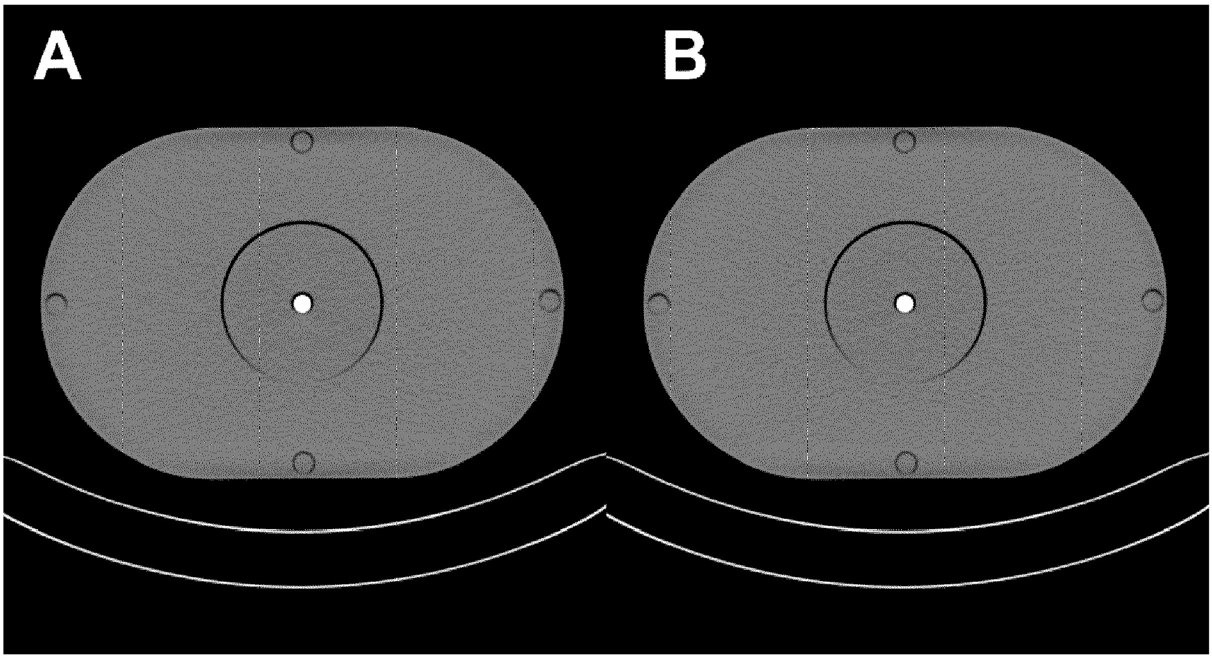


Figure 1

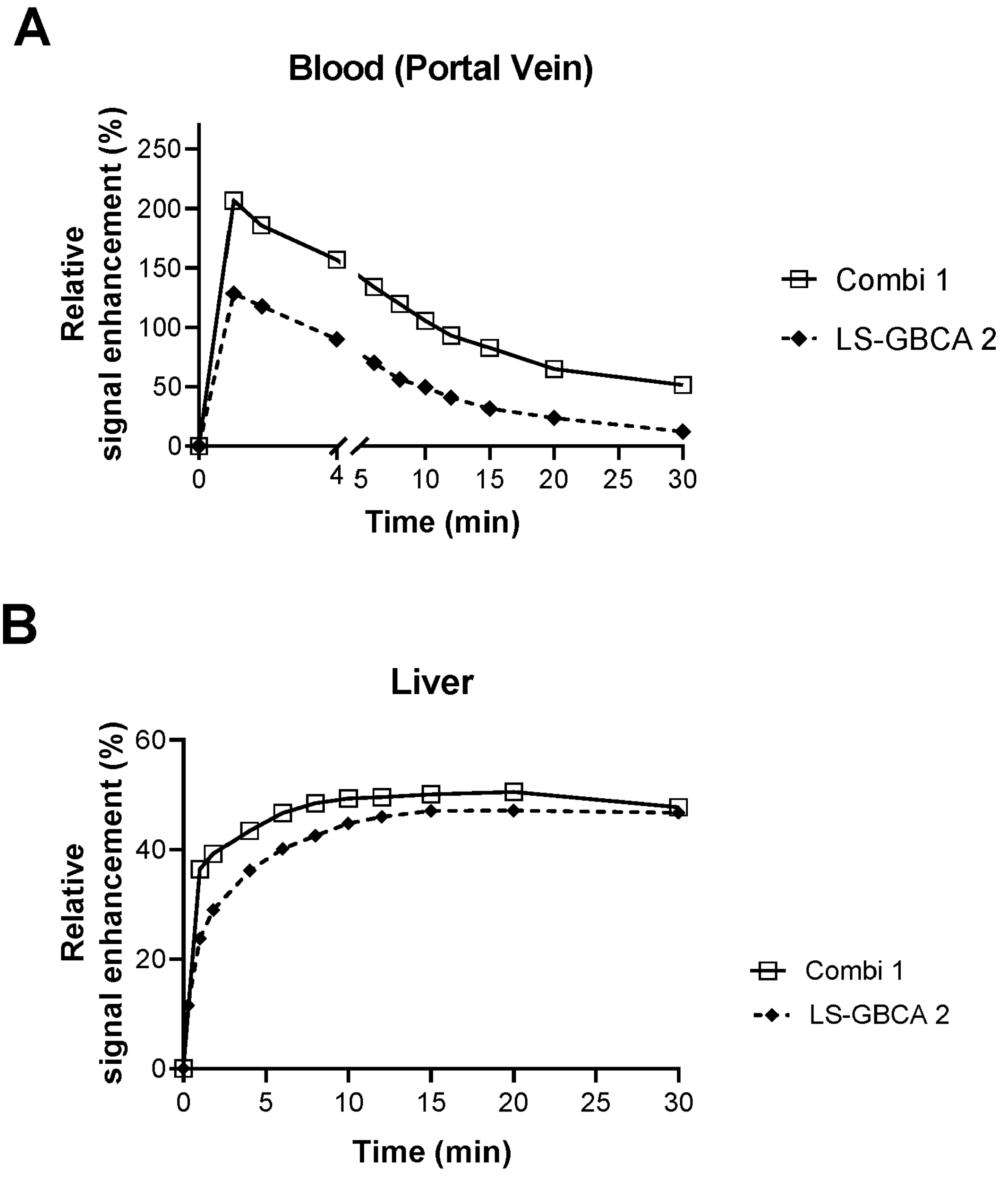
A
Blood (Portal Vein)
Relative signal enhancement (%)
250
200
150
100
50
0
0
4
5
10
15
20
25
30
Time (min)
Combi 1
LS-GBCA 2
B
Liver
Relative signal enhancement (%)
60
40
20
0
0
5
10
15
20
25
30
Time (min)
Combi 1
LS-GBCA 2

Figure 2

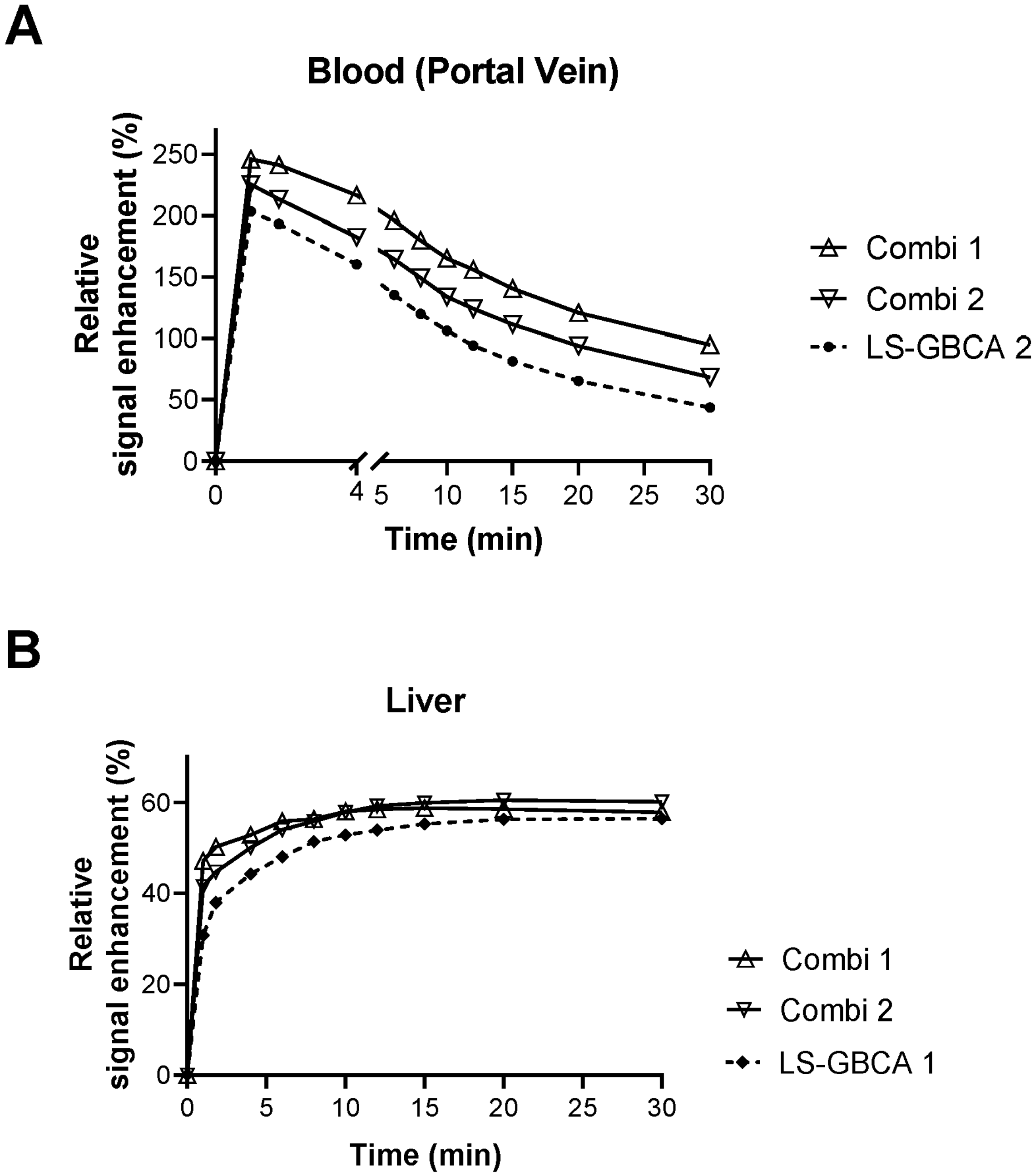
A
Blood (Portal Vein)
Relative signal enhancement (%)
250
200
150
100
50
0
0
4
5
10
15
20
25
30
Time (min)
Combi 1
Combi 2
LS-GBCA 2
B
Liver
Relative signal enhancement (%)
60
40
20
0
0
5
10
15
20
25
30
Time (min)
Combi 1
Combi 2
LS-GBCA 1

Figure 3

Europäisches Patentamt
European Patent Office
Office européen des brevets

# EUROPEAN SEARCH REPORT

Application Number
EP 22 19 4919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>A | WO 2017/093336 A1 (GE HEALTHCARE AS [NO]) 8 June 2017 (2017-06-08)<br>* paragraphs [0066] - [0097] *<br>----- | 1-3,8-15<br>4-7 | INV.<br>A61K49/00<br>A61K49/04<br>A61K49/10<br>C07D257/02 |
| A | AH RUM BAEK ET AL: "Gadolinium Complex of 1,4,7,10-Tetraazacyclododecane-1,4,7-trisacetic Acid (DO3A);Ethoxybenzyl (EOB) Conjugate as a New Macrocyclic Hepatobiliary MRI Contrast Agent", JOURNAL OF MEDICINAL CHEMISTRY, vol. 60, no. 12, 31 May 2017 (2017-05-31), pages 4861-4868, XP055448653, US<br>ISSN: 0022-2623, DOI: 10.1021/acs.jmedchem.7b00060<br>* abstract *<br>* compound 6 *<br>----- | 1-15 | |
| A,D | WO 2018/096082 A1 (BAYER PHARMA AG [DE]) 31 May 2018 (2018-05-31)<br>* claims 1-15 *<br>* examples *<br>----- | 1-15 | TECHNICAL FIELDS SEARCHED (IPC) |
| A | WO 2020/104602 A1 (BAYER AG [DE]; BAYER PHARMA AG [DE]) 28 May 2020 (2020-05-28)<br>* examples 4,5 *<br>----- | 1-15 | A61K<br>C07D |

-/--

The present search report has been drawn up for all claims

3

| Place of search | Date of completion of the search | Examiner |
|---|---|---|
| The Hague | 6 February 2023 | Bliem, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 19 4919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CAROLINE ROBIC ET AL: "Physicochemical and Pharmacokinetic Profiles of Gadopiclenol : A New Macrocyclic Gadolinium Chelate With High T1 Relaxivity", INVESTIGATIVE RADIOLOGY, vol. 54, no. 8, 1 August 2019 (2019-08-01), pages 475-484, XP055761852, US ISSN: 0020-9996, DOI: 10.1097/RLI.0000000000000563 * abstract * * figure 1 * ----- | 1-15 | |
| | | | TECHNICAL FIELDS SEARCHED (IPC) |

The present search report has been drawn up for all claims

3

| Place of search | Date of completion of the search | Examiner |
|---|---|---|
| The Hague | 6 February 2023 | Bliem, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 19 4919

This annex lists the patent family members relating to the patent documents cited in the above-mentioned European search report. The members are as contained in the European Patent Office EDP file on
The European Patent Office is in no way liable for these particulars which are merely given for the purpose of information.

06-02-2023

| Patent document cited in search report | Publication date | Patent family member(s) | Publication date |
|---|---|---|---|
| WO 2017093336 A1 | 08-06-2017 | CN 108289970 A | 17-07-2018 |
| | | EP 3383441 A1 | 10-10-2018 |
| | | JP 7049993 B2 | 07-04-2022 |
| | | JP 2019500333 A | 10-01-2019 |
| | | JP 2022046482 A | 23-03-2022 |
| | | US 2018303960 A1 | 25-10-2018 |
| | | WO 2017093336 A1 | 08-06-2017 |
| WO 2018096082 A1 | 31-05-2018 | AR 110244 A1 | 13-03-2019 |
| | | CA 3044877 A1 | 31-05-2018 |
| | | CN 110035996 A | 19-07-2019 |
| | | EP 3544964 A1 | 02-10-2019 |
| | | ES 2814555 T3 | 29-03-2021 |
| | | JP 7034160 B2 | 11-03-2022 |
| | | JP 2020513402 A | 14-05-2020 |
| | | KR 20190086538 A | 22-07-2019 |
| | | RU 2019119159 A | 28-12-2020 |
| | | TW 201825480 A | 16-07-2018 |
| | | US 2020283420 A1 | 10-09-2020 |
| | | US 2021221798 A1 | 22-07-2021 |
| | | UY 37500 A | 29-06-2018 |
| | | WO 2018096082 A1 | 31-05-2018 |
| WO 2020104602 A1 | 28-05-2020 | AU 2019382881 A1 | 20-05-2021 |
| | | BR 112021007707 A2 | 27-07-2021 |
| | | CA 3120665 A1 | 28-05-2020 |
| | | CL 2021001331 A1 | 22-10-2021 |
| | | CN 113164628 A | 23-07-2021 |
| | | CO 2021006034 A2 | 20-05-2021 |
| | | EP 3883613 A1 | 29-09-2021 |
| | | IL 283281 A | 29-07-2021 |
| | | JP 2022508185 A | 19-01-2022 |
| | | KR 20210095168 A | 30-07-2021 |
| | | PE 20211471 A1 | 05-08-2021 |
| | | SG 11202104657R A | 29-06-2021 |
| | | US 2022008562 A1 | 13-01-2022 |
| | | WO 2020104602 A1 | 28-05-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017093336 A
- WO 2016193190 A
- WO 2018096082 A
- WO 2022013454 A
- EP 2022056541 W
- WO 2019051197 A
- WO 9731905 A


### Non-patent literature cited in the description


- **ROHRER M et al.** *Invest Radiol.,* November 2005, vol. 40 (11), 715-24
- **KAHN J et al.** *Radiology,* 2017, vol. 282 (3), 708-716
- **FRENZEL et al.** *Invest Radiol.,* December 2008, vol. 43 (12), 817-28
- Historical development of x-ray contrast media for urography and angiography. **CLAUSS W ; SPECK U et al.** Computed tomography. Springer, 1996, 1-11
- **NOWAK et al.** *Med Phys,* 03 December 2011, vol. 8 (12), 6469-82
- **WELLE et al.** *Abdominal Radiology,* 2021, vol. 46, 4588-4600
- *CHEMICAL ABSTRACTS,* 162148-48,3
- **L. DAI ; J. ZHANG ; Y. CHEN ; L.E. MACKENZIE ; R. PAL ; G.-L. LAW.** *Inorg. Chem.,* 2019, vol. 58, 12506-12510
- **Q. YUAN ; P. XUE ; M. FANG ; E. FU ; C. WU.** *Synthetic Communications,* 2003, vol. 33 (11), 1911-1916
- **T.W. GREENE ; P.G.M. WUTS.** Protective Groups in Organic Synthesis. Wiley, 1999
- *CHEMICAL ABSTRACTS,* 72594-77-5
- *CHEMICAL ABSTRACTS,* 112-35-6
- *CHEMICAL ABSTRACTS,* 16853-85-3
- *CHEMICAL ABSTRACTS,* 7647-01-0
- *CHEMICAL ABSTRACTS,* 123-11-5
- *CHEMICAL ABSTRACTS,* 16940-66-2
- *CHEMICAL ABSTRACTS,* 603-35-0
- *CHEMICAL ABSTRACTS,* 870-50-8
- *CHEMICAL ABSTRACTS,* 6192-52-5
- *CHEMICAL ABSTRACTS,* 7440-05-3
- *CHEMICAL ABSTRACTS,* 584-08-7
- *CHEMICAL ABSTRACTS,* 64-18-6
- **ROBIC et al.** *Invest Radiol.,* August 2019, vol. 54 (8), 475-484
- *Invest Radiol.,* November 2005, vol. 40 (11), 715-24
- Isolation of Rat Hepatocytes. **PAPELEU P. et al.** Cytochrome P450 Protocols. Methods in Molecular Biology. Humana Press, 2006, vol. 320